# EUROPEAN PATENT APPLICATION

(11) **EP 1 586 643 A2**
(43) Date of publication of application: **19.10.2005**
(21) Application number: 05010702.8
(22) Date of filing: 17.11.2000
(51) Int. Cl.: C12N 15/12, C07K 14/47, C07K 14/575, C07K 14/705, A61K 38/17, C07K 16/18, C07K 16/28, G01N 33/53, G01N 33/68

(54) **Polypeptides and nucleic acids encoding same**

(30) Priority: 19.11.1999 US 166336 P; 29.11.1999 US 167785 P; 08.03.2000 US 187844 P; 16.11.2000 US 715417
(62) Divisional of application: 00978739.1
(71) Applicant: Curagen Corporation, New Haven, CT 06511 (US)
(72) Inventor: Shimkets, Richard A., West Haven, CT 06516 (US); Lichenstein, Henri, Madison, CT 06443 (US); Vernet, Corine, Branford, CT 06471 (US); Fernandes, Elma, Branford, CT 06405 (US)
(74) Representative: MacLean, Martin Robert

(57) **Abstract**

The present invention provides novel isolated NOVX polynucleotides and polypeptides encoded by the NOVX polynucleotides. Also provided are the antibodies that immunospecifically bind to a NOVX polypeptide or any derivative, variant, mutant or fragment of the NOVX polypeptide, polynucleotide or antibody. The invention additionally provides methods in which the NOVX polypeptide, polynucleotide and antibody are utilized in the detection and treatment of a broad range of pathological states, as well as to other uses.

## Description

### BACKGROUND OF THE INVENTION

The invention generally relates to nucleic acids and polypeptides encoded therefrom. More specifically, the invention relates to nucleic acids encoding membrane bound and secreted polypeptides, as well as vectors, host cells, antibodies, and recombinant methods for producing these nucleic acids and polypeptides.

### SUMMARY OF THE INVENTION

The invention is based, in part, upon the discovery of a novel polynucleotide sequences encoding novel polypeptides.

Accordingly, in one aspect, the invention provides an isolated nucleic acid molecule that includes the sequence of SEQ ID NO: 2n-1, wherein n is an integer between 1-16 or a fragment, homolog, analog or derivative thereof. The nucleic acid can include, *e*.*g*., a nucleic acid sequence encoding a polypeptide at least 85% identical to a polypeptide that includes the amino acid sequences of SEQ ID NO: 2n, wherein n is an integer between 1-16. The nucleic acid can be, *e*.*g*., a genomic DNA fragment, or a cDNA molecule.

Also included in the invention is a vector containing one or more of the nucleic acids described herein, and a cell containing the vectors or nucleic acids described herein.

The invention is also directed to host cells transformed with a vector comprising any of the nucleic acid molecules described above.

In another aspect, the invention includes a pharmaceutical composition that includes an NOVX nucleic acid and a pharmaceutically acceptable carrier or diluent.

In a further aspect, the invention includes a substantially purified NOVX polypeptide, *e*.*g*., any of the NOVX polypeptides encoded by an NOVX nucleic acid, and fragments, homologs, analogs, and derivatives thereof. The invention also includes a pharmaceutical composition that includes an NOVX polypeptide and a pharmaceutically acceptable carrier or diluent.

In still a further aspect, the invention provides an antibody that binds specifically to an NOVX polypeptide. The antibody can be, *e*.*g*., a monoclonal or polyclonal antibody, and fragments, homologs, analogs, and derivatives thereof. The invention also includes a pharmaceutical composition including NOVX antibody and a pharmaceutically acceptable carrier or diluent. The invention is also directed to isolated antibodies that bind to an epitope on a polypeptide encoded by any of the nucleic acid molecules described above.

The invention also includes kits comprising any of the pharmaceutical compositions described above.

The invention further provides a method for producing an NOVX polypeptide by providing a cell containing an NOVX nucleic acid, *e*.*g*., a vector that includes an NOVX nucleic acid, and culturing the cell under conditions sufficient to express the NOVX polypeptide encoded by the nucleic acid. The expressed NOVX polypeptide is then recovered from the cell. Preferably, the cell produces little or no endogenous NOVX polypeptide. The cell can be, *e.g.,* a prokaryotic cell or eukaryotic cell.

The invention is also directed to methods of identifying an NOVX polypeptide or nucleic acid in a sample by contacting the sample with a compound that specifically binds to the polypeptide or nucleic acid, and detecting complex formation, if present.

The invention further provides methods of identifying a compound that modulates the activity of an NOVX polypeptide by contacting an NOVX polypeptide with a compound and determining whether the NOVX polypeptide activity is modified.

The invention is also directed to compounds that modulate NOVX polypeptide activity identified by contacting an NOVX polypeptide with the compound and determining whether the compound modifies activity of the NOVX polypeptide, binds to the NOVX polypeptide, or binds to a nucleic acid molecule encoding an NOVX polypeptide.

In another aspect, the invention provides a method of determining the presence of or predisposition of an NOVX-associated disorder in a subject. The method includes providing a sample from the subject and measuring the amount of NOVX polypeptide in the subject sample. The amount of NOVX polypeptide in the subject sample is then compared to the amount of NOVX polypeptide in a control sample. An alteration in the amount of NOVX polypeptide in the subject protein sample relative to the amount of NOVX polypeptide in the control protein sample indicates the subject has a tissue proliferation-associated condition. A control sample is preferably taken from a matched individual, *i*.*e*., an individual of similar age, sex, or other general condition but who is not suspected of having a tissue proliferation-associated condition. Alternatively, the control sample may be taken from the subject at a time when the subject is not suspected of having a tissue proliferation-associated disorder. In some embodiments, the NOVX is detected using an NOVX antibody.

In a further aspect, the invention provides a method of determining the presence of or predisposition of an NOVX-associated disorder in a subject. The method includes providing a nucleic acid sample, *e*.*g*., RNA or DNA, or both, from the subject and measuring the amount of the NOVX nucleic acid in the subject nucleic acid sample. The amount of NOVX nucleic acid sample in the subject nucleic acid is then compared to the amount of an NOVX nucleic acid in a control sample. An alteration in the amount of NOVX nucleic acid in the sample relative to the amount of NOVX in the control sample indicates the subject has a tissue proliferation-associated disorder.

In a still further aspect, the invention provides a method of treating or preventing or delaying an NOVX-associated disorder. The method includes administering to a subject in which such treatment or prevention or delay is desired an NOVX nucleic acid, an NOVX polypeptide, or an NOVX antibody in an amount sufficient to treat, prevent, or delay a tissue proliferation-associated disorder in the subject.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences and their polypeptides. The sequences are collectively referred to as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table 1 provides a summary of the NOVX nucleic acids and their encoded polypeptides.

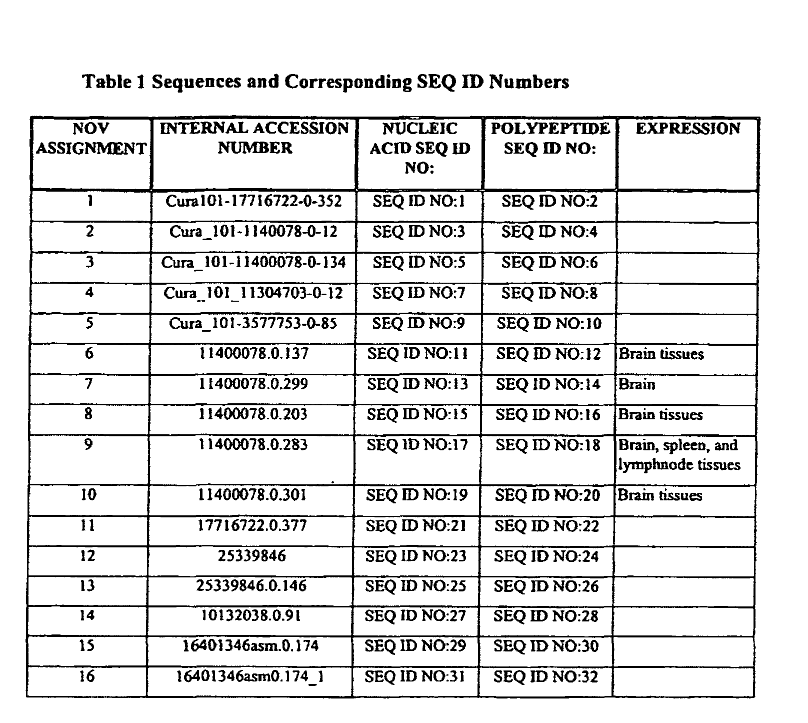

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

For example, NOV1 NOV4, NOV5, NOV7, NOV9-11, and NOV 13-16 all contain casein kinase II phosphorylation sites, which is charactertic of serine/threonine kinases. Thus, the NOV1 NOV4, NOV5, NOV7, NOV9-11, and NOV 13-16 nucleic acids and polypeptides, antibodies and related compounds according to the invention will be useful in therapeutic and diagnostic applications in serine/threonine kinase related disorders such as for example, cancer, *e*.*g*., Peutz-Jeghers syndrome, cellular proliferative disorders,and contraception.

Simarily, NOV2, NOV3, NOV6 and NOV8, is homologous to members of EGF-like super-family of proteins. Proteins currently known to contain one or more copies of an EGF-like pattern is large and varied, however, a common feature is that these repeats are found in the extracellular domain of membrane-bound proteins or in proteins known to be secreted. Proteins which contain EGF-like domains function in regulation of developmental stages, apoptosis, cell adhesion, growth migration, differentiation nucleic acid management, cell structure/motility, protein management, transcriptional regulation, signal transduction, metabolism and cell-to-cell interaction. Thus, the NOV2, NOV3, NOV 6and NOV8, nucleic acids and polypeptides, antibodies and related compounds according to the invention will be useful in therapeutic and diagnostic applications in cell proliferative disorders, *e*.*g*., cancer, inflammatory disorders, immune system disorders, cellular adhesion-related disorders.

Furthermore, NOV6, NOV7, NOV8, NOV9 and NOV10 polypeptides are isoforms of each other demonstrating sequence homology to fibrillin proteins and may have similar function. Fibrillin proteins EGF-like proteins ad have neen implicted in a variety of disease states, for example, genetically transmitted cardiovascular diseases, *e.g.,* hypertrophic cardiomyopathy, long-QT syndrome, and, marfan syndrome. Accordingly, NOV6, NOV7, NOV8, NOV9 or NOV10 a a nucleic acids and polypeptides, antibodies and related compounds according to the invention will be useful in therapeutic and diagnostic applications in these disorders.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit, *e*.*g*., differentiation, cellular proliferation, apoptosis and inflammation.

A NOVX nucleic acid may also be useful for detecting specific cell-types. For example a NOV6, NOV7 , NOV 8 and NOV 10 nucleic acid according to the invention can be present in different levels in the brain. According a NOV6, NOV7 , NOV 8 and NOV 10 nucleic acid can be used to detect brainn tissue. Simarily, A NOV9 nucleic acid according to the invention can be present in different level in lymphoid tissues, specifically lymphnodes. Accoriding a NOV9 nucleic acid can be used to detect lymphoid tissue.

Additional utilities for NOVX nucleic acids and polypeptides according to the invention are disclosed herein.

### NOV1

A NOV1 sequence according to the invention is a nucleic acid sequence encoding a polypeptide related serine/threonine kinase family of proteins. A NOV1 nucleic acid and polypeptide according to the invention includes the nucleic acid and encoded polypeptide sequence of a sequence named 17716722-0-352. A NOV1 nucleic acid and its encoded polypeptide includes the sequences shown in **Table 2**. The disclosed nucleic acid (SEQ ID NO: 1) is 791 bp nucleotides in length and contains an open reading frame (ORF) that begins with an initiation codon at nucleotides 222-224 and ends with a stop codon at nucleotides 681-683. The representative ORF includes a 170 amino acid polypeptide (SEQ ID NO: 2).

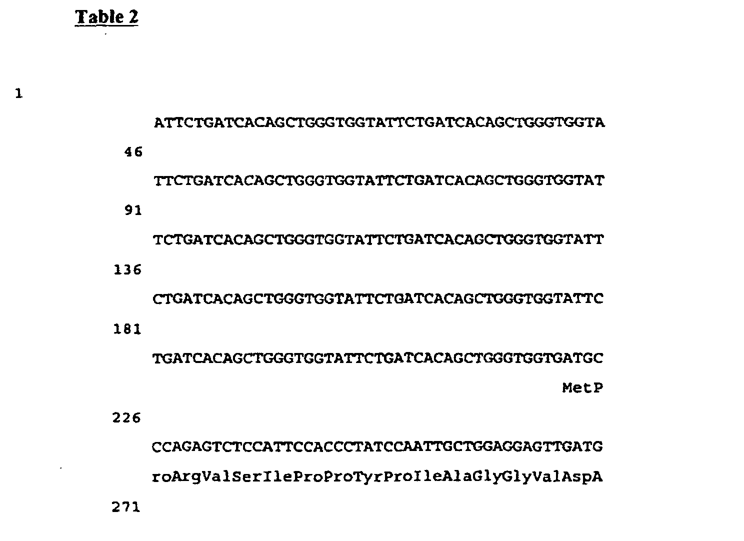

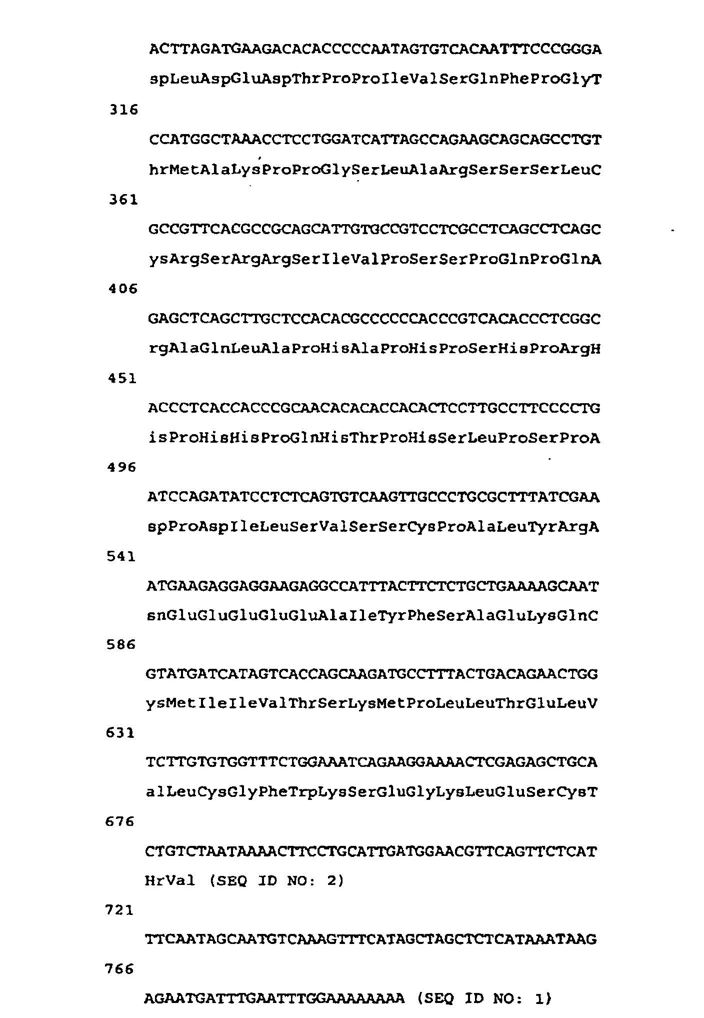

The encoded nucleotide has homology to the Sant Domain Protein SMRTR protein (GenBank Accession. No. AAD52614) which belongs to the aldehyde dehydrogenase family and contains at least forty four casein kinase II phosphorylation sites (a serine kinase). A search of the PROSITE database of protein families and domains confirmed that a NOV1 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33).

The amino acid sequence comprising the Casein kinase II phosphorylation site signature sequence includes amino acids 85-88 of SEQ ID NO: 2.

Based on its relatedness to the Sant Domain Protein SMRTR protein and the presence of a casein kinase II phosphorylation site, the NOV1 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV2

A NOV2 sequence according to the invention is a nucleic acid sequence encoding a polypeptide containing EGF-like domains. A NOV2 nucleic acid and polypeptide according to the invention includes the nucleic acid and encoded polypeptide sequence of a sequence named 1140078-0-12. A NOV2 nucleic acid and its encoded polypeptide includes the sequences shown in Table 3. The disclosed nucleic acid (SEQ ID NO: 3) is 2011 **bp** nucleotides in length and contains an open reading frame (ORF) that begins with an initiation codon at nucleotides 877-879 and ends with a stop codon at nucleotides 1822-1824. The representative ORF includes a 315 amino acid polypeptide (SEQ ID NO: 4) with a predicted molecular weight of 63327 Da. A NOV2 polypeptide is predicted by PSORT program to localize extracellularly with a certainty of 0.3700. The programs PSORT and SignalP predict that there is may be a signal peptide with the most likely cleavage occurring between residues 21 and 22.

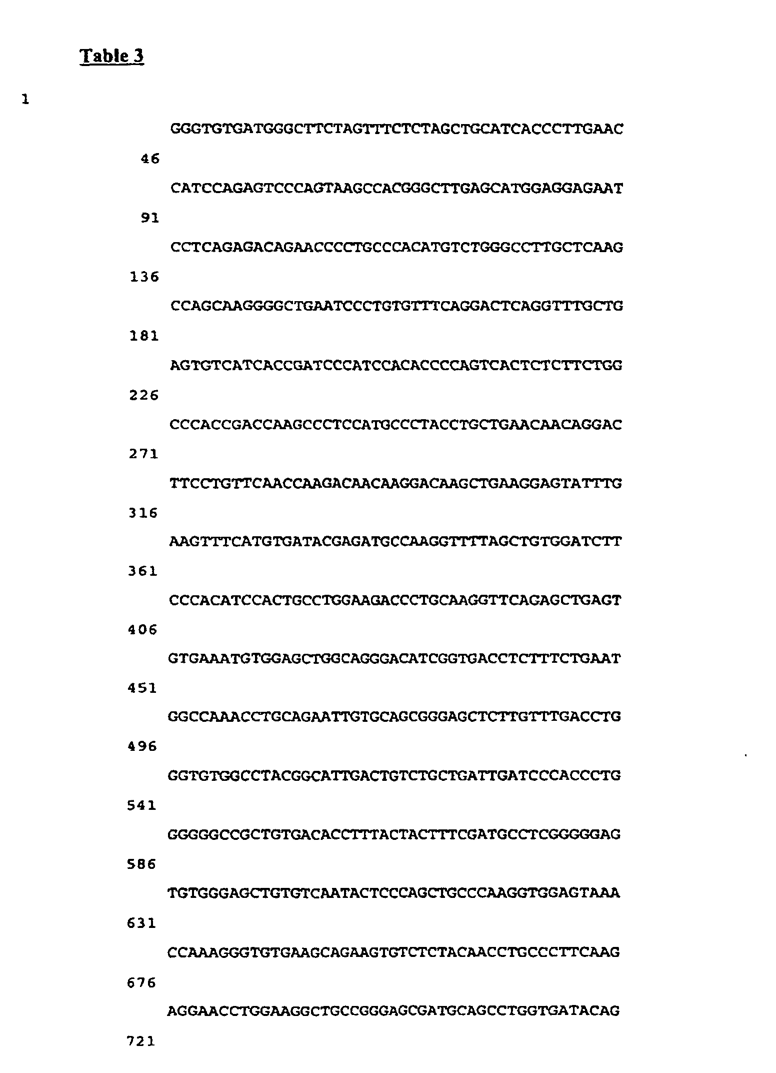

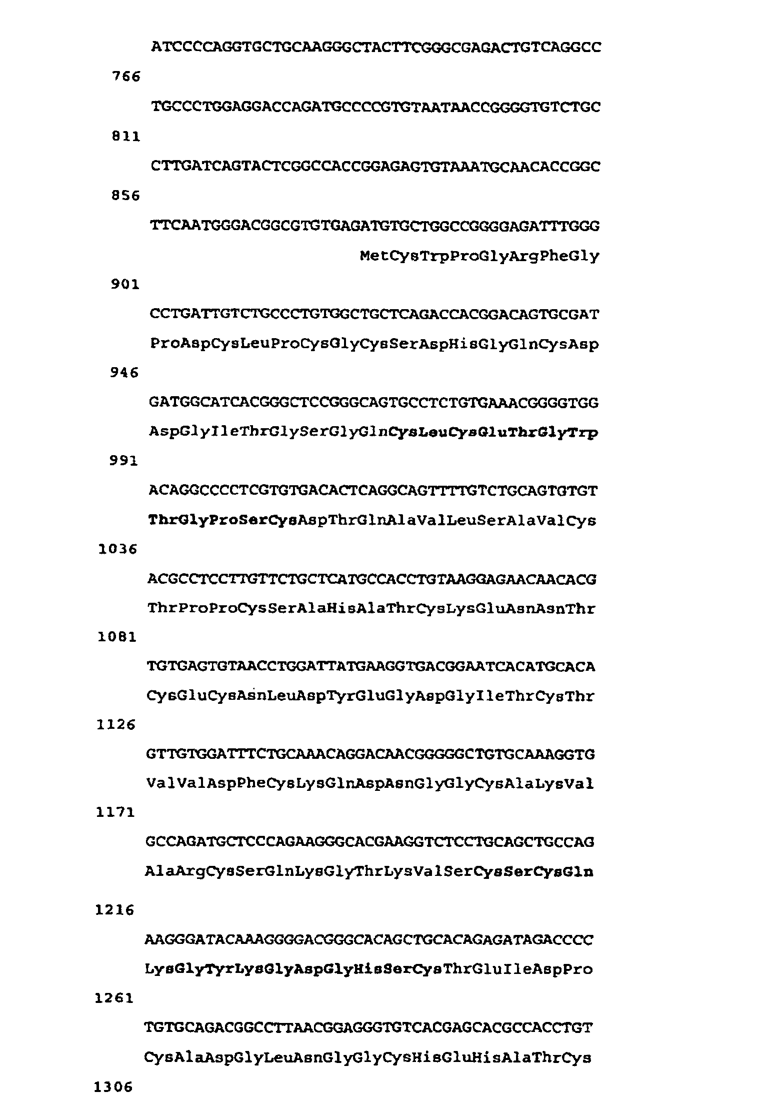

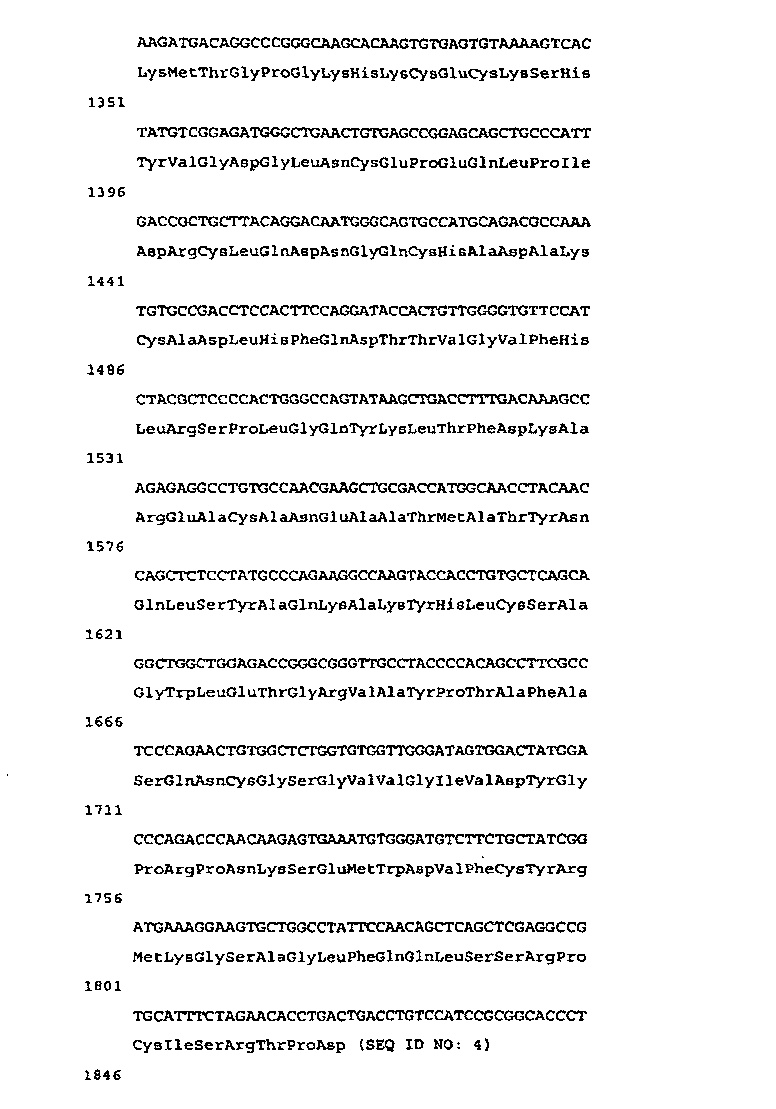

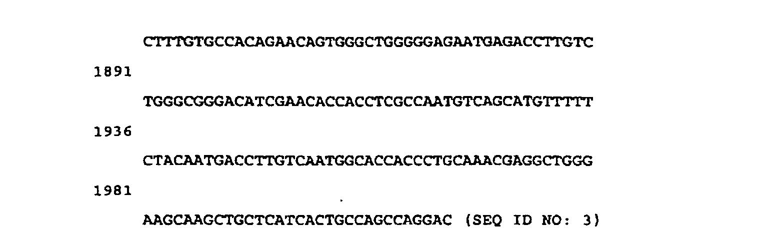

The encoded nucleotide has homology (approximately 62% identity) human mRNA for KIAA0246 (GenBank Accession. No. D87433). Similarly, the encoded polypeptide has homology (approximately 51% identity) KIAA0246 protein, which contains multiple EGF-like domains (GenBank Accession. No. Q93072). A search of the PROSITE database of protein families and domains confirmed that a NOV2 polypeptide is a member of the EGF-like family which is defined by polypeptides containing a stretch of highly conserved amino acid residues:
C-x-C-x(5)-G-x(2)-C (EGF-like domain signature sequence I; SEQ ID NO: 34) and
C-x-C-x(2)-[GP]-[FYW]-x(4,8)-C (EGF-like domain signature sequence 2; SEQ ID NO: 35)

The amino acid sequence comprising the EGF-like 1 signature sequence includes amino acids 32-42 of SEQ ID NO: 4 (illustrated by bold in SEQ ID NO: 4, Table 3). The amino acid sequence comprising the EGF-like 2 signature sequence includes amino acids 110-123 of SEQ ID NO: 4.

Proteins currently known to contain one or more copies of an EGF-like pattern is large and varied, however, a common feature is that these repeats are found in the extracellular domain of membrane-bound proteins or in proteins known to be secreted. Proteins which contain EGF-like domains function in regulation of developmental stages, apoptosis, cell adhesion, growth migration, differentiation nucleic acid management, cell structure/motility, protein management, transcriptional regulation, signal transduction, metabolism and cell-to-cell interaction.

Based on its relatedness to the KIAA0246 and the presence of two EGF-like signature sequence, the NOV2 protein is a novel member of the EGF-like domain family. The discovery of molecules related to EGF-like proteins satisfies a need in the art by providing new diagnostic or therapeutic compositions useful in the treatment of disorders associated with alterations in the expression of members of EGF- like proteins. Nucleic acids, polypeptides, antibodies, and other compositions of the present invention are useful in a variety of diseases and pathologies, including by way of nonlimiting example, cell proliferative disorders, *e*.*g*., cancer, inflammatory disorders, immune system disorders, cellular adhesion-related disorders

### NOV3

A NOV3 sequence according to the invention is a nucleic acid sequence encoding a polypeptide containing EGF-like domains. A NOV3 nucleic acid and polypeptide according to the invention includes the nucleic acid and encoded polypeptide sequence of a sequence named 1140078-0-134. A NOV nucleic acid and its encoded polypeptide includes the sequences shown in Table 4. The disclosed nucleic acid (SEQ ID NO: 5) is 1804 bp nucleotides in length and contains an open reading frame (ORF) that begins with an initiation codon at nucleotides 877-879 and ends with a stop codon at nucleotides 1609-1611. The representative ORF includes a 244 amino acid polypeptide (SEQ ID NO: 6). A NOV3 polypeptide is predicted by PSORT program to localize extracellularly with a certainty of 0.3700. The programs PSORT and SignalP predict that there is may be a signal peptide with the most likely cleavage occurring between residues 21 and 22.

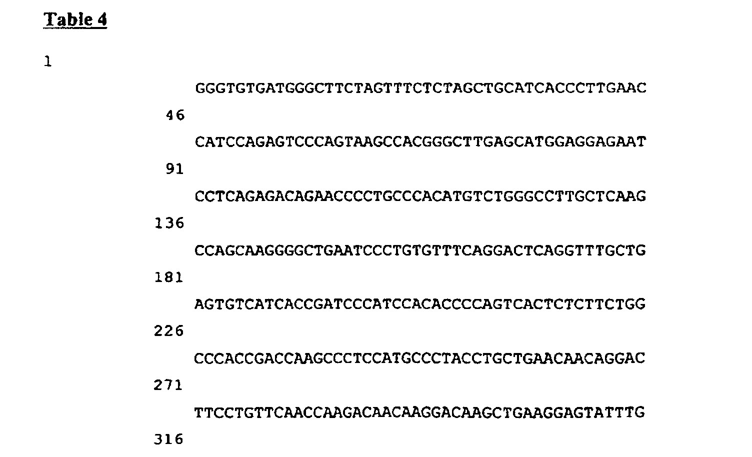

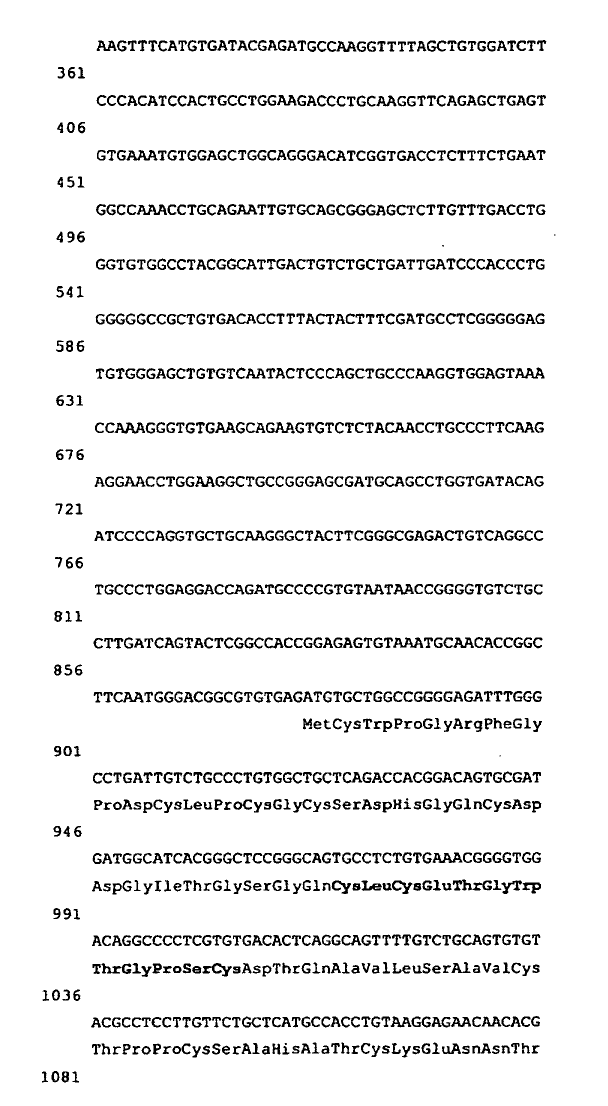

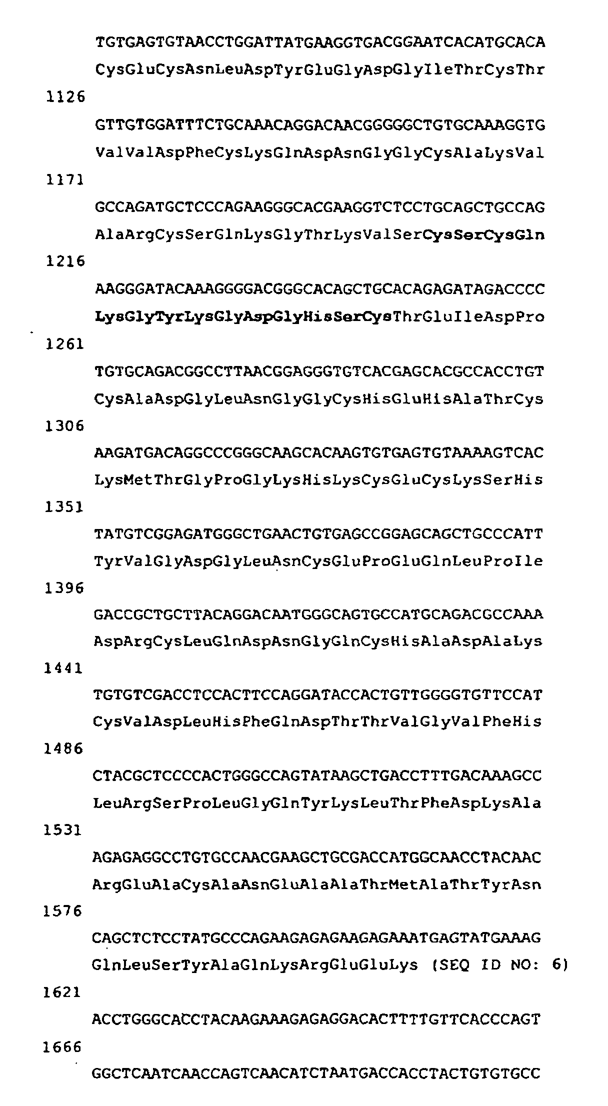

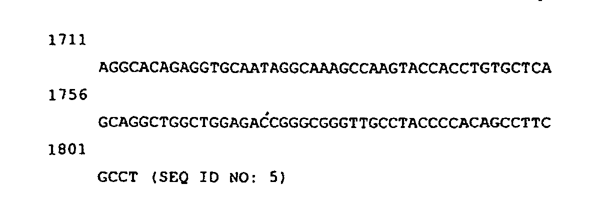

The encoded nucleotide has homology (approximately 62% identity) human mRNA for KIAA0246 (GenBank Accession. No. D87433). Similarly, the encoded polypeptide has homology (approximately 52% identity) KIAA0246 protein, which contains multiple EGF-like domains (GenBank Accession. No. Q93072). A search of the PROSITE database of protein families and domains confirmed that a NOV3 polypeptide is a member of the EGF-like family which is defined by polypeptides containing a stretch of highly conserved amino acid residues:
C-x-C-x(5)-G-x(2)-C (EGF-like domain signature sequence 1; SEQ ID NO: 34) and
C-x-C-x(2)-[GP]-[FYW]-x(4,8)-C (EGF-like domain signature sequence 2; SEQ ID NO: 35)

The amino acid sequence comprising the EGF-like I signature sequence includes amino acids 32-42 of SEQ ID NO: 6 (illustrated by bold in SEQ ID NO: 6, Table 4). The amino acid sequence comprising the EGF-like 2 signature sequence includes amino acids 110-123 of SEQ ID NO: 6.

Proteins currently known to contain one or more copies of an EGF-like pattern is large and varied, however, a common feature is that these repeats are found in the extracellular domain of membrane-bound proteins or in proteins known to be secreted. Proteins which contain EGF-like domains function in regulation of developmental stages, apoptosis, cell adhesion, growth migration, differentiation nucleic acid management, cell structure/motility, protein management, transcriptional regulation, signal transduction, metabolism and cell-to-cell interaction.

Based on its relatedness to the KIAA0246 and the presence of two EGF-like signature sequence, the NOV2 protein is a novel member of the EGF-like domain family. The discovery of molecules related to EGF-like proteins satisfies a need in the art by providing new diagnostic or therapeutic compositions useful in the treatment of disorders associated with alterations in the expression of members of EGF- like proteins. Nucleic acids, polypeptides, antibodies, and other compositions of the present invention are useful in a variety of diseases and pathologies, including by way of nonlimiting example, those involving cell proliferative disorders, *e*.*g*., cancer, inflammatory disorders, immune system disorders, cellular adhesion-related disorders **NOV4**

A NOV4 sequence according to the invention is a nucleic acid sequence encoding a polypeptide of the serine/threonine kinase family of proteins. A NOV4 nucleic acid and polypeptide according to the invention includes the nucleic acid and encoded polypeptide sequence of a sequence named 11304703-0-12. A NOV4 nucleic acid and its encoded polypeptide includes the sequences shown in Table 5. The disclosed nucleic acid (SEQ ID NO: 7) is 1450 bp nucleotides in length and contains an open reading frame (ORF) that begins with an initiation codon at nucleotides 173-175 and ends with a stop codon at nucleotides 848-850. The representative ORF includes a 225 amino acid polypeptide (SEQ ID NO: 8).

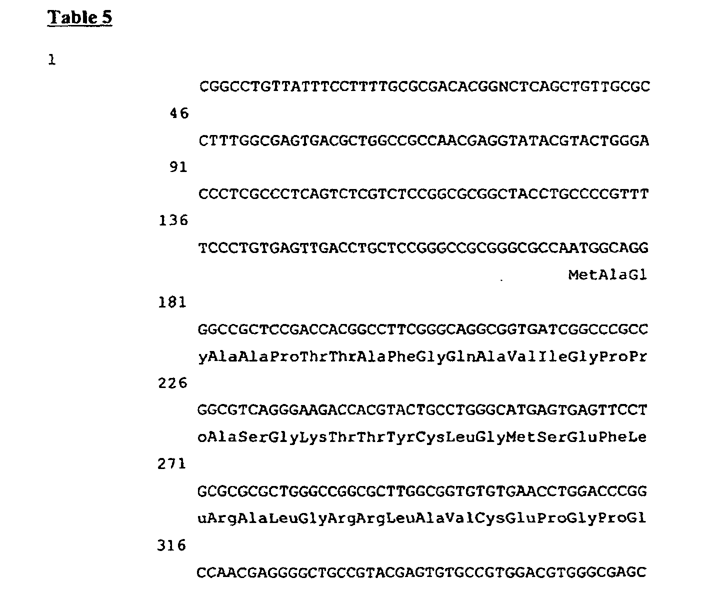

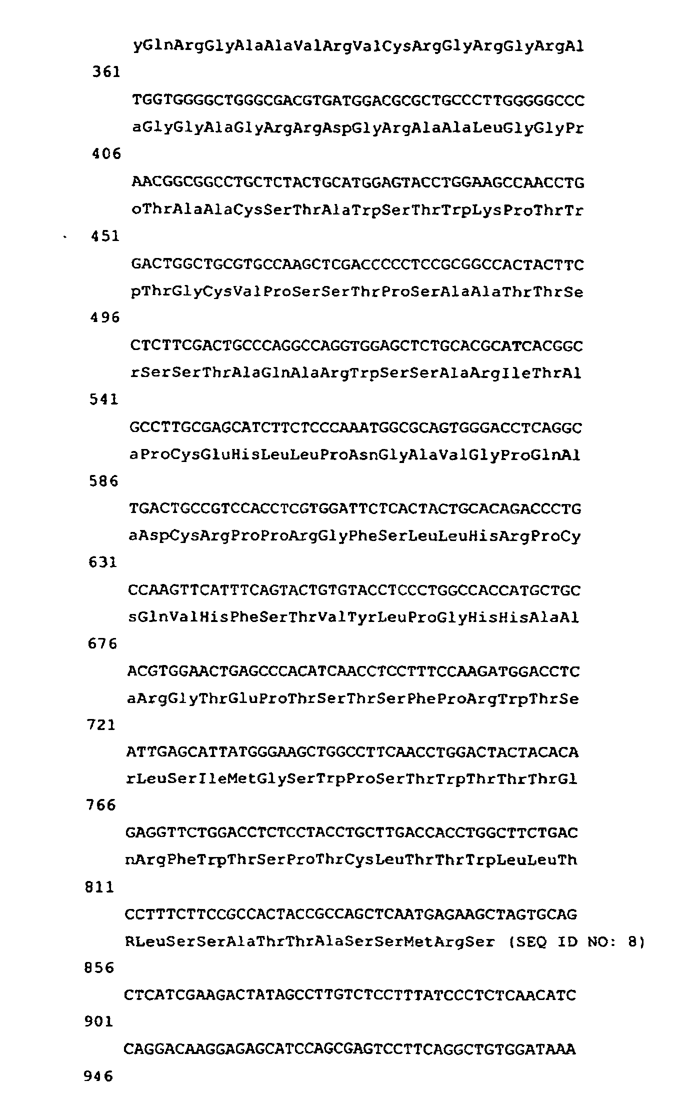

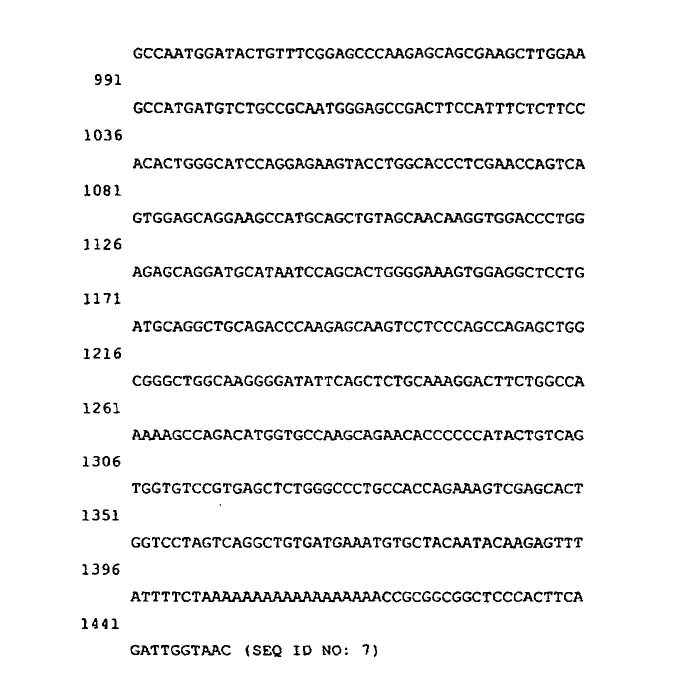

The encoded nucleotide has homology the homo sapiens HTRA serine protease gene (GenBank Accession. No. AF157623). Similarly, the encoded polypeptide has homology baker's yeast p protein. (GenBank Accession. No. Q08726). A search of the PROSITE database of protein families and domains confirmed that a NOV4 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising the Casein kinase II phosphorylation site signature sequence includes amino acids 114-117 of SEQ ID NO: 8 (illustrated by bold in SEQ ID NO: 8, Table 5).

Based on its relatedness to the homo sapiens HTRA serine protease gene, baker's yeast proetin and the presence of a casein kinase II phosphorylation site, the NOV4 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV5

A NOV5 sequence according to the invention is a nucleic acid sequence encoding a polypeptide of the serine/threonine kinase family of proteins. A NOV5 nucleic acid and polypeptide according to the invention includes the nucleic acid and encoded polypeptide sequence of a sequence named 3577753-085. A NOV5 nucleic acid and its encoded polypeptide includes the sequences shown in Table 6. The disclosed nucleic acid (SEQ ID NO: 9) is 1324 bp nucleotides in length and contains an open reading frame (ORF) that begins with an initiation codon at nucleotides 69-71 and ends with a stop codon at nucleotides 663-665. The representative ORF includes a 198 amino acid polypeptide (SEQ ID NO: 10).

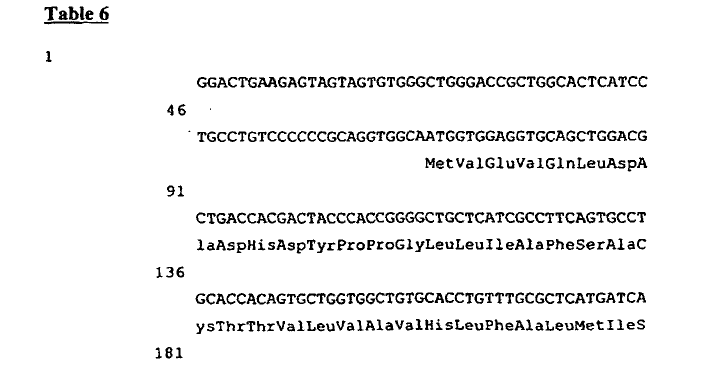

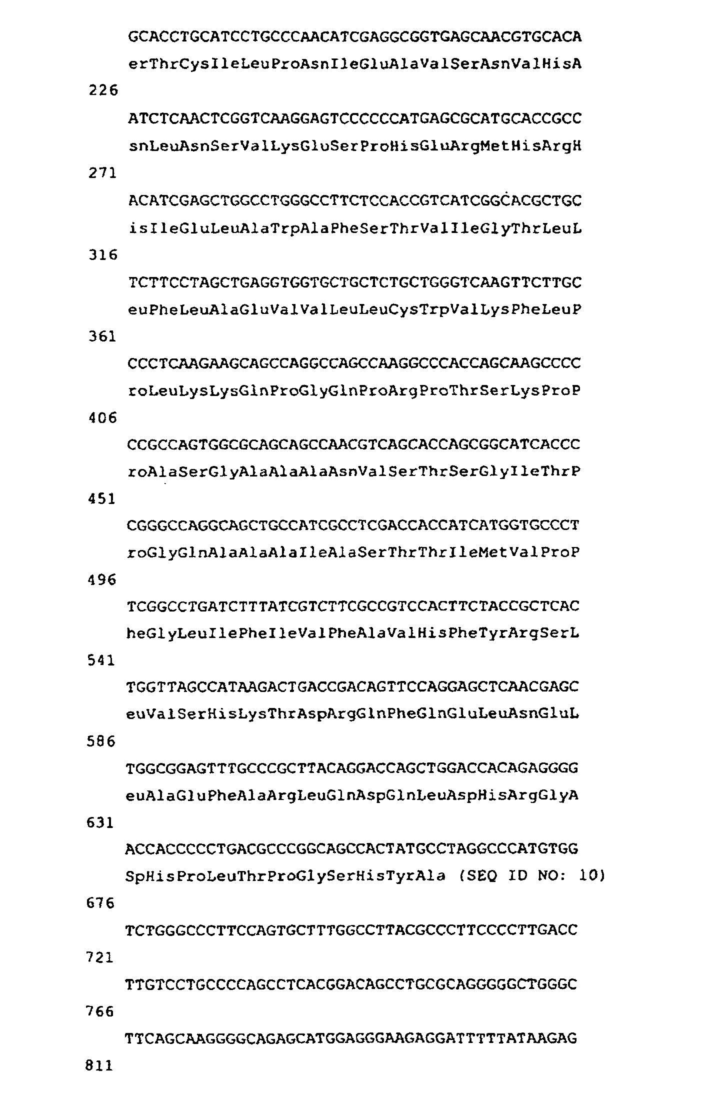

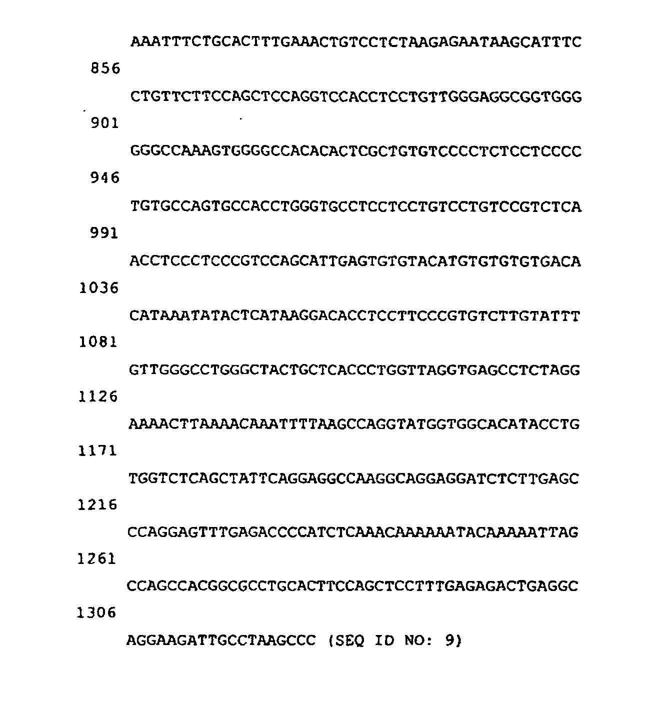

The encoded nucleotide has homology Drosophilia melangoster F protein (GenBank Accession. No. AF188634). Similarly, the encoded polypeptide has homology fruit fly protein (GenBank Accession. No. AAFo1457). A search of the PROSITE database of protein families and domains confirmed that a NOV5 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising two Casein kinase II phosphorylation site signature sequences includes amino acids 53-56 and 57-60 of SEQ ID NO: 10.

Based on its relatedness to the drosophilia melangoster F protein and fruit fly protein and the presence of two casein kinase II phosphorylation sites the NOV5 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV6

A NOV6 sequence according to the invention is a nucleic acid sequence encoding a polypeptide containing EGF-like domains. A NOV6 nucleic acid and polypeptide according to the invention includes the nucleic acid and encoded polypeptide sequence of a sequence named 1140078-0-137. A NOV6 nucleic acid and its encoded polypeptide includes the sequences shown in Table 9. The disclosed nucleic acid (SEQ ID NO: 11) is 2512 bp nucleotides in length. The representative ORF includes a 244 amino acid polypeptide (SEQ ID NO: 12) with a molecular weighy of 73167.6 daltons. A NOV6 polypeptide is predicted by PSORT program to localize to the cytoplasm with a certainty of 0.6500.

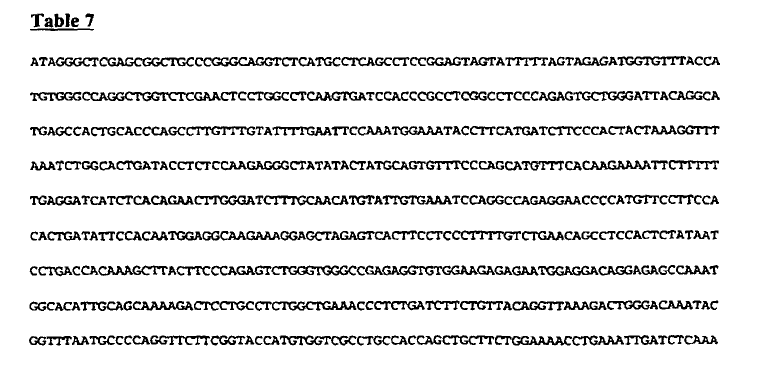

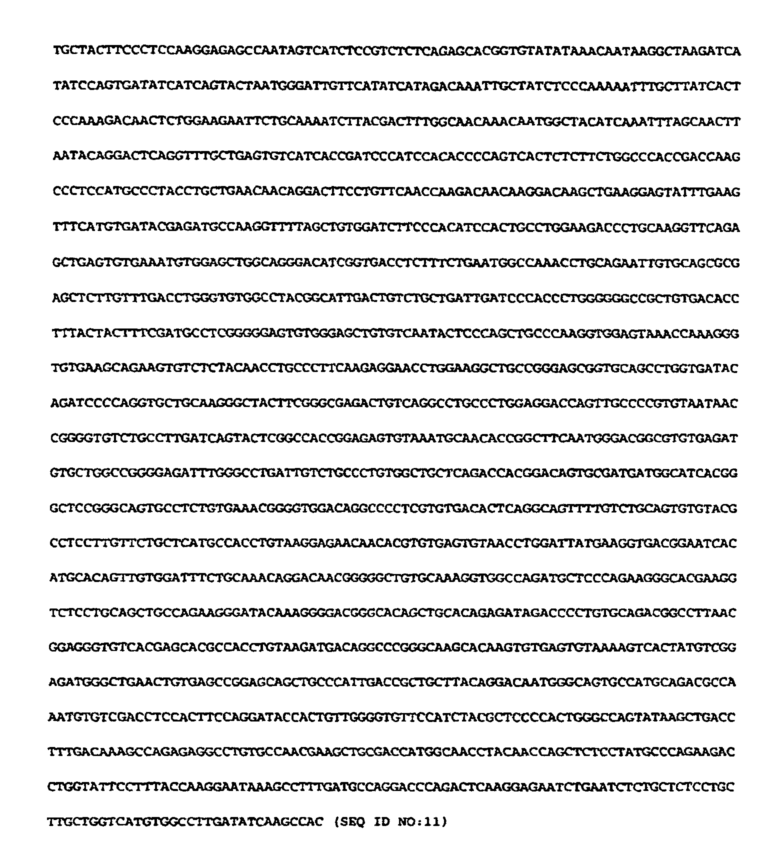

MEARKELESLPPFCLNSLHSIILTTKLTSQSLGGPRGVEERMEDRRAKWHIAAKDSCLWLKPSDLLLQVKDWDKYGLMPQVLRYHVVACHQLLLE
NLKLISNATSLQGEPIVISVSQSTVYINNKAKIISSDIISTNGIVHIIDKLLSPKNLLITPKDNSGRILQNLTTLATNNGYIKFSNLIQDSGLLS
VITDPIHTPVTLFWPTDQALHALPAEQQDFLFNQDNKDKLKEYLKFHVIRDAKVLAVDLPTSTAWKTLQGSELSVKCGAGRDIGDLFLNGQTCRI
VQRELLFDLGVAYGIDCLLIDPTLGGRCDTFTTFDASGECGSCVNTPSCPRWSKPKGVKQKCLYNLPFKRNLEGCRERCSLVIQIPRCCKGYFGR
DCQACPGGPVAPCNNRGVCLDQYSATGECKCNTGFNGTACEMCWPGRFGPDCLPCGCSDHGQCDDGITGSGQCLCETGWTGPSCDTQAVLSAVCT
PPCSAHATCKENNTCECNLDYEGDGITCTVVDFCKQDNGGCAKVARCSQKGTKVSCSCQKGYKGDGHSCTEIDPCADGLNGGCHEHATCKMTGPG
KHKCECKSHYVGDGLNCEPEQLPIDRCLQDNGQCHADAKCVDLHFQDTTVGVFHLRSPLGQYKLTFDKAREACANEAATMATYNQLSYAQKTWYS
FTKE (SEQ ID NO: 12)

The encoded polypeptide has homology (approximately 99% identity) hypothetical 115.7 kD human protein (GenBank Accession. No CAB61358), which contains multiple EGF-like domains . A search of the PROSITE database of protein families and domains confirmed that a NOV2 polypeptide is a member of the EGF-like family which is defined by polypeptides containing a stretch of highly conserved amino acid residues:
C-x-C-x(5)-G-x(2)-C (EGF-like domain signature sequence 1; SEQ ID NO: 34)
C-x-C-x(2)-[GP]-[FYW]-x(4,8)-C (EGF-like domain signature sequence 2; SEQ ID NO: 35) and
C-x(1,2)-C-x(5)-G-x(2)-C-x(2)-C-x(3,4)-[FYW]-x(3,15)-C (Laminin-type EGF-like (LE) domain signature; SEQ ID NO: 36)

The amino acid sequence comprising the EGF-like 1 signature sequence includes amino acids 409 -420 and 453-464 of SEQ ID NO: 12. The amino acid sequence comprising the EGF-like 2 signature sequence includes amino acids 409-423 and 531-544 of SEQ ID NO: 12. These signature sequences are illustrated by bold in SEQ ID NO: 12, Table 7. The amino acid sequence comprising the laminin-type EGF-Like (LE) domain signature sequence includes amino acids 409-443 of SEQ ID NO: 12 (illustrated by underline in SEQ ID NO: 12, Table 7).

Proteins currently known to contain one or more copies of an EGF-like pattern is large and varied, however, a common feature is that these repeats are found in the extracellular domain of membrane-bound proteins or in proteins known to be secreted. Proteins which contain EGF-like domains function in regulation of developmental stages, apoptosis, cell adhesion, growth migration, differentiation nucleic acid management, cell structure/motility, protein management, transcriptional regulation, signal transduction, metabolism and cell-to-cell interaction.

Based on its relatedness to the KIAA0246 and the presence of two EGF-like signature sequence, the NOV2 protein is a novel member of the EGF-like domain family. The discovery of molecules related to EGF-like proteins satisfies a need in the art by providing new diagnostic or therapeutic compositions useful in the treatment of disorders associated with alterations in the expression of members of EGF- like proteins. Nucleic acids, polypeptides, antibodies, and other compositions of the present invention are useful in a variety of diseases and pathologies, including by way of nonlimiting example, those involving cell proliferative disorders, *e*.*g*., cancer, inflammatory disorders, immune system disorders, cellular adhesion-related disorders

### NOV7

A NOV7 sequence according to the invention is a nucleic acid sequence encoding a polypeptide of the serine/threonine kinase family of proteins. A NOV7 nucleic acid and polypeptide according to the invention includes the nucleic acid and encoded polypeptide sequence of a sequence named 11400078.0.299. A NOV7 nucleic acid and its encoded polypeptide includes the sequences shown in Table 8. The disclosed nucleic acid (SEQ ID NO: 13) is 1624 bp nucleotides in length. The representative ORF includes a 381 amino acid polypeptide (SEQ ID NO: 14). SignalPep and PSort search programs predictes localization of NOV7 to the cytoplasm with a certainty=0.6500). The predicted molecular weight of NOV7 protein is 42565.8 daltons.

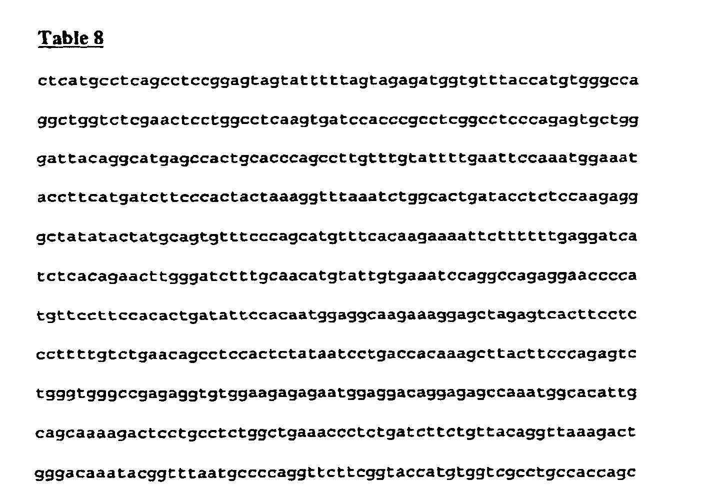

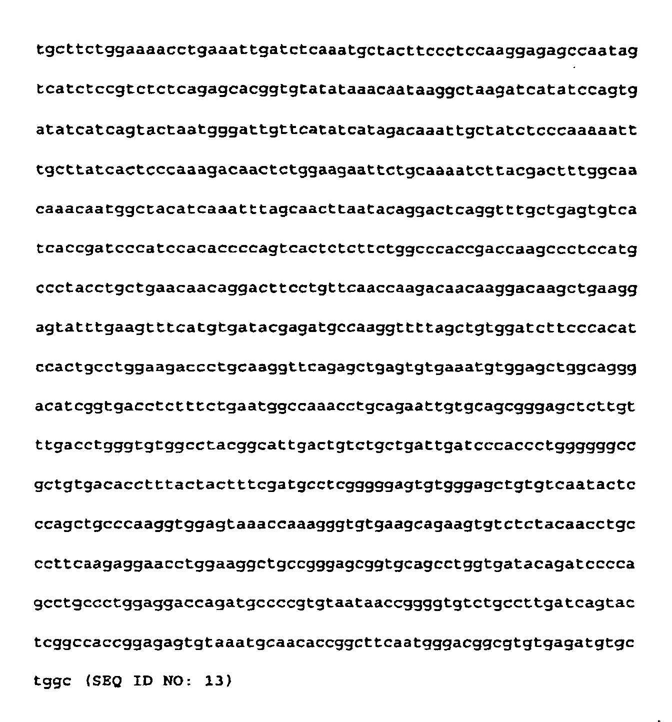

MEARKELESLPPFCLNSLHSIILTTKLTSQSLGGPRGVEERMEDRRAKWHIAAKDSCLWLKPSDLLLQVK
DWDKYGLMPQVLRYHVVACHQLLLENLKLISNATSLQGEPIVISVSQSTVYINNKAKIISSDIISTNGIVHIIDKL
LSPKNLLITPKDNSGRILQNLTTLATNNGYIKFSNLIQDSGLLSVITDPIHTPVTLFWPTDQALHALPAEQQDFLF
NQDNKDKLKEYLKFHVIRDAKVLAVDLPTSTAWKTLQGSELSVKCGAGRDIGDLFLNGQTCRIVQRELLFDLGVAY
GIDCLLIDPTLGGRCDTFTTFDASGECGSCVNTPSCPRWSKPKGVKQKCLYNLPFKRNLEGCRERCSLVIQIPSLP
WRTRCPV (SEQ ID NO:14)

The encoded polypeptide has homology (approximately 99% identity) hypothetical 115.7 kD human protein (GenBank Accession. No CAB61358), which contains at least eleven casein kinase II phosphorylation sites (a serine kinase). A search of the PROSITE database of protein families and domains confirmed that a NOV7 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising two Casein kinase II phosphorylation site signature sequences includes amino acids 155-158 and 317-320 of SEQ ID NO: 14 (illustrated by bold in SEQ ID NO: 14, Table 8)

Based on its relatedness to the homo sapiens hypothetical 115.7 kD human protein and the presence of twocasein kinase II phosphorylation sites, the NOV7 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV8

A NOV sequence according to the invention is a nucleic acid sequence encoding a polypeptide containing EGF-like domains. A NOV8 nucleic acid and polypeptide according to the invention includes the nucleic acid and encoded polypeptide sequence of a sequence named 1140078-0-203. A NOV8 nucleic acid and its encoded polypeptide includes the sequences shown in Table 9. The disclosed nucleic acid (SEQ ID NO: 15) is 2483 bp nucleotides in length. The representative ORF includes a 669 amino acid polypeptide (SEQ ID NO: 16) with a molecular weight of 73183.5 daltons. A NOV8 polypeptide is predicted by PSORT program to localize to the cytoplasm with a certainty of 0.6500.

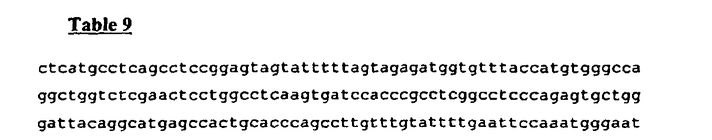

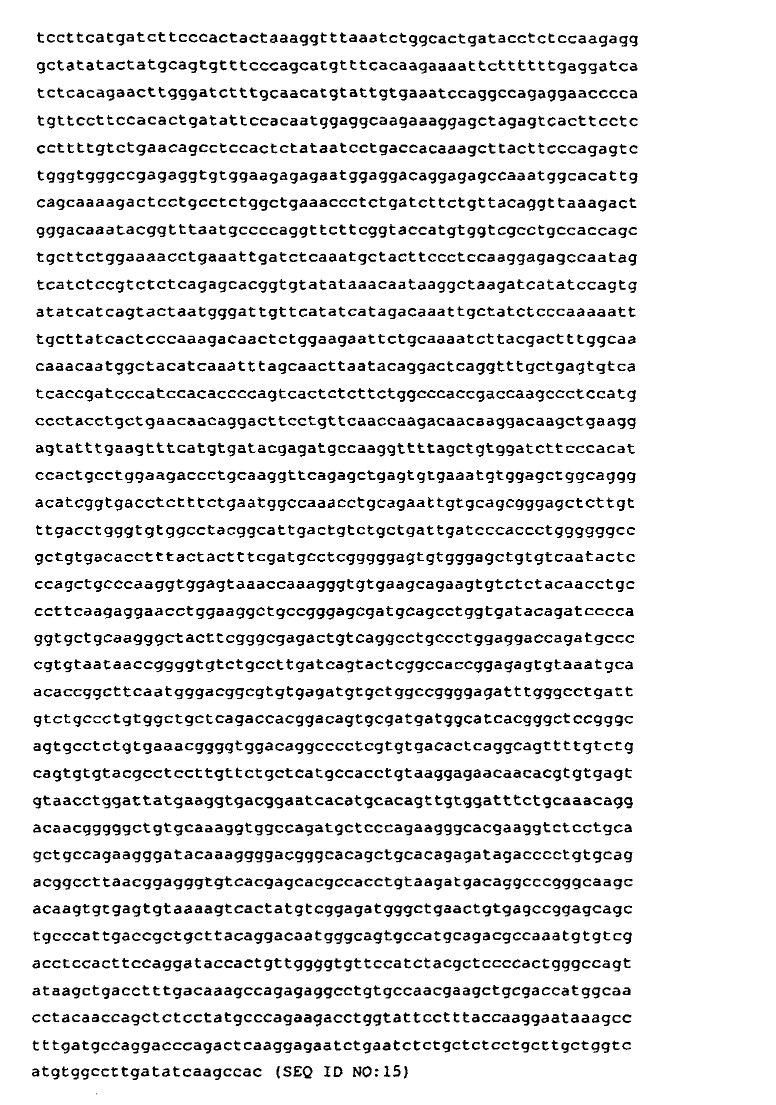









 (SEQ ID NO:
16)

The encoded polypeptide has homology (approximately 99% identity) hypothetical 115.7 kD human protein (GenBank Accession. No CAB61358), which contains multiple EGF-like domains . A search of the PROSITE database of protein families and domains confirmed that a NOV2 polypeptide is a member of the EGF-like family which is defined by polypeptides containing a stretch of highly conserved amino acid residues:
C-x-C-x(5)-G-x(2)-C (EGF-like domain signature sequence 1; SEQ ID NO: 34)
C-x-C-x(2)-[GP]-[FYW]-x(4,8)-C (EGF-like domain signature sequence 2; SEQ ID NO: 35) and
C-x(1,2)-C-x(5)-G-x(2)-C-x(2)-C-x(3,4)-[FYW]-x(3,15)-C (Laminin-type EGF-like (LE) domain signature; SEQ ID NO: 36)

The amino acid sequence comprising the EGF-like 1 signature sequence includes amino acids 409 -420 and 453-464 of SEQ ID NO: 16. The amino acid sequence comprising the EGF-like 2 signature sequence includes amino acids 531-544 of SEQ ID NO: 16. These signature sequences are illustrated by bold in SEQ ID NO: 16, Table 9. The amino acid sequence comprising the laminin-type EGF-like (LE) domain signature sequence includes amino acids 409-443 of SEQ ID NO: 16 (illustrated by underline in SEQ ID NO: 16, Table 9).

Proteins currently known to contain one or more copies of an EGF-like pattern is large and varied, however, a common feature is that these repeats are found in the extracellular domain of membrane-bound proteins or in proteins known to be secreted. Proteins which contain EGF-like domains function in regulation of developmental stages, apoptosis, cell adhesion, growth migration, differentiation nucleic acid management, cell structurelmotility, protein management, transcriptional regulation, signal transduction, metabolism and cell-to-cell interaction.

Based on its relatedness to the KIAA0246 and the presence of two EGF-like signature sequence, the NOV2 protein is a novel member of the EGF-like domain family. The discovery of molecules related to EGF-like proteins satisfies a need in the art by providing new diagnostic or therapeutic compositions useful in the treatment of disorders associated with alterations in the expression of members of EGF- like proteins. Nucleic acids, polypeptides, antibodies, and other compositions of the present invention are useful in a variety of diseases and pathologies, including by way of nonlimiting example, those involving cell proliferative disorders, *e*.*g*., cancer, inflammatory disorders, immune system disorders, cellular adhesion-related disorders.

### NOV9

A NOV9 sequence according to the invention is a nucleic acid sequence encoding a polypeptide of the serine/threonine kinase family of proteins. A NOV9 nucleic acid and polypeptide according to the invention includes the nucleic acid and encoded polypeptide sequence of a sequence named 1140078-0-283. A NOV9 nucleic acid and its encoded polypeptide includes the sequences shown in Table 10. The disclosed nucleic acid (SEQ ID NO: 17) is 3625 bp nucleotides in length. The representative ORF includes a 545 amino acid polypeptide (SEQ ID NO: 18), with a molecular weight of 60038.8 daltons. A NOV9 polypeptide is predicted by PSORT program to localize to the cytoplasm with a certainty of 0.6500.

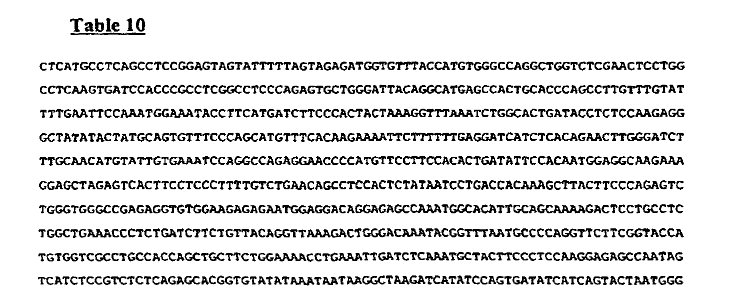

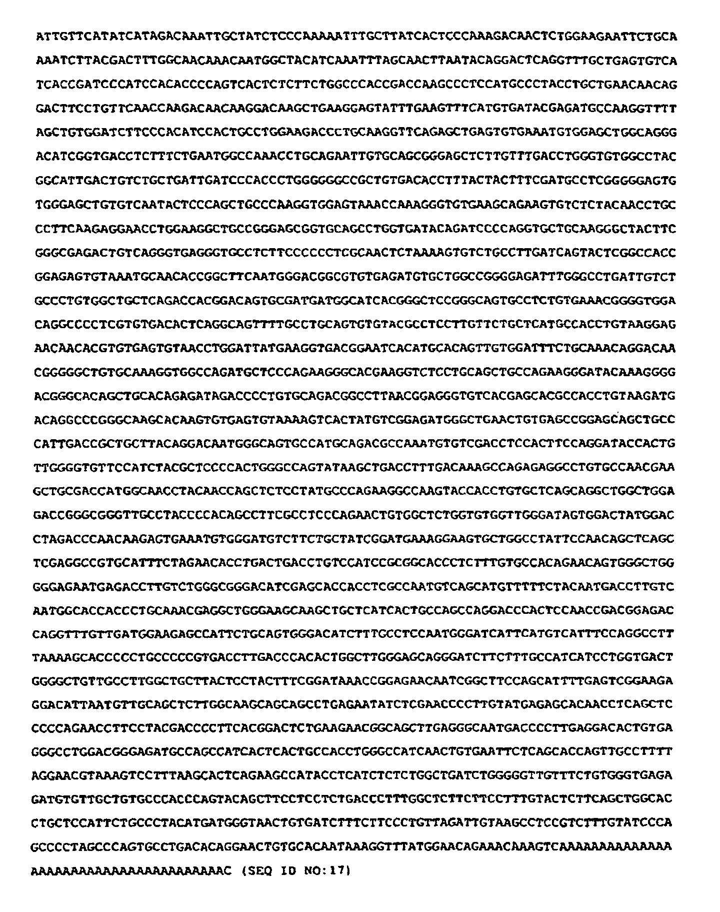

MEARKELESLPPFCLNSLHSIILTTKLTSQSLGGPRGVEERMEDRRAKWHIAAKDSCLWLKPSDLLLQVKDWDKYGLMPQVLRYHVVACHQLLLE
NLKLISNATSLQGEPIVISVSQSTVYINNKAKIISSDIISTNGIVHIIDKLLSPKNLLITPKDNSGRILQNLTTLATNNGYIKFSNLIQDSGLLS
VITDPIHTPVTLFWPTDQALHALPAEQQDFLFNQDNKDKLKEYLKFHVIRDAKVLAVDLPTSTAWKTLQGSELSVKCGAGRDIGDLFLNGQTCRI
VQRELLFDLGVAYGIDCLLIDPTLGGRCDTFTTFDASGECGSCVNTPSCPRWSKPKGVKQKCLYNLPFKRNLEGCRERCSLVIQIPRCCKGYFGR
DCQGEGASSPLATLKVSALISTRPPESVNATPASMGRRVRCAGRGDLGLIVCPVAAQTTDSAMMASRAPGSASVKRGGQAPRVTLRQFCLQCVRL
LVLLMPPVRRTTRVSVTWIMKVTESHAQLWISANRTTGAVQRWPDAPRRARRSPAAARRDTKGTGTAAQR (SEQ ID NO:18)

The encoded polypeptide has homology (approximately 99% identity) hypothetical 115.7 kD human protein (GenBank Accession. No CAB61358), which contains at least eleven casein kinase II phosphorylation sites (a serine kinase). A search of the PROSITE database of protein families and domains confirmed that a NOV9 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising two Casein kinase II phosphorylation site signature sequences includes amino acids 155-158 and 317-320 of SEQ ID NO: 18 (illustrated by bold in SEQ ID NO:18, Table 10).

Based on its relatedness to hypothetical 115.7 kD human protein and the presence of two casein kinase II phosphorylation sites, the NOV9 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV10

A NOV10 sequence according to the invention is a nucleic acid sequence encoding a polypeptide of the serine/threonine kinase family of proteins. A NOV10 nucleic acid and polypeptide according to the invention includes the nucleic acid and encoded polypeptide sequence of a sequence named 11400078.0.301. A NOV10 nucleic acid and its encoded polypeptide includes the sequences shown in Table 11. The disclosed nucleic acid (SEQ ID NO:19) is 1577 bp nucleotides in length. The representative ORF includes a 334 amino acid polypeptide (SEQ ID NO:20). SignalPep and PSort search programs predictes localization of NOV10 to the cytoplasm with a certainty of 0.6500). The predicted molecular weight of NOV10 protein is 37170.4 daltons.

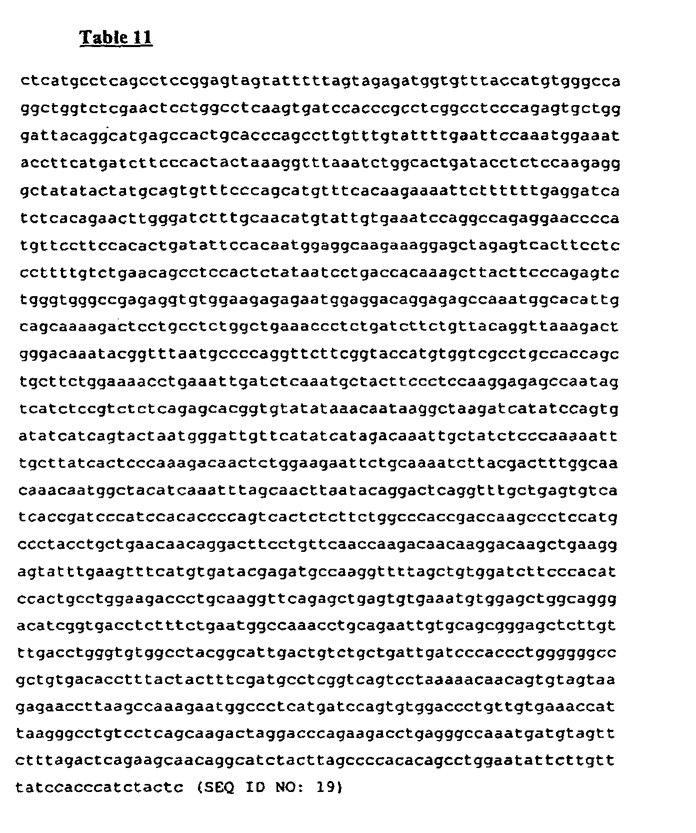

MEARKELESLPPFCLNSLHSIILTTKLTSQSLGGPRGVEERMEDRRAKWHIAAKDSCLWLKPSDLLLQVKDWDKYG
LMPQVLRYHVVACHQLLLENLKLISNATSLQGEPIVISVSQSTVYINNKAKIISSDIISTNGIVHIIDKLLSPKNL
LITPKDNSGRILQNLTTLATNNGYIKFSNLIQDSGLLSVITDPIHTPVTLFWPTDQALHALPAEQQDFLFNQDNKD
KLKEYLKFHVIRDAKVLAVDLPTSTAWKTLQGSELSVKCGAGRDIGDLFLNGQTCRIVQRELLFDLGVAYGIDCLL
IDPTLGGRCDTFTTFDASVSPKNNSVVREP (SEQ ID NO: 20)

The encoded polypeptide has homology (approximately 99% identity) hypothetical 115.7 kD human protein (GenBank Accession. No CAB61358), which contains at least eleven casein kinase II phosphorylation sites (a serine kinase). A search of the PROSITE database of protein families and domains confirmed that a NOV7 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising two Casein kinase II phosphorylation site signature sequences includes amino acids 155-158 and 317-320 ofSEQ ID NO: 20 (illustrated by bold in SEQ ID NO:20, Table 11)
Based on its relatedness to hypothetical 115.7 kD human protein and the presence of two casein kinase II phosphorylation sites, the NOV10 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV11

A NOV11 sequence according to the invention is a nucleic acid sequence encoding a polypeptide of the serine/threonine kinase family of proteins. A NOV1I nucleic acid and polypeptide according to the invention includes the nucleic acid and encoded polypeptide sequence of a sequence named 17716722.0.377. A NOV11 nucleic acid and its encoded polypeptide includes the sequences shown in Table 12. The representative ORF includes a 280 amino acid polypeptide (SEQ ID NO: 22). SignalPep and PSort search programs predictes localization of NOV10 to the cytoplasm with a certainty of 0.6500). The predicted molecular weight of NOV10 protein is 30359.7 daltons.

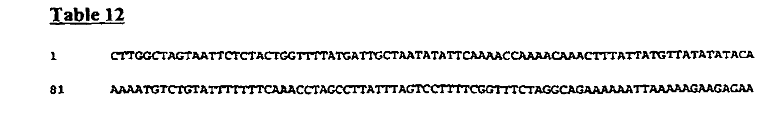

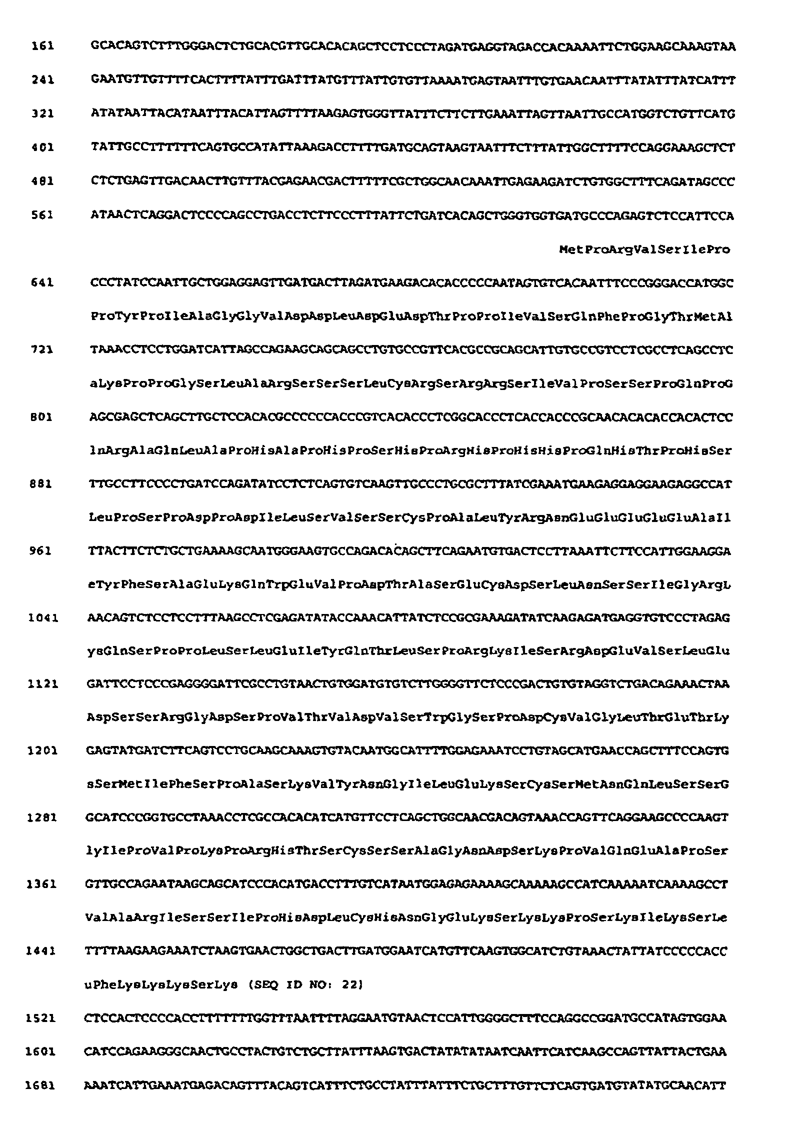

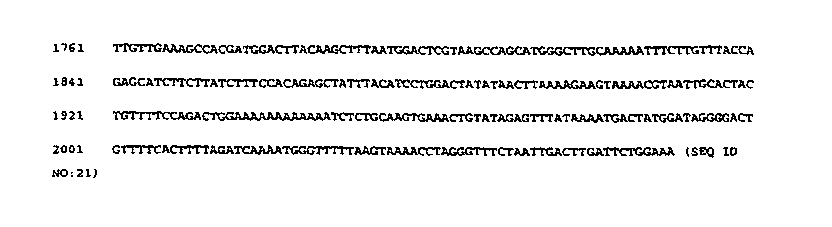

The encoded polypeptide has homology (approximately 52% identity) Sant Domain Protein smter (GenBank Accession. No AAD52614), which contains at least forty four casein kinase II phosphorylation sites (a serine kinase). A search of the PROSITE database of protein families and domains confirmed that a NOV11 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising five Casein kinase II phosphorylation site signature sequences includes amino acids 125-128, 127-130, 158-161, 163-166 and 168-171 of SEQ ID NO: 22.

Based on its relatedness to hypothetical Sant Domain Protein smter and the presence of five casein kinase II phosphorylation sites, the NOV1I protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV12

A NOV12 sequence according to the invention is a nucleic acid sequence encoding a polypeptide containing EGF-like domains. A NOV3 nucleic acid and polypeptide according to the invention includes the nucleic acid and encoded polypeptide sequence of a sequence named 25339846. A NOV nucleic acid and its encoded polypeptide includes the sequences shown in Table 13. The representative ORF includes a 182 amino acid polypeptide (SEQ ID NO: 24). A NOV3 polypeptide is predicted by PSORT program to localize to the peroxisome with a certainty of 0.5026. The molecular weight is 21128.2 daltons.

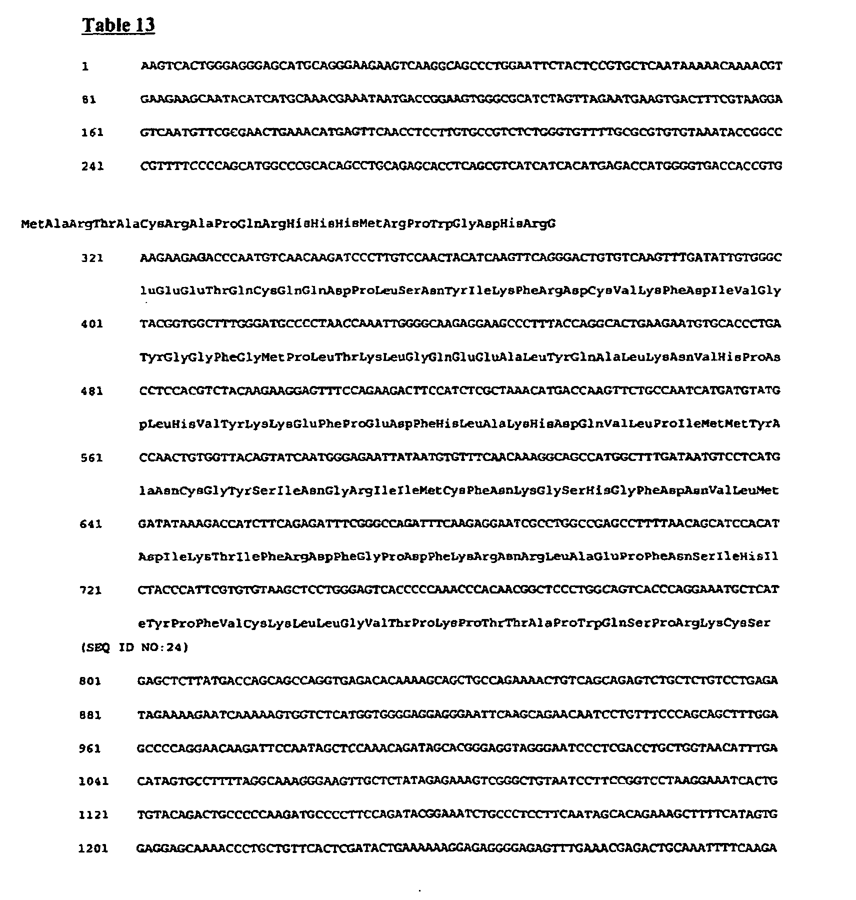

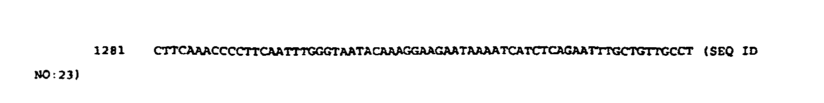

The encoded polypeptide has homology (approximately 54% identity) plasma-cell membrane glycoprotein pc-1 (GenBank Accession. No CAB56566), which contains a tyrosine kinase phoshorylation site. A search of the PROSITE database of protein families and domains revealed that a NOV12 polypeptide includes signature sequences characteristic of tyrosine kinases phosphorylation site. This signature sequence includes the consensus pattern:
[RK]-x(2)-[DE]-x(3)-Y or [RK]x(3)-[DE]-x(2)-Y [Y is the phosphorylation site] (SEQ ID NO: 37)

The amino acid sequence comprising the tyrosine phosphorylation signature sequences includes amino acids 58-66 of SEQ ID NO: 24. Accordingly, a NOV 12 polypeptide may be a novel tyrosine kinase.

### NOV13

A NOV 13 sequence according to the invention is a nucleic acid sequence encoding a polypeptide of the serine/threonine kinase family of proteins. A NOV 13 nucleic acid and polypeptide according to the invention includes the nucleic acid and encoded polypeptide sequence of a sequence named 25339846.0.146. A NOV13 nucleic acid and its encoded polypeptide includes the sequences shown in Table 16. The representative ORF includes a 171 amino acid polypeptide (SEQ ID NO: 26). SignalPep and PSort search programs predictes localization of NOV13 to the cytoplasm with a certainty of 0.4500). The predicted molecular weight of NOV13 protein is 21128.2 daltons.

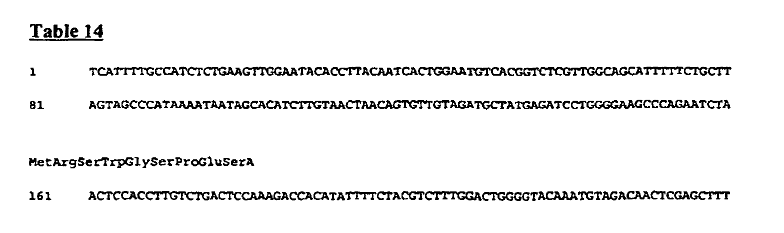

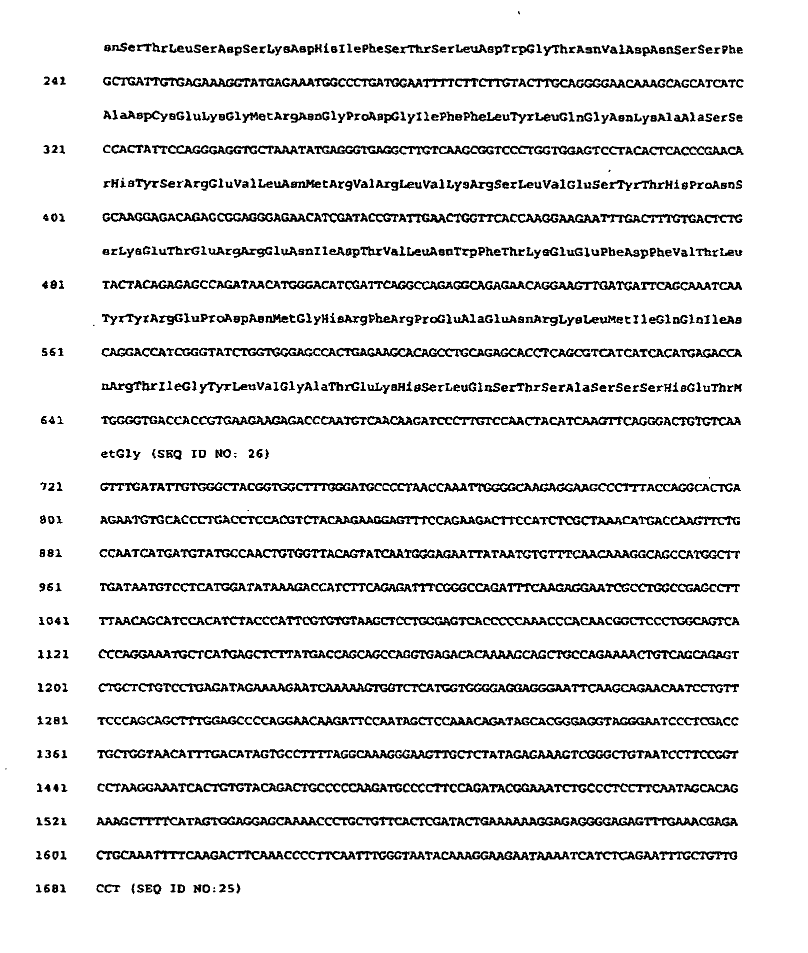

The encoded polypeptide has homology (approximately 54% identity) plasma-cell membrane glycoprotein pc-1 (GenBank Accession. No CAB56566), which contains at least four casein kinase II phosphorylation sites (a serine kinase). A search of the PROSITE database of protein families and domains confirmed that a NOV13 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising seven Casein kinase II phosphorylation site signature sequences includes amino acids 12-15, 23-26, 29-32, 35-38, 80-83, 107-110 and 165-168 of SEQ ID NO: 26.

Based on its relatedness to plasma- cell membrane glycoprotein pc-1 and the presence of seven casein kinase II phosphorylation sites, the NOV13 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV14

A NOV 14 sequence according to the invention is a nucleic acid sequence encoding a polypeptide of the serine/threonine kinase family of proteins. A NOV 14 nucleic acid and polypeptide according to the invention includes the nucleic acid and encoded polypeptide sequence of a sequence named 10132038.091. A NOV14 nucleic acid and its encoded polypeptide includes the sequences shown in Table 15. The disclosed nucleic acid (SEQ ID NO: 27) is 2912 bp nucleotides in length. The representative ORF includes a 926 amino acid polypeptide (SEQ ID NO:28). SignalPep and PSort search programs predictes localization of NOV14 to the mitocondrial matrix space with a certainty of 0.4712). The predicted molecular weight of NOV14 protein is 104117.1 daltons.

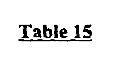

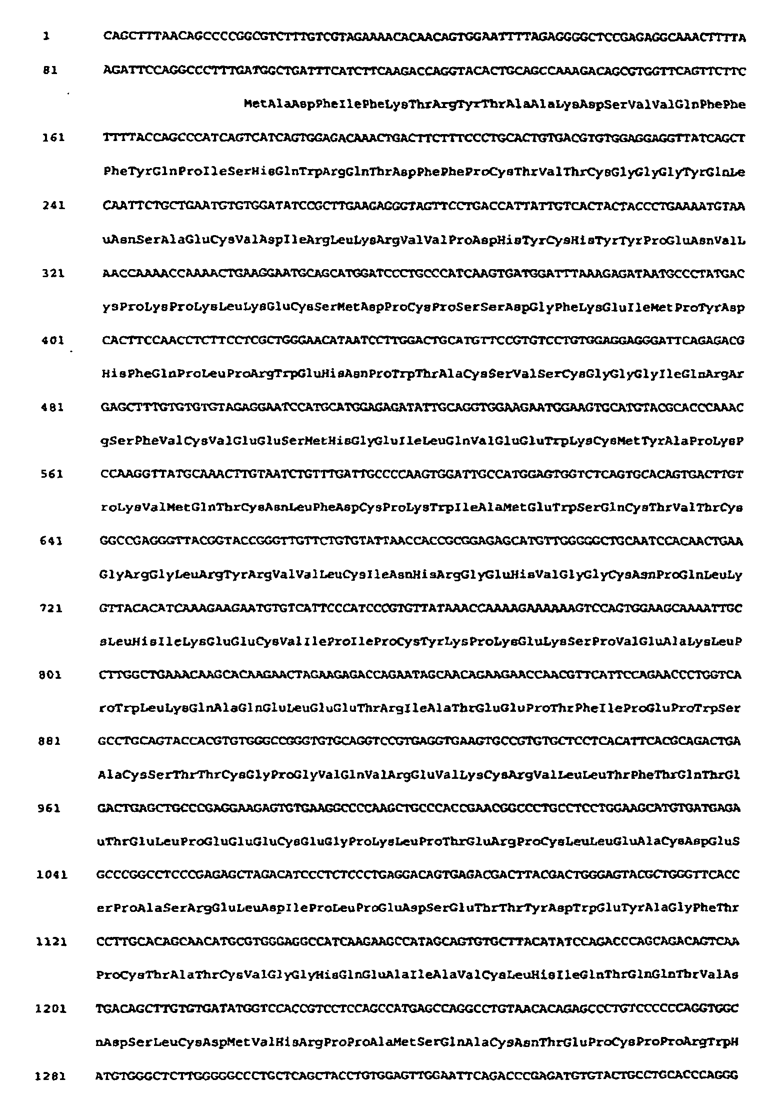

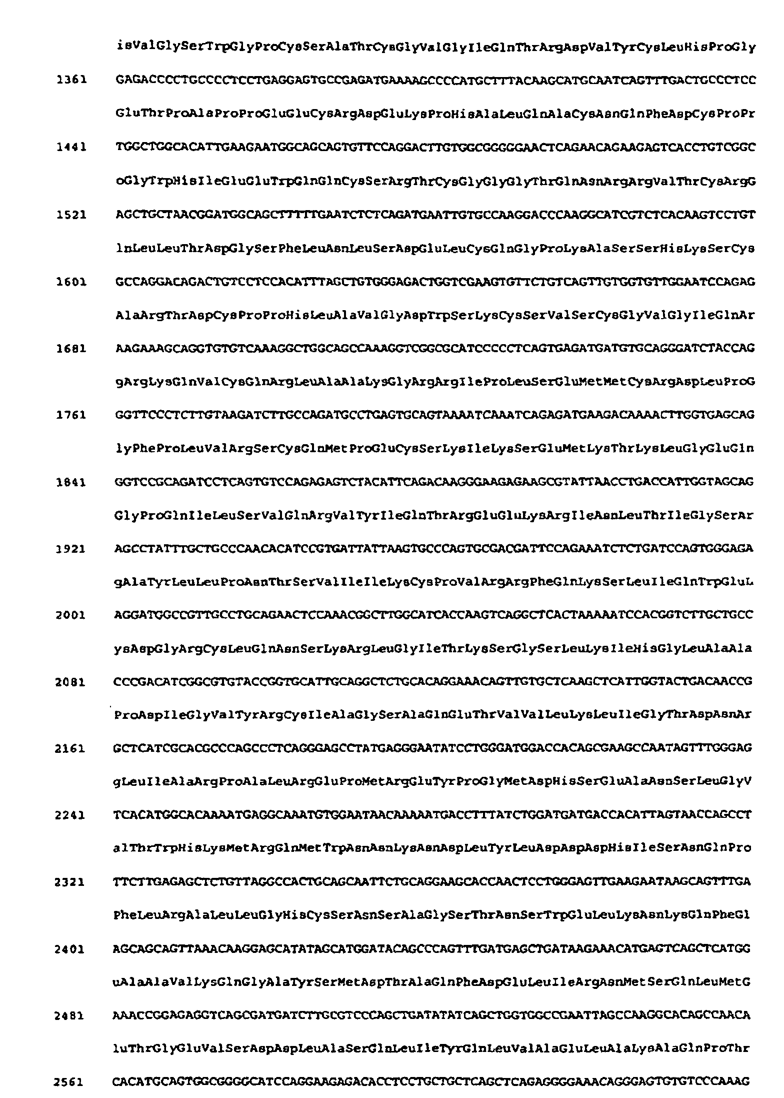

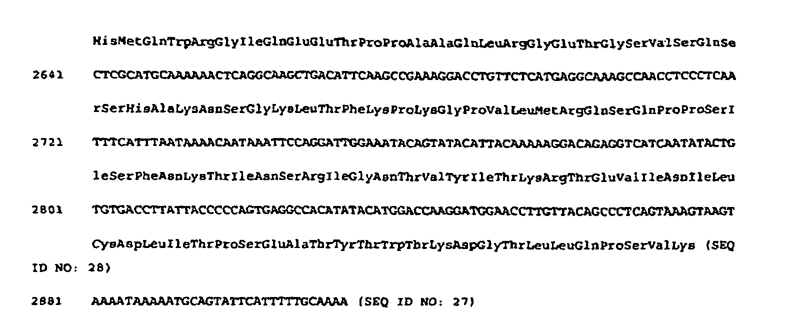

The encoded polypeptide has homology (approximately 32% identity) Caenorhabditis elegan (GenBank Accession. No P90884). A search of the PROSITE database of protein families and domains revealed that a NOV14 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising nine Casein kinase II phosphorylation site signature sequences includes amino acids 228-231, 285-288, 287-290, 331-334, 366-369, 370-373, 595-598, 673-676 and 906-909 of SEQ ID NO: 28.

Based on its relatedness to Caenorhabditis elegan and the presence of nine casein kinase II phosphorylation sites, the NOV14 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV15

A NOV 15 sequence according to the invention is a nucleic acid sequence encoding a polypeptide of the serine/threonine kinase family of proteins. A NOV15 nucleic acid and polypeptide according to the invention includes the nucleic acid and encoded polypeptide sequence of a sequence named 16401346asm.0.174 acid and its encoded polypeptide includes the sequences shown in Table 16. The representative ORF includes a 884 amino acid polypeptide (SEQ ID NO: 30 SignalPep and PSort search programs predictes localization of NOV16 to the cytoplasm space with a certainty of 0.6500). The predicted molecular weight of NOV14 protein is 94718.1 daltons.

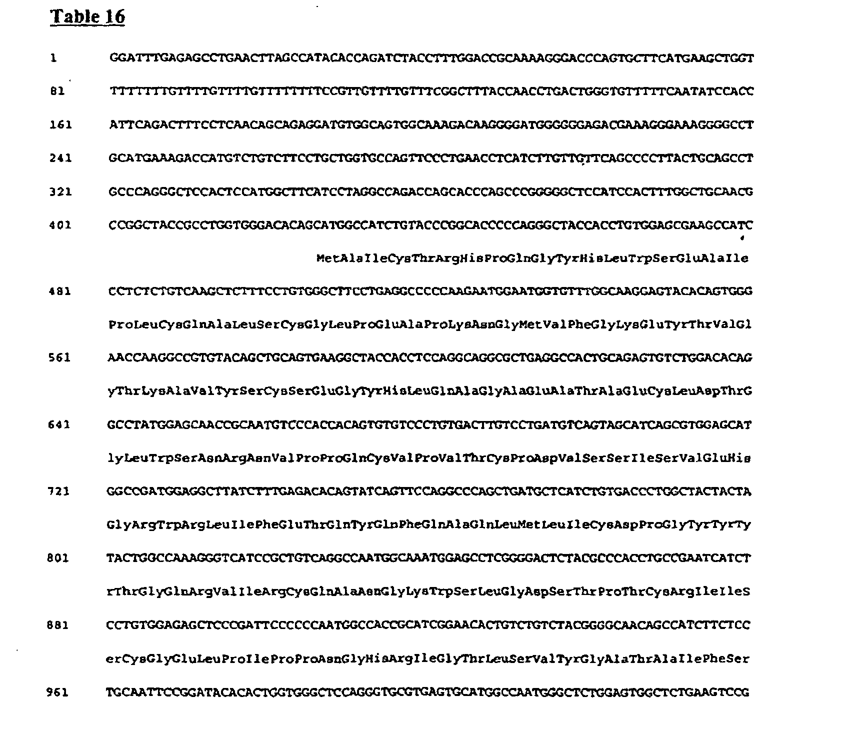

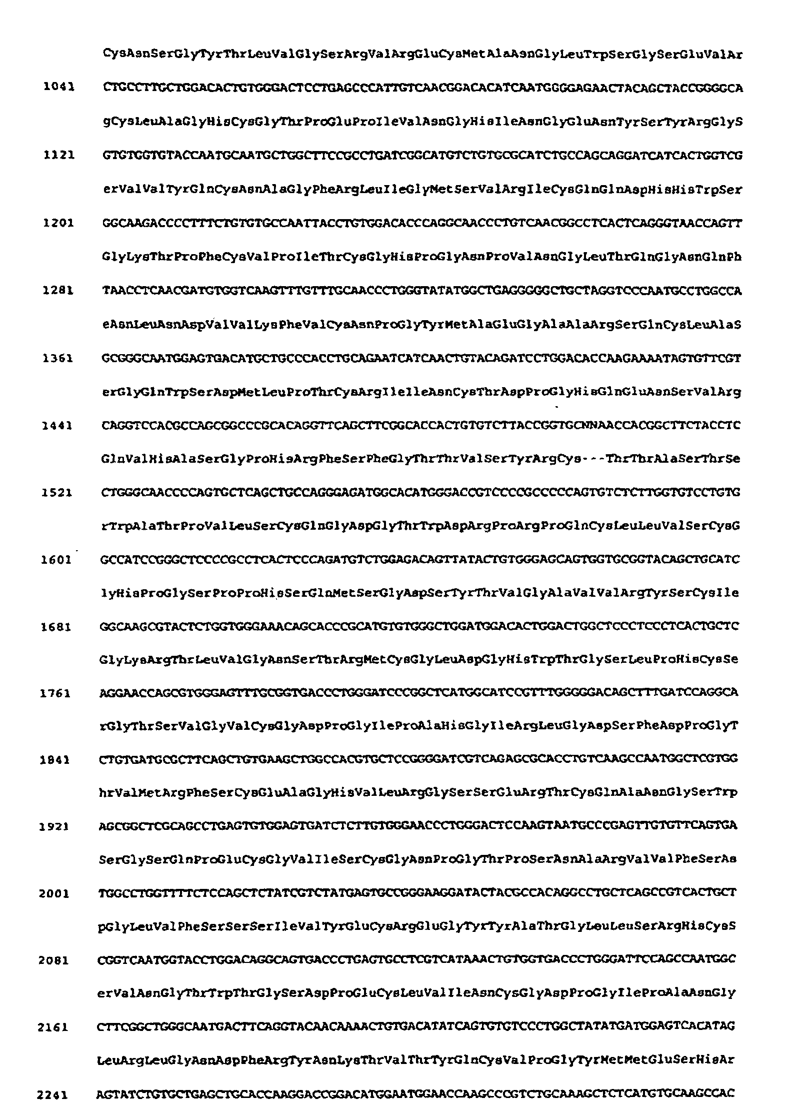

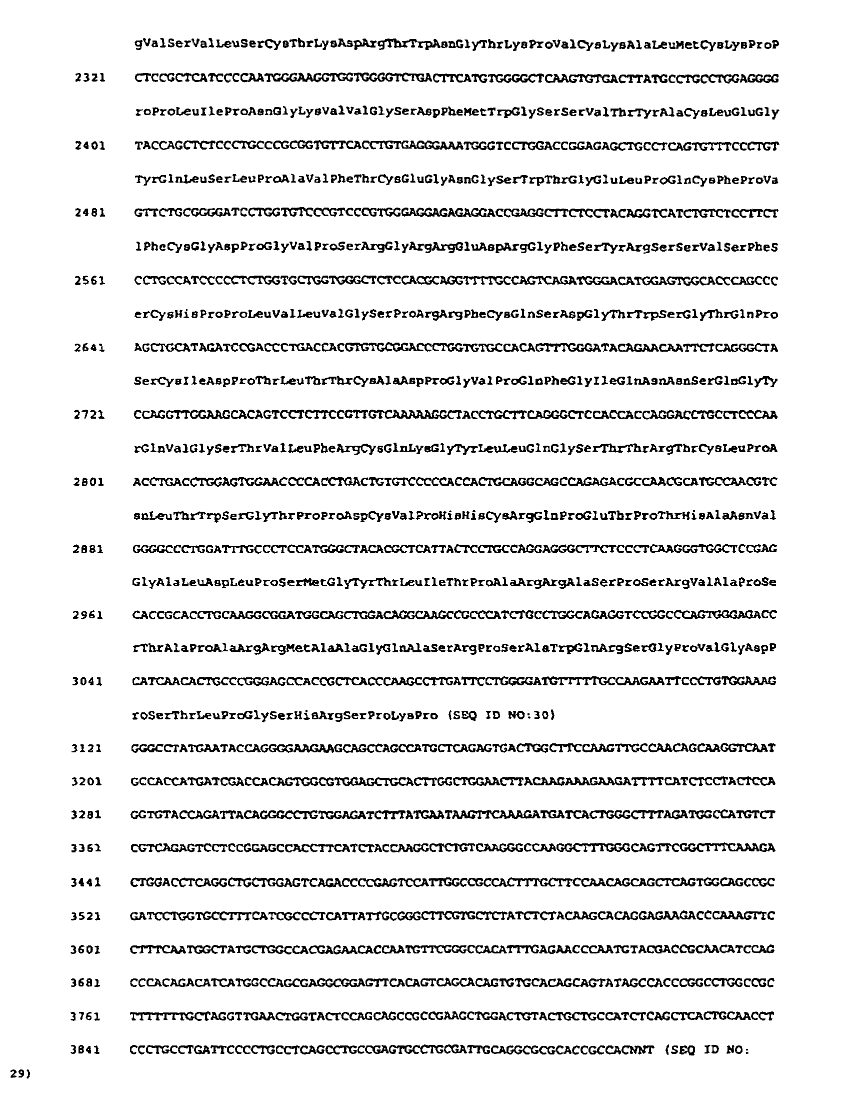

The encoded polypeptide has homology (approximately 29 %entity) complement receptpr 1 papio hamadryas (Gen Bank Q29528) A search of the PROSITE database of protein families and domains revealed that a NOV15 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising eleven Casein kinase II phosphorylation site signature sequences includes amino acids 1-51, 87-90, 140-143, 152-155, 200-203, 501-504, 558-561, 560-563, 739-742, 747-750, and 798-801 of SEQ ID NO: 30.

Based on its relatedness to complement receptpr 1 papio hamadryas and the presence of eleven casein kinase II phosphorylation sites, the NOV15 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOV16

A NOV16 sequence according to the invention is a nucleic acid sequence encoding a polypeptide of the serine/threonine kinase family of proteins. A NOV16 nucleic acid and polypeptide according to the invention includes the nucleic acid and encoded polypeptide sequence of a sequence named 1640134asm0.174_1. A NOV16 nucleic acid and its encoded polypeptide includes the sequences shown in Table 17. The representative ORF includes a 882 amino acid polypeptide (SEQ ID NO: 32). SignalPep and PSort search programs predictes localization of NOV16 to the cytoplasm space with a certainty of 0.6500). The predicted molecular weight of NOV16 protein is 94686.1 daltons.

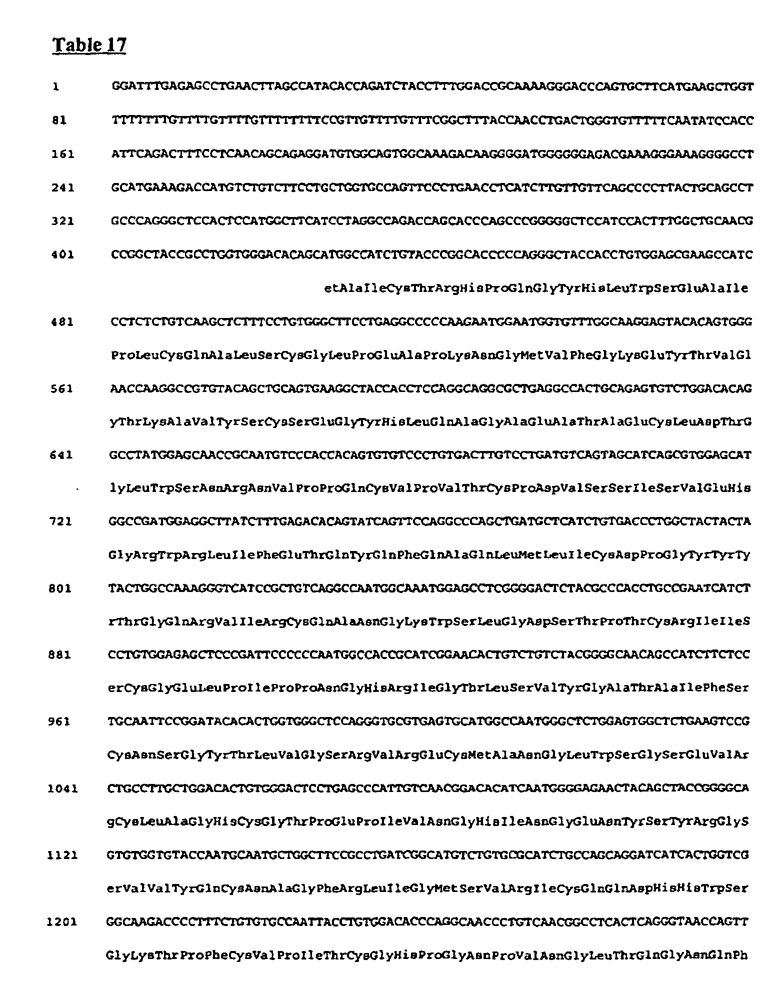

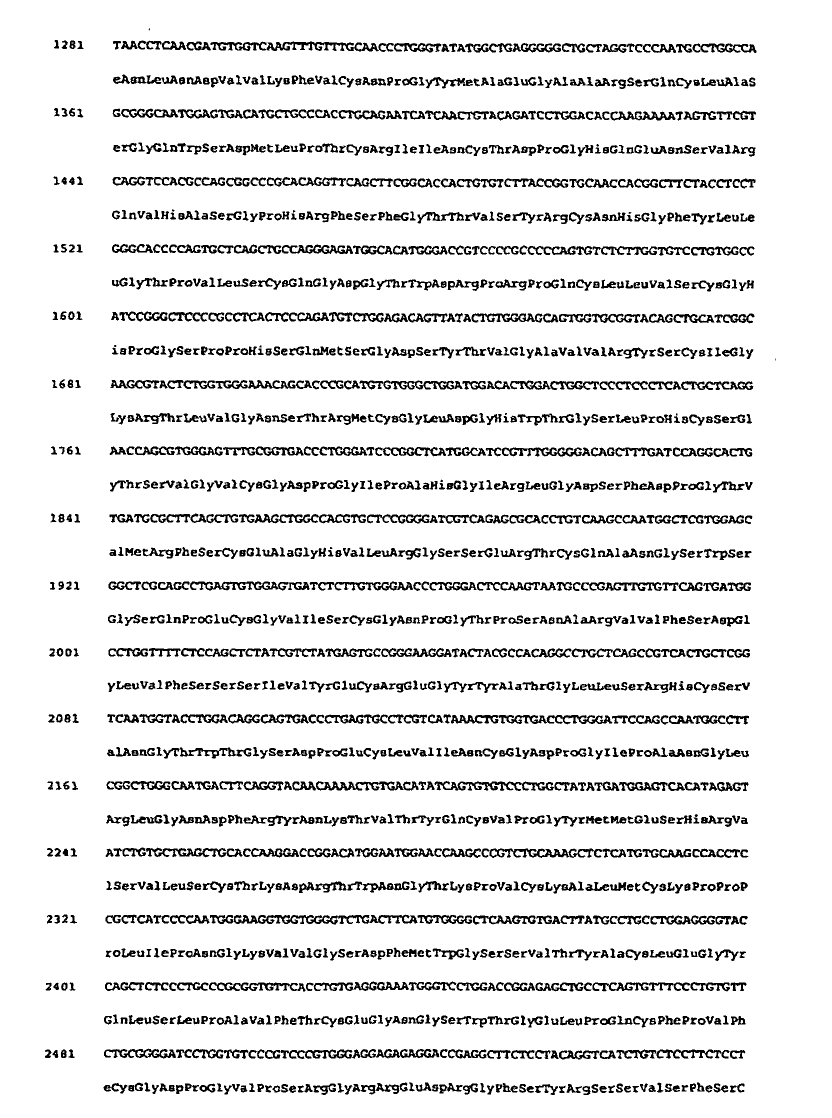

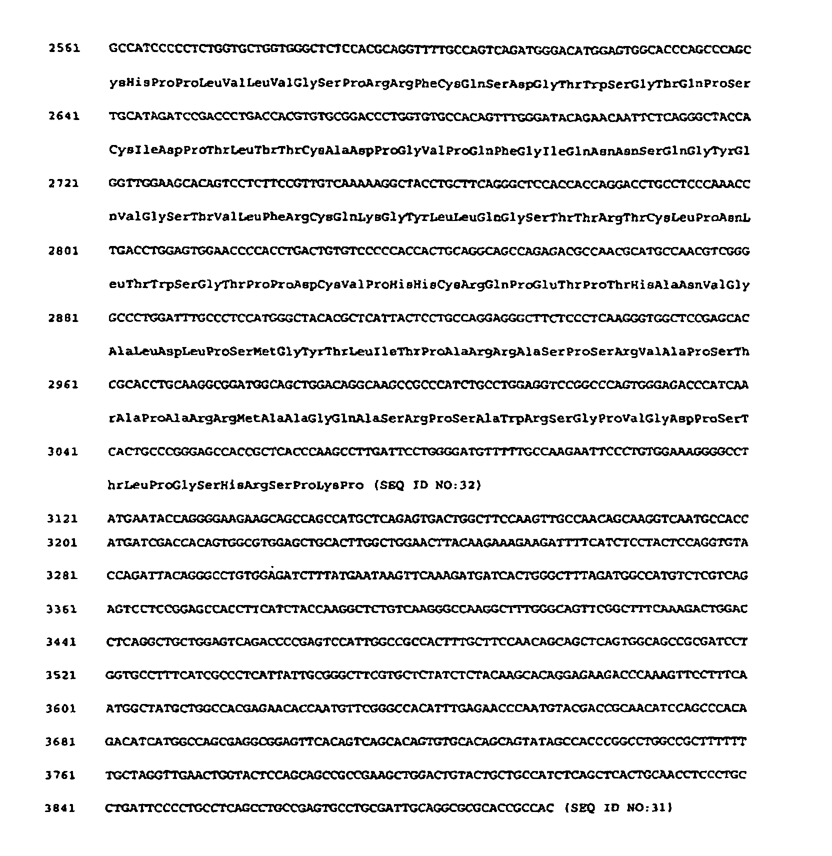

The encoded polypeptide has homology (approximately 43% identity) complement receptpr I papio hamadryas (GenBank Accession. No Q29528). A search of the PROSITE database of protein families and domains revealed that a NOV14 polypeptide is a member of the serine/theonine kinase family which can be defined by a polypeptide containing a stretch of highly conserved amino acid residues:
[ST]-x(2)-[DE] (Casein kinase II phosphorylation site; SEQ ID NO: 33)

The amino acid sequence comprising eleven Casein kinase II phosphorylation site signature sequences includes amino acids 51-54, 140-143, 152-155, 200-203, 500-503, 557-560, 559-562, 738-741, 746-749 and 797-800 of SEQ ID NO: 32.

Based on its relatedness to complement receptpr I papio hamadryas and the presence of eleven casein kinase II phosphorylation sites, the NOV16 protein is a novel member of serine/threonine kinase family. The discovery of molecules related to serine/threonine kinases satisfies a need in the art by providing new diagnostic or therapeutic compositions useful in the treatment of disorders associated with alterations in the expression of members of serine/threonine kinase proteins. Nucleic acids, polypeptides, antibodies, and other compositions of the present invention are useful in a variety of diseases and pathologies, including by way of nonlimiting example, those involving cancer, *e*.*g*.,cellular proliferative disorders and contraception.

### NOVX Nucleic Acids

The nucleic acids of the invention include those that encode a NOVX polypeptide or protein. As used herein, the terms polypeptide and protein are interchangeable.

In some embodiments, a NOVX nucleic acid encodes a mature NOVX polypeptide. As used herein, a "mature" form of a polypeptide or protein described herein relates to the product of a naturally occurring polypeptide or precursor form or proprotein. The naturally occurring polypeptide, precursor or proprotein includes, by way of nonlimiting example, the full length gene product, encoded by the corresponding gene. Alternatively, it may be defined as the polypeptide, precursor or proprotein encoded by an open reading frame described herein. The product "mature" form arises, again by way of nonlimiting example, as a result of one or more naturally occurring processing steps that may take place within the cell in which the gene product arises. Examples of such processing steps leading to a "mature" form of a polypeptide or protein include the cleavage of the N-terminal methionine residue encoded by the initiation codon of an open reading frame, or the proteolytic cleavage of a signal peptide or leader sequence. Thus a mature form arising from a precursor polypeptide or protein that has residues 1 to N, where residue 1 is the *N*-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

Among the NOVX nucleic acids is the nucleic acid whose sequence is provided in SEQ ID NO:2n-1, wherein n is an integer between 1-16, or a fragment thereof. Additionally, the invention includes mutant or variant nucleic acids of SEQ ID NO:2n-1, wherein n is an integer between 1-16, or a fragment thereof, any of whose bases may be changed from the corresponding bases shown in SEQ ID NO:2n-1, wherein n is an integer between 1-16, while still encoding a protein that maintains at least one of its NOVX-like activities and physiological functions (*i*.*e*., modulating angiogenesis, neuronal development). The invention further includes the complement of the nucleic acid sequence of SEQ ID NO:2n-1, wherein n is an integer between 1-16, including fragments, derivatives, analogs and homologs thereof. The invention additionally includes nucleic acids or nucleic acid fragments, or complements thereto, whose structures include chemical modifications.

One aspect of the invention pertains to isolated nucleic acid molecules that encode NOVX proteins or biologically active portions thereof. Also included are nucleic acid fragments sufficient for use as hybridization probes to identify NOVX-encoding nucleic acids (*e*.*g*., NOVX mRNA) and fragments for use as polymerase chain reaction (PCR) primers for the amplification or mutation of NOVX nucleic acid molecules. As used herein, the term "nucleic acid molecule" is intended to include DNA molecules (*e*.*g*., cDNA or genomic DNA), RNA molecules (*e*.*g*., mRNA), analogs of the DNA or RNA generated using nucleotide analogs, and derivatives, fragments and homologs thereof. The nucleic acid molecule can be single-stranded or double-stranded, but preferably is double-stranded DNA.

"Probes" refer to nucleic acid sequences of variable length, preferably between at least about 10 nucleotides (nt), 100 nt, or as many as about, *e*.*g*., 6,000 nt, depending on use. Probes are used in the detection of identical, similar, or complementary nucleic acid sequences. Longer length probes are usually obtained from a natural or recombinant source, are highly specific and much slower to hybridize than oligomers. Probes may be single- or double-stranded and designed to have specificity in PCR, membrane-based hybridization technologies, or ELISA-like technologies.

An "isolated" nucleic acid molecule is one that is separated from other nucleic acid molecules that are present in the natural source of the nucleic acid. Examples of isolated nucleic acid molecules include, but are not limited to, recombinant DNA molecules contained in a vector, recombinant DNA molecules maintained in a heterologous host cell, partially or substantially purified nucleic acid molecules, and synthetic DNA or RNA molecules. Preferably, an "isolated" nucleic acid is free of sequences which naturally flank the nucleic acid (*i.e*., sequences located at the 5' and 3' ends of the nucleic acid) in the genomic DNA of the organism from which the nucleic acid is derived. For example, in various embodiments, the isolated NOVX nucleic acid molecule can contain less than about 50 kb, 25 kb, 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the present invention, *e*.*g*., a nucleic acid molecule having the nucleotide sequence of SEQ ID NO:2n-1, wherein n is an integer between 1-16, or a complement of any of this nucleotide sequence, can be isolated using standard molecular biology techniques and the sequence information provided herein. Using all or a portion of the nucleic acid sequence of SEQ ID NO:2n-1, wherein n is an integer between 1-16, as a hybridization probe, NOVX nucleic acid sequences can be isolated using standard hybridization and cloning techniques (*e*.*g*., as described in Sambrook *et al*., eds., MOLECULAR CLONING: A LABORATORY MANUAL 2^{nd} Ed., Cold Spring Harbor Laboratory Press, Cold Spring Harbor, NY, 1989; and Ausubel, *et al*., eds., CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e*.*g*., using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment, an oligonucleotide comprising a nucleic acid molecule less than 100 nt in length would further comprise at lease 6 contiguous nucleotides of SEQ ID NO:2n-1, wherein n is an integer between 1-16, or a complement thereof. Oligonucleotides may be chemically synthesized and may be used as probes.

In another embodiment, an isolated nucleic acid molecule of the invention comprises a nucleic acid molecule that is a complement of the nucleotide sequence shown in SEQ ID NO:2n-1, wherein n is an integer between 1-16, or a portion of this nucleotide sequence. A nucleic acid molecule that is complementary to the nucleotide sequence shown in SEQ ID NO:2n-1, wherein n is an integer between 1-16is one that is sufficiently complementary to the nucleotide sequence shown in SEQ ID NO:2n-1, wherein n is an integer between 1-16that it can hydrogen bond with little or no mismatches to the nucleotide sequence shown in SEQ ID NO:2n-1, wherein n is an integer between 1-16, thereby forming a stable duplex.

As used herein, the term "complementary" refers to Watson-Crick or Hoogsteen base pairing between nucleotide units of a nucleic acid molecule, and the term "binding" means the physical or chemical interaction between two polypeptides or compounds or associated polypeptides or compounds or combinations thereof. Binding includes ionic, non-ionic, Von der Waals, hydrophobic interactions, etc. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Moreover, the nucleic acid molecule of the invention can comprise only a portion of the nucleic acid sequence of SEQ ID NO:2n-1, wherein n is an integer between 1-16, *e*.*g*., a fragment that can be used as a probe or primer, or a fragment encoding a biologically active portion of NOVX. Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, 85%, 90%, 95%, 98%, or even 99% identity (with a preferred identity of 80-99%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. See *e*.*g*. Ausubel, *et al*., CURRENT PROTOCOLS IN MOLECULAR BIOLOGY, John Wiley & Sons, New York, NY, 1993, and below. An exemplary program is the Gap program (Wisconsin Sequence Analysis Package, Version 8 for UNIX, Genetics Computer Group, University Research Park, Madison, WI) using the default settings, which uses the algorithm of Smith and Waterman (Adv. Appl. Math., 1981, 2: 482-489, which is incorporated herein by reference in its entirety).

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of a NOVX polypeptide. Isoforms can be expressed in different tissues of the same organism as a result of, for example, alternative splicing ofRNA. Alternatively, isoforms can be encoded by different genes. In the present invention, homologous nucleotide sequences include nucleotide sequences encoding for a NOVX polypeptide of species other than humans, including, but not limited to, mammals, and thus can include, *e*.*g*., mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the nucleotide sequence encoding human NOVX protein. Homologous nucleic acid sequences include those nucleic acid sequences that encode conservative amino acid substitutions (see below) in SEQ ID NO:2, 4, 6 or 8, as well as a polypeptide having NOVX activity. Biological activities of the NOVX proteins are described below. A homologous amino acid sequence does not encode the amino acid sequence of a human NOVX polypeptide.

The nucleotide sequence determined from the cloning of the human NOVX gene allows for the generation of probes and primers designed for use in identifying and/or cloning NOVX homologues in other cell types, *e.g.,* from other tissues, as well as NOVX homologues from other mammals. The probe/primer typically comprises a substantially purified oligonucleotide. The oligonucleotide typically comprises a region of nucleotide sequence that hybridizes under stringent conditions to at least about 12, 25, 50, 100, 150, 200, 250, 300, 350 or 400 or more consecutive sense strand nucleotide sequence of SEQ ID NO:2n-1, wherein n is an integer between 1-16; or an anti-sense strand nucleotide sequence of SEQ ID NO:2n-1, wherein n is an integer between 1-16; or of a naturally occurring mutant of SEQ ID NO:2n-1, wherein n is an integer between 1-16.

Probes based on the human NOVX nucleotide sequence can be used to detect transcripts or genomic sequences encoding the same or homologous proteins. In various embodiments, the probe further comprises a label group attached thereto, *e*.*g*., the label group can be a radioisotope, a fluorescent compound, an enzyme, or an enzyme co-factor. Such probes can be used as a part of a diagnostic test kit for identifying cells or tissue which misexpress a NOVX protein, such as by measuring a level of a NOVX-encoding nucleic acid in a sample of cells from a subject *e*.*g*., detecting NOVX mRNA levels or determining whether a genomic NOVX gene has been mutated or deleted.

A "polypeptide having a biologically active portion of NOVX" refers to polypeptides exhibiting activity similar, but not necessarily identical to, an activity of a polypeptide of the present invention, including mature forms, as measured in a particular biological assay, with or without dose dependency. A nucleic acid fragment encoding a "biologically active portion of NOVX" can be prepared by isolating a portion of SEQ ID NO:2n-1, wherein n is an integer between 1-16that encodes a polypeptide having a NOVX biological activity (biological activities of the NOVX proteins are described below), expressing the encoded portion of NOVX protein (*e*.*g*., by recombinant expression *in vitro)* and assessing the activity of the encoded portion of NOVX. For example, a nucleic acid fragment encoding a biologically active portion of NOVX can optionally include an ATP-binding domain. In another embodiment, a nucleic acid fragment encoding a biologically active portion of NOVX includes one or more regions.

### NOVX Variants

The invention further encompasses nucleic acid molecules that differ from the nucleotide sequences shown in SEQ ID NO:2n-1, wherein n is an integer between 1-16due to the degeneracy of the genetic code. These nucleic acids thus encode the same NOVX protein as that encoded by the nucleotide sequence shown in SEQ ID NO:2n-1, wherein n is an integer between 1-16*e*.*g*., the polypeptide of SEQ ID NO:2n, wherein n is an integer between 1-16. In another embodiment, an isolated nucleic acid molecule of the invention has a nucleotide sequence encoding a protein having an amino acid sequence shown in SEQ ID NO: 2

In addition to the human NOVX nucleotide sequence shown in SEQ ID NO:2n-1, wherein n is an integer between 1-16, it will be appreciated by those skilled in the art that DNA sequence polymorphisms that lead to changes in the amino acid sequences of NOVX may exist within a population (*e*.*g*., the human population). Such genetic polymorphism in the NOVX gene may exist among individuals within a population due to natural allelic variation. As used herein, the terms "gene" and "recombinant gene" refer to nucleic acid molecules comprising an open reading frame encoding a NOVX protein, preferably a mammalian NOVX protein. Such natural allelic variations can typically result in 1-5% variance in the nucleotide sequence of the NOVX gene. Any and all such nucleotide variations and resulting amino acid polymorphisms in NOVX that are the result of natural allelic variation and that do not alter the functional activity of NOVX are intended to be within the scope of the invention.

Moreover, nucleic acid molecules encoding NOVX proteins from other species, and thus that have a nucleotide sequence that differs from the human sequence of SEQ ID NO:2n-1, wherein n is an integer between I-16are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions. For example, a soluble human NOVX cDNA can be isolated based on its homology to human membrane-bound NOVX. Likewise, a membrane-bound human NOVX cDNA can be isolated based on its homology to soluble human NOVX.

Accordingly, in another embodiment, an isolated nucleic acid molecule of the invention is at least 6 nucleotides in length and hybridizes under stringent conditions to the nucleic acid molecule comprising the nucleotide sequence of SEQ ID NO:2n-1, wherein n is an integer between 1-16. In another embodiment, the nucleic acid is at least 10, 25, 50, 100, 250, 500 or 750 nucleotides in length. In another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i*.*e*., nucleic acids encoding NOVX proteins derived from species other than human) or other related sequences (*e*.*g*., paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5°C lower than the thermal melting point (Tₘ) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (*e*.*g*., 10 nt to 50 nt) and at least about 60°C for longer probes, primers and oligonucleotides. Stringent conditions may also be achieved with the addition of destabilizing agents, such as formamide.

Stringent conditions are known to those skilled in the art and can be found in CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions is hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C. This hybridization is followed by one or more washes in 0.2X SSC, 0.01% BSA at 50°C. An isolated nucleic acid molecule of the invention that hybridizes under stringent conditions to the sequence of SEQ ID NO:2n-1, wherein n is an integer between 1-16corresponds to a naturally occurring nucleic acid molecule. As used herein, a "naturally-occurring" nucleic acid molecule refers to an RNA or DNA molecule having a nucleotide sequence that occurs in nature (*e.g*., encodes a natural protein).

In a second embodiment, a nucleic acid sequence that is hybridizable to the nucleic acid molecule comprising the nucleotide sequence of SEQ ID NO:2n-1, wherein n is an integer between 1-16, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well known in the art. See, *e.g.,* Ausubel *et al*. *(*eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY.

In a third embodiment, a nucleic acid that is hybridizable to the nucleic acid molecule comprising the nucleotide sequence of SEQ ID NO:2n-1, wherein n is an integer between 1-16, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e*.*g*., as employed for cross-species hybridizations). See, *e.g.,* Ausubel *et al*. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981, *Proc Natl Acad Sci USA 78:* 6789-6792.

### Conservative mutations

In addition to naturally-occurring allelic variants of the NOVX sequence that may exist in the population, the skilled artisan will further appreciate that changes can be introduced by mutation into the nucleotide sequence of SEQ ID NO:2n-1, wherein n is an integer between 1-16, thereby leading to changes in the amino acid sequence of the encoded NOVX protein, without altering the functional ability of the NOVX protein. For example, nucleotide substitutions leading to amino acid substitutions at "non-essential" amino acid residues can be made in the sequence of SEQ ID NO:2n-1, wherein n is an integer between 1-16. A "non-essential" amino acid residue is a residue that can be altered from the wild-type sequence of NOVX without altering the biological activity, whereas an "essential" amino acid residue is required for biological activity. For example, amino acid residues that are conserved among the NOVX proteins of the present invention, are predicted to be particularly unamenable to alteration.

Another aspect of the invention pertains to nucleic acid molecules encoding NOVX proteins that contain changes in amino acid residues that are not essential for activity. Such NOVX proteins differ in amino acid sequence from SEQ ID NO:2n, wherein n is an integer between 1-16 , yet retain biological activity. In one embodiment, the isolated nucleic acid molecule comprises a nucleotide sequence encoding a protein, wherein the protein comprises an amino acid sequence at least about 75% homologous to the amino acid sequence of SEQ ID NO: 2, 4, 6, or 8. Preferably, the protein encoded by the nucleic acid is at least about 80% homologous to SEQ ID NO:2n, wherein n is an integer between 1-16, more preferably at least about 90%, 95%, 98%, and most preferably at least about 99% homologous to SEQ ID NO:2n, wherein n is an integer between 1-16.

An isolated nucleic acid molecule encoding a NOVX protein homologous to the protein of can be created by introducing one or more nucleotide substitutions, additions or deletions into the nucleotide sequence of SEQ ID NO:2n-1, wherein n is an integer between 1-16, such that one or more amino acid substitutions, additions or deletions are introduced into the encoded protein.

Mutations can be introduced into the nucleotide sequence of SEQ ID NO:2n-1, wherein n is an integer between 1-16by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (*e*.*g*., lysine, arginine, histidine), acidic side chains (*e*.*g*., aspartic acid, glutamic acid), uncharged polar side chains (*e*.*g*., glycine, asparagine, glutamine, serine, threonine, tyrosine, cysteine), nonpolar side chains (*e*.*g*., alanine, valine, leucine, isoleucine, proline, phenylalanine, methionine, tryptophan), beta-branched side chains (*e*.*g*., threonine, valine, isoleucine) and aromatic side chains (*e*.*g*., tyrosine, phenylalanine, tryptophan, histidine). Thus, a predicted nonessential amino acid residue in NOVX is replaced with another amino acid residue from the same side chain family. Alternatively, in another embodiment, mutations can be introduced randomly along all or part of a NOVX coding sequence, such as by saturation mutagenesis, and the resultant mutants can be screened for NOVX biological activity to identify mutants that retain activity. Following mutagenesis of SEQ ID NO:2n-1, wherein n is an integer between 1-16the encoded protein can be expressed by any recombinant technology known in the art and the activity of the protein can be determined.

In one embodiment, a mutant NOVX protein can be assayed for (1) the ability to form protein:protein interactions with other NOVX proteins, other cell-surface proteins, or biologically active portions thereof, (2) complex formation between a mutant NOVX protein and a NOVX receptor; (3) the ability of a mutant NOVX protein to bind to an intracellular target protein or biologically active portion thereof; (*e*.*g*., avidin proteins); (4) the ability to bind NOVX protein; or (5) the ability to specifically bind an anti-NOVX protein antibody.

### Antisense NOVX Nucleic Acids

Another aspect of the invention pertains to isolated antisense nucleic acid molecules that are hybridizable to or complementary to the nucleic acid molecule comprising the nucleotide sequence of SEQ ID NO: 1, 3, 5 or 7, or fragments, analogs or derivatives thereof An "antisense" nucleic acid comprises a nucleotide sequence that is complementary to a "sense" nucleic acid encoding a protein, *e*.*g*., complementary to the coding strand of a double-stranded cDNA molecule or complementary to an mRNA sequence. In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of a NOVX protein of SEQ ID NO:2n, wherein n is an integer between 1-16 or antisense nucleic acids complementary to a NOVX nucleic acid sequence of SEQ ID NO:2n-1, wherein n is an integer between 1-16are additionally provided.

In one embodiment, an antisense nucleic acid molecule is antisense to a "coding region" of the coding strand of a nucleotide sequence encoding NOVX. The term "coding region" refers to the region of the nucleotide sequence comprising codons which are translated into amino acid residues (*e*.*g*., the protein coding region of human NOVX corresponds to SEQ ID NO:2n, wherein n is an integer between 1-16). In another embodiment, the antisense nucleic acid molecule is antisense to a "noncoding region" of the coding strand of a nucleotide sequence encoding NOVX. The term "noncoding region" refers to 5' and 3' sequences which flank the coding region that are not translated into amino acids (*i*.*e*., also referred to as 5' and 3' untranslated regions).

Given the coding strand sequences encoding NOVX disclosed herein (*e*.*g*., SEQ ID NO:2n-1, wherein n is an integer between 1-16), antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site ofNOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e*.*g*., phosphorothioate derivatives and acridine substituted nucleotides can be used.

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i.e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a NOVX protein to thereby inhibit expression of the protein, *e*.*g*., by inhibiting transcription and/or translation. The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.,* by linking the antisense nucleic acid molecules to peptides or antibodies that bind to cell surface receptors or antigens. The antisense nucleic acid molecules can also be delivered to cells using the vectors described herein. To achieve sufficient intracellular concentrations of antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al*. (1987) *Nucleic Acids Res* 15: 6625-6641). The antisense nucleic acid molecule can also comprise a 2'-o-methylribonucleotide (Inoue *et al*. (1987) *Nucleic Acids Res* 15: 6131-6148) or a chimeric RNA -DNA analogue (Inoue *et al*. (1987) *FEBS Lett* 215: 327-330).

Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

### NOVX Ribozymes and PNA moieties

In still another embodiment, an antisense nucleic acid of the invention is a ribozyme. Ribozymes are catalytic RNA molecules with ribonuclease activity that are capable of cleaving a single-stranded nucleic acid, such as a mRNA, to which they have a complementary region. Thus, ribozymes (*e*.*g*., hammerhead ribozymes (described in Haselhoff and Gerlach (1988) *Nature* 334:585-591)) can be used to catalytically cleave NOVX mRNA transcripts to thereby inhibit translation of NOVX mRNA. A ribozyme having specificity for a NOVX-encoding nucleic acid can be designed based upon the nucleotide sequence of a NOVX DNA disclosed herein (*i*.*e*., SEQ ID NO;2n-1, wherein n is an integer between 1-16). For example, a derivative of a Tetrahymena L-19 IVS RNA can be constructed in which the nucleotide sequence of the active site is complementary to the nucleotide sequence to be cleaved in a NOVX-encoding mRNA. See, *e.g.,* Cech *et al*. U.S. Pat. No. 4,987,071; and Cech *et al*. U.S. Pat. No. 5,116,742. Alternatively, NOVX mRNA can be used to select a catalytic RNA having a specific ribonuclease activity from a pool of RNA molecules. See, *e*.*g*., Bartel *et al*., (1993) *Science* 261:1411-1418.

Alternatively, NOVX gene expression can be inhibited by targeting nucleotide sequences complementary to the regulatory region of the NOVX (*e*.*g*., the NOVX promoter and/or enhancers) to form triple helical structures that prevent transcription of the NOVX gene in target cells. See generally, Helene. (1991) *Anticancer Drug Des*. 6: 569-84; Helene. *et al*. (1992) *Ann. N.Y. Acad. Sci.* 660:27-36; and Maher (1992) *Bioassays* 14: 807-15.

In various embodiments, the nucleic acids of NOVX can be modified at the base moiety, sugar moiety or phosphate backbone to improve, *e*.*g*., the stability, hybridization, or solubility of the molecule. For example, the deoxyribose phosphate backbone of the nucleic acids can be modified to generate peptide nucleic acids (see Hyrup *et al*. (1996) *Bioorg Med Chem* 4: 5-23). As used herein, the terms "peptide nucleic acids" or "PNAs" refer to nucleic acid mimics, *e*.*g*., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al*. (1996) above; Perry-O'Keefe *et al*. (1996) *PNAS 93:* 14670-675.

PNAs of NOVX can be used in therapeutic and diagnostic applications. For example, PNAs can be used as antisense or antigene agents for sequence-specific modulation of gene expression by, *e*.*g*., inducing transcription or translation arrest or inhibiting replication. PNAs of NOVX can also be used, *e*.*g*., in the analysis of single base pair mutations in a gene by, *e*.*g*., PNA directed PCR clamping; as artificial restriction enzymes when used in combination with other enzymes, *e*.*g*., S 1 nucleases (Hyrup B. (1996) above); or as probes or primers for DNA sequence and hybridization (Hyrup *et al*. (1996), above; Perry-O'Keefe (1996), above).

In another embodiment, PNAs of NOVX can be modified, *e*.*g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes, *e.g*., RNase H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) above). The synthesis of PNA-DNA chimeras can be performed as described in Hyrup (1996) above and Finn *et al*. (1996) *Nucl Acids Res* 24: 3357-63. For example, a DNA chain can be synthesized on a solid support using standard phosphoramidite coupling chemistry, and modified nucleoside analogs, *e*.*g*., 5'-(4-methoxytrityl) amino-5'-deoxy-thymidine phosphoramidite, can be used between the PNA and the 5' end of DNA (Mag *et al*. (1989) *Nucl Acid Res* 17: 5973-88). PNA monomers are then coupled in a stepwise manner to produce a chimeric molecule with a 5' PNA segment and a 3' DNA segment (Finn *et al*. (1996) above). Alternatively, chimeric molecules can be synthesized with a 5'DNA segment and a 3' PNA segment. See, Petersen *et al*. (1975) *Bioorg Med Chem Lett* 5: 1119-11124.

In other embodiments, the oligonucleotide may include other appended groups such as peptides (*e*.*g*., for targeting host cell receptors *in vivo*), or agents facilitating transport across the cell membrane (see, *e*.*g*., Letsinger *et al*., 1989, *Proc. Natl. Acad. Sci. U.S.A*. 86:6553-6556; Lemaitre *et al*., 1987, Proc. *Natl. Acad. Sci.* 84:648-652; PCT Publication No. W088/09810) or the blood-brain barrier (see, *e*.*g*., PCT Publication No. W089/10134). In addition, oligonucleotides can be modified with hybridization triggered cleavage agents (See, *e.g.,* Krol *et al*., 1988, *BioTechniques* 6:958-976) or intercalating agents. (See, *e*.*g*., Zon, 1988, *Pharm. Res*. 5: 539-549). To this end, the oligonucleotide may be conjugated to another molecule, *e*.*g*., a peptide, a hybridization triggered cross-linking agent, a transport agent, a hybridization-triggered cleavage agent, etc.

### NOVX Polypeptides

A NOVX polypeptide of the invention includes the NOVX-like protein whose sequence is provided in SEQ ID NO:2n, wherein n is an integer between 1-16. The invention also includes a mutant or variant protein any of whose residues may be changed from the corresponding residue shown in SEQ ID NO:2n, wherein n is an integer between 1-16 while still encoding a protein that maintains its NOVX-like activities and physiological functions, or a functional fragment thereof. In some embodiments, up to 20% or more of the residues may be so changed in the mutant or variant protein. In some embodiments, the NOVX polypeptide according to the invention is a mature polypeptide.

In general, a NOVX -like variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, a NOVX protein or polypeptide can be synthesized chemically using standard peptide synthesis techniques.

An "isolated" or "purified" protein or biologically active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX protein in which the protein is separated from cellular components of the cells from which it is isolated or recombinantly produced. In one embodiment, the language "substantially free of cellular material" includes preparations of NOVX protein having less than about 30% (by dry weight) of non-NOVX protein (also referred to herein as a "contaminating protein"), more preferably less than about 20% of non-NOVX protein, still more preferably less than about 10% of non-NOVX protein, and most preferably less than about 5% non-NOVX protein. When the NOVX protein or biologically active portion thereof is recombinantly produced, it is also preferably substantially free of culture medium, *i*.*e*., culture medium represents less than about 20%, more preferably less than about 10%, and most preferably less than about 5% of the volume of the protein preparation.

The language "substantially free of chemical precursors or other chemicals" includes preparations of NOVX protein in which the protein is separated from chemical precursors or other chemicals that are involved in the synthesis of the protein. In one embodiment, the language "substantially free of chemical precursors or other chemicals" includes preparations of NOVX protein having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically active portions of a NOVX protein include peptides comprising amino acid sequences sufficiently homologous to or derived from the amino acid sequence of the NOVX protein, *e.g.,* the amino acid sequence shown in SEQ ID NO:2n, wherein n is an integer between 1-16 that include fewer amino acids than the full length NOVX proteins, and exhibit at least one activity of a NOVX protein. Typically, biologically active portions comprise a domain or motif with at least one activity of the NOVX protein. A biologically active portion of a NOVX protein can be a polypeptide which is, for example, 10, 25, 50, 100 or more amino acids in length.

A biologically active portion of a NOVX protein of the present invention may contain at least one of the above-identified domains conserved between the NOVX proteins, *e*.*g*. TSR modules. Moreover, other biologically active portions, in which other regions of the protein are deleted, can be prepared by recombinant techniques and evaluated for one or more of the functional activities of a native NOVX protein.

In an embodiment, the NOVX protein has an amino acid sequence shown in SEQ ID NO:2n, wherein n is an integer between 1-16. In other embodiments, the NOVX protein is substantially homologous to SEQ ID NO:2n, wherein n is an integer between 1-16 and retains the functional activity of the protein of SEQ ID NO:2n, wherein n is an integer between 1-16 yet differs in amino acid sequence due to natural allelic variation or mutagenesis, as described in detail below. Accordingly, in another embodiment, the NOVX protein is a protein that comprises an amino acid sequence at least about 45% homologous to the amino acid sequence of SEQ ID NO:2n, wherein n is an integer between 1-16 and retains the functional activity of the NOVX proteins of SEQ ID NO:2n, wherein n is an integer between 1-16.

### Determining homology between two or more sequence

To determine the percent homology of two amino acid sequences or of two nucleic acids, the sequences are aligned for optimal comparison purposes (*e*.*g*., gaps can be introduced in either of the sequences being compared for optimal alignment between the sequences). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i*.*e*., as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. See, *Needleman and Wunsch* 1970 *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NO:2n-1, wherein n is an integer between 1-16.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (*e*.*g*., A, T, C, G, U, or I, in the case of nucleic acids) occurs in both sequences to yield the number of matched positions, dividing the number of matched positions by the total number of positions in the region of comparison (*i*.*e*., the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region. The term "percentage of positive residues" is calculated by comparing two optimally aligned sequences over that region of comparison, determining the number of positions at which the identical and conservative amino acid substitutions, as defined above, occur in both sequences to yield the number of matched positions, dividing the number of matched positions by the total number of positions in the region of comparison (*i*.*e*., the window size), and multiplying the result by 100 to yield the percentage of positive residues.

### Chimeric and fusion proteins

The invention also provides NOVX chimeric or fusion proteins. As used herein, a NOVX "chimeric protein" or "fusion protein" comprises a NOVX polypeptide operatively linked to a non-NOVX polypeptide. An "NOVX polypeptide" refers to a polypeptide having an amino acid sequence corresponding to NOVX, whereas a "non-NOVX polypeptide" refers to a polypeptide having an amino acid sequence corresponding to a protein that is not substantially homologous to the NOVX protein, *e*.*g*., a protein that is different from the NOVX protein and that is derived from the same or a different organism. Within a NOVX fusion protein the NOVX polypeptide can correspond to all or a portion of a NOVX protein. In one embodiment, a NOVX fusion protein comprises at least one biologically active portion of a NOVX protein. In another embodiment, a NOVX fusion protein comprises at least two biologically active portions of a NOVX protein. Within the fusion protein, the term "operatively linked" is intended to indicate that the NOVX polypeptide and the non-NOVX polypeptide are fused in-frame to each other. The non-NOVX polypeptide can be fused to the N-terminus or C-terminus of the NOVX polypeptide.

For example, in one embodiment a NOVX fusion protein comprises a NOVX polypeptide operably linked to the extracellular domain of a second protein. Such fusion proteins can be further utilized in screening assays for compounds that modulate NOVX activity (such assays are described in detail below).

In another embodiment, the fusion protein is a GST-NOVX fusion protein in which the NOVX sequences are fused to the C-terminus of the GST (i.e., glutathione S-transferase) sequences. Such fusion proteins can facilitate the purification of recombinant NOVX.

In another embodiment, the fusion protein is a NOVX-immunoglobulin fusion protein in which the NOVX sequences comprising one or more domains are fused to sequences derived from a member of the immunoglobulin protein family. The NOVX-immunoglobulin fusion proteins of the invention can be incorporated into pharmaceutical compositions and administered to a subject to inhibit an interaction between a NOVX ligand and a NOVX protein on the surface of a cell, to thereby suppress NOVX-mediated signal transduction *in vivo*. In one nonlimiting example, a contemplated NOVX ligand of the invention is the NOVX receptor. The NOVX-immunoglobulin fusion proteins can be used to affect the bioavailability of a NOVX cognate ligand. Inhibition of the NOVX ligand/NOVX interaction may be useful therapeutically for both the treatment of proliferative and differentiative disorders, *e*.*g*., cancer as well as modulating (*e*.*g*., promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with a NOVX ligand.

A NOVX chimeric or fusion protein of the invention can be produced by standard recombinant DNA techniques. For example, DNA fragments coding for the different polypeptide sequences are ligated together in-frame in accordance with conventional techniques, *e*.*g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that already encode a fusion moiety (*e*.*g*., a GST polypeptide). A NOVX-encoding nucleic acid can be cloned into such an expression vector such that the fusion moiety is linked in-frame to the NOVX protein.

### NOVX agonists and antagonists

The present invention also pertains to variants of the NOVX proteins that function as either NOVX agonists (mimetics) or as NOVX antagonists. Variants of the NOVX protein can be generated by mutagenesis, *e*.*g*., discrete point mutation or truncation of the NOVX protein. An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX protein that function as either NOVX agonists (mimetics) or as NOVX antagonists can be identified by screening combinatorial libraries of mutants, *e*.*g*., truncation mutants, of the NOVX protein for NOVX protein agonist or antagonist activity. In one embodiment, a variegated library of NOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e*.*g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are known in the art (see, *e.g.,* Narang (1983) *Tetrahedron* 39:3; Itakura *et al*. (1984) *Annu Rev Biochem* 53:323; Itakura *et al*. (1984) *Science* 198:1056; Ike *et al*. (1983) *Nucl Acid Res* 11:477.

### Polypeptide libraries

In addition, libraries of fragments of the NOVX protein coding sequence can be used to generate a variegated population of NOVX fragments for screening and subsequent selection of variants of a NOVX protein. In one embodiment, a library of coding sequence fragments can be generated by treating a double stranded PCR fragment of a NOVX coding sequence with a nuclease under conditions wherein nicking occurs only about once per molecule, denaturing the double stranded DNA, renaturing the DNA to form double stranded DNA that can include sense/antisense pairs from different nicked products, removing single stranded portions from reformed duplexes by treatment with S1 nuclease, and ligating the resulting fragment library into an expression vector. By this method, an expression library can be derived which encodes N-terminal and internal fragments of various sizes of the NOVX protein.

Several techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recrusive ensemble mutagenesis (REM), a new technique that enhances the frequency of functional mutants in the libraries, can be used in combination with the screening assays to identify NOVX variants (Arkin and Yourvan (1992) PNAS 89:7811-7815; Delgrave *et al.* (1993) Protein Engineering 6:327-331).

### NOVX Antibodies

Also included in the invention are antibodies to NOVX proteins, or fragments of NOVX proteins. The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F_{(ab')2} fragments, and an F_{ab} expression library. In general, an antibody molecule obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated NOVX-related protein of the invention may be intended to serve as an antigen, or a portion or fragment thereof, and additionally can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as an amino acid sequence shown in SEQ ID NO: 2, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX-related protein that is located on the surface of the protein, *e*.*g*., a hydrophilic region. A hydrophobicity analysis of the human NOVX-related protein sequence will indicate which regions of a NOVX-related protein are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e*.*g*., Hopp and Woods, 1981, *Proc. Nat. Acad. Sci. USA* 78: 3824-3828; Kyte and Doolittle *1982, J. Mol. Biol*. 157:105-142, each of which is incorporated herein by reference in its entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). Preferably, antibodies having a high degree of specificity and a high binding affinity for the target antigen are isolated.

After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### Human Antibodies

Fully human antibodies relate to antibody molecules in which essentially the entire sequences of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison ( Nature 368, 812-13 (1994)); Fishwild et al,( Nature Biotechnology 14, 845-51 (1996)); Neuberger (Nature Biotechnology 14, 826 (1996)); and Lonberg and Huszar (Intem. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see e.g., U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production ofbispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al*., 1991 *EMBO J*., 10:3655-3659.

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (Fc R), such as Fc RI (CD64), Fc RII (CD32) and Fc RIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

### NOVX Recombinant Expression Vectors and Host Cells

Another aspect of the invention pertains to vectors, preferably expression vectors, containing a nucleic acid encoding a NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e*.*g*., bacterial vectors having a bacterial origin of replication and episomal mammalian vectors). Other vectors (*e*.*g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors ofutility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e*.*g*., in an *in* *vitro* transcription/translation system or in a host cell when the vector is introduced into the host cell).

The term "regulatory sequence" is intended to includes promoters, enhancers and other expression control elements (*e*.*g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e*.*g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e*.*g*., NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al*., (1988) *Gene* 69:301-315) and pET 11d (Studier *et al*., GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e*.*g*., Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g.,* Wada, *et al*., 1992. *Nucl. Acids Res*. 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSecl (Baldari, *et al*., 1987. *EMBO J.* 6: 229-234), pMFa (Kurjan and Herskowitz, 1982. *Cell* 30: 933-943), pJRY88 (Schultz *et al*., 1987. *Gene* 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells (*e*.*g*., SF9 cells) include the pAc series (Smith, *et al*., 1983. *Mol*. *Cell. Biol*. 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaufman, *et al*., 1987. *EMBO J.* 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e*.*g*., Chapters 16 and 17 of Sambrook, *et al*., MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type *(e.g.,* tissue-specific regulatory elements are used to express the nucleic acid). Tissue-specific regulatory elements are known in the art. Non-limiting examples of suitable tissue-specific promoters include the albumin promoter (liver-specific; Pinkert, *et al*., 1987. *Genes Dev.* 1: 268-277), lymphoid-specific promoters (Calame and Eaton, 1988. *Adv. Immunol.* 43: 235-275), in particular promoters of T cell receptors (Winoto and Baltimore, 1989. *EMBO J.* 8: 729-733) and immunoglobulins (Banerji, *et al*., 1983. *Cell* 33: 729-740; Queen and Baltimore, 1983. *Cell* 33: 741-748), neuron-specific promoters (*e*.*g*., the neurofilament promoter; Byme and Ruddle, 1989. *Proc. Natl. Acad. Sci. USA* 86: 5473-5477), pancreas-specific promoters (Edlund, *et al*., 1985. *Science* 230: 912-916), and mammary gland-specific promoters (*e*.*g*., milk whey promoter; U.S. Pat. No. 4,873,316 and European Application Publication No. 264,166). Developmentally-regulated promoters are also encompassed, *e.g.,* the murine hox promoters (Kessel and Gruss, 1990. *Science* 249: 374-379) and the α-fetoprotein promoter (Campes and Tilghman, 1989. *Genes Dev.* 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense *genes see, e*.*g*., Weintraub, *et al*., "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as human, Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid (*e*.*g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al*. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e*.*g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (*e*.*g*., cells that have incorporated the selectable marker gene will survive, while the other cells die).

A host cell of the invention, such as a prokaryotic or eukaryotic host cell in culture, can be used to produce (*i*.*e*., express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g*., an embryonic cell of the animal, prior to development of the animal.

A transgenic animal of the invention can be created by introducing NOVX-encoding nucleic acid into the male pronuclei of a fertilized oocyte (*e*.*g*., by microinjection, retroviral infection) and allowing the oocyte to develop in a pseudopregnant female foster animal. Sequences including SEQ ID NO:2n-1, wherein n is an integer between 1-16can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan, 1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX transgene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of a NOVX gene into which a deletion, addition or substitution has been introduced to thereby alter, *e*.*g*., functionally disrupt, the NOVX gene. The NOVX gene can be a human gene (*e*.*g*., the DNA of SEQ ID NO:2n-1, wherein n is an integer between 1-16), but more preferably, is a non-human homologue of a human NOVX gene. For example, a mouse homologue of human NOVX gene of SEQ ID NO:2n-1, wherein n is an integer between 1-16can be used to construct a homologous recombination vector suitable for altering an endogenous NOVX gene in the mouse genome. In one embodiment, the vector is designed such that, upon homologous recombination, the endogenous NOVX gene is functionally disrupted (*i*.*e*., no longer encodes a functional protein; also referred to as a "knock out" vector).

Alternatively, the vector can be designed such that, upon homologous recombination, the endogenous NOVX gene is mutated or otherwise altered but still encodes functional protein (*e*.*g*., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e*.*g*., Thomas, *et al*., 1987. *Cell* 51: 503 for a description of homologous recombination vectors. The vector is ten introduced into an embryonic stem cell line (*e*.*g*., by electroporation) and cells in which the introduced NOVX gene has homologously-recombined with the endogenous NOVX gene are selected. *See, e.g.,* Li, *et al*., 1992. *Cell* 69: 915.

The selected cells are then injected into a blastocyst of an animal (*e*.*g*., a mouse) to form aggregation chimeras. *See, e*.*g*., Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr. Opin. Biotechnol.* 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e*.*g*., Lakso, *et al*., 1992. *Proc. Natl. Acad. Sci. USA* 89: 6232-6236. Another example of a recombinase system is the FLP recombinase system of *Saccharomyces cerevisiae. See,* O'Gorman, *et al*., 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g.*, by mating two transgenic animals, one containing a transgene encoding a selected protein and the other containing a transgene encoding a recombinase.

Clones of the non-human transgenic animals described herein can also be produced according to the methods described in Wilmut, *et al*., 1997. *Nature* 385: 810-813. In brief, a cell (*e*.*g*., a somatic cell) from the transgenic animal can be isolated and induced to exit the growth cycle and enter Go phase. The quiescent cell can then be fused, *e*.*g*., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell *(e.g.,* the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The antibodies disclosed herein can also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

A pharmaceutical composition of the invention is formulated to be compatible with its intended route of administration. Examples of routes of administration include parenteral, *e*.*g*., intravenous, intradermal, subcutaneous, oral (*e*.*g*., inhalation), transdermal (*i*.*e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e*.*g*., a NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e*.*g*., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral orparenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see, e*.*g*., U.S. Patent No. 5,328,470) or by stereotactic injection *(see, e.g.,* Chen, *et al*., 1994. *Proc. Natl*. *Acad. Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e*.*g*., retroviral vectors, the pharmaceutical preparation can include one or more cells that produce the gene delivery system.

Antibodies specifically binding a protein of the invention, as well as other molecules identified by the screening assays disclosed herein, can be administered for the treatment of various disorders in the form of pharmaceutical compositions. Principles and considerations involved in preparing such compositions, as well as guidance in the choice of components are provided, for example, in Remington : The Science And Practice Of Pharmacy 19th ed. (Alfonso R. Gennaro, et al., editors) Mack Pub. Co., Easton, Pa. : 1995; Drug Absorption Enhancement : Concepts, Possibilities, Limitations, And Trends, Harwood Academic Publishers, Langhome, Pa., 1994; and Peptide And Protein Drug Delivery (Advances In Parenteral Sciences, Vol. 4), 1991, M. Dekker, New York. If the antigenic protein is intracellular and whole antibodies are used as inhibitors, internalizing antibodies are preferred. However, liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e*.*g*., Marasco *et al*., 1993 *Proc. Natl. Acad. Sci. USA*, 90: 7889-7893. The formulation herein can also contain more than one active compound as necessary for the particular indication being treated, preferably those with complementary activities that do not adversely affect each other. Alternatively, or in addition, the composition can comprise an agent that enhances its function, such as, for example, a cytotoxic agent, cytokine, chemotherapeutic agent, or growth-inhibitory agent. Such molecules are suitably present in combination in amounts that are effective for the purpose intended. The active ingredients can also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions.

The formulations to be used for in vivo administration must be sterile. This is readily accomplished by filtration through sterile filtration membranes.

Sustained-release preparations can be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The pharmaceutical compositions can be included in a container, pack, or dispenser together with instructions for administration.

### Screening and Detection Methods

The isolated nucleic acid molecules of the invention can be used to express NOVX protein (*e*.*g*., via a recombinant expression vector in a host cell in gene therapy applications), to detect NOVX mRNA (*e*.*g*., in a biological sample) or a genetic lesion in a NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein . In addition, the anti-NOVX antibodies of the invention can be used to detect and isolate NOVX proteins and modulate NOVX activity. For example, NOVX activity includes growth and differentiation, antibody production, and tumor growth.

The invention further pertains to novel agents identified by the screening assays described herein and uses thereof for treatments as described, *supra*.

### Screening Assays

The invention provides a method (also referred to herein as a "screening assay") for identifying modulators, *i*.*e*., candidate or test compounds or agents (*e*.*g*., peptides, peptidomimetics, small molecules or other drugs) that bind to NOVX proteins or have a stimulatory or inhibitory effect on, *e*.*g*., NOVX protein expression or NOVX protein activity. The invention also includes compounds identified in the screening assays described herein.

In one embodiment, the invention provides assays for screening candidate or test compounds which bind to or modulate the activity of the membrane-bound form of a NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e*.*g*., Lam, 1997. *Anticancer Drug Design* 12: 145.

A "small molecule" as used herein, is meant to refer to a composition that has a molecular weight of less than about 5 kD and most preferably less than about 4 kD. Small molecules can be, *e*.*g*., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, *et al*., 1993. *Proc. Natl. Acad. Sci. U.S.A*. 90: 6909; Erb, *et al*., 1994. *Proc. Natl. Acad. Sci. U.S.A*. 91: 11422; Zuckermann, *et al*., 1994. *J. Med. Chem.* 37: 2678; Cho, *et al*., 1993. *Science* 261: 1303; Carrell, *et al*., 1994. *Angew. Chem. Int. Ed. Engl.* 33: 2059; Carell, *et al*., 1994. *Angew. Chem. Int. Ed. Engl.* 33: 2061; and Gallop, *et al*., 1994. *J. Med. Chem.* 37: 1233.

Libraries of compounds may be presented in solution (*e*.*g*., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids (Cull, *el al*., 1992. *Proc. Natl. Acad. Sci. USA* 89: 1865-1869) or on phage (Scott and Smith, 1990. *Science* 249: 386-390; Devlin, 1990. *Science* 249: 404-406; Cwirla, *et al*., 1990. *Proc. Natl. Acad. Sci. U.S.A*. 87: 6378-6382; Felici, 1991. *J. Mol. Biol.* 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to a NOVX protein determined. The cell, for example, can be of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (*e*.*g*., stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with a NOVX target molecule. As used herein, a "target molecule" is a molecule with which a NOVX protein binds or interacts in nature, for example, a molecule on the surface of a cell which expresses a NOVX interacting protein, a molecule on the surface of a second cell, a molecule in the extracellular milieu, a molecule associated with the internal surface of a cell membrane or a cytoplasmic molecule. A NOVX target molecule can be a non-NOVX molecule or a NOVX protein or polypeptide of the invention In one embodiment, a NOVX target molecule is a component of a signal transduction pathway that facilitates transduction of an extracellular signal (*e*.*g*. a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with a NOVX target molecule can be accomplished by one of the methods described above for determining direct binding. In one embodiment, determining the ability of the NOVX protein to bind to or interact with a NOVX target molecule can be accomplished by determining the activity of the target molecule. For example, the activity of the target molecule can be determined by detecting induction of a cellular second messenger of the target (*i*.*e*. intracellular Ca²⁺, diacylglycerol, IP₃, etc.), detecting catalytic/enzymatic activity of the target an appropriate substrate, detecting the induction of a reporter gene (comprising a NOVX-responsive regulatory element operatively linked to a nucleic acid encoding a detectable marker, *e*.*g*., luciferase), or detecting a cellular response, for example, cell survival, cellular differentiation, or cell proliferation.

In yet another embodiment, an assay of the invention is a cell-free assay comprising contacting a NOVX protein or biologically-active portion thereof with a test compound and determining the ability of the test compound to bind to the NOVX protein or biologically-active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with a NOVX protein, wherein determining the ability of the test compound to interact with a NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (*e*.*g*. stimulate or inhibit) the activity of the NOVX protein or biologically-active portion thereof. Determining the ability of the test compound to modulate the activity of NOVX can be accomplished, for example, by determining the ability of the NOVX protein to bind to a NOVX target molecule by one of the methods described above for determining direct binding. In an alternative embodiment, determining the ability of the test compound to modulate the activity of NOVX protein can be accomplished by determining the ability of the NOVX protein further modulate a NOVX target molecule. For example, the catalytic/enzymatic activity of the target molecule on an appropriate substrate can be determined as described above.

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with a NOVX protein, wherein determining the ability of the test compound to interact with a NOVX protein comprises determining the ability of the NOVX protein to preferentially bind to or modulate the activity of a NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, N-dodecyl--N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (*e*.*g*., at physiological conditions for salt and pH). Following incubation, the beads or microtiter plate wells are washed to remove any unbound components, the matrix immobilized in the case of beads, complex determined either directly or indirectly, for example, as described, *supra*. Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (*e*.*g*., biotinylation kit, Pierce Chemicals, Rockford, III.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (*i*.*e*., statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay (*see, e*.*g*., U.S. Patent No. 5,283,317; Zervos, *et al*., 1993. *Cell* 72: 223-232; Madura, *et al*., 1993. *J. Biol. Chem.* 268: 12046-12054; Bartel, *et al*., 1993. *Biotechniques* 14: 920-924; Iwabuchi, *et al*., 1993. *Oncogene* 8: 1693-1696; and Brent WO 94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e*.*g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo*, forming a NOVX-dependent complex, the DNA-binding and activation domains of the transcription factor are brought into close proximity. This proximity allows transcription of a reporter gene (*e*.*g*., LacZ) that is operably linked to a transcriptional regulatory site responsive to the transcription factor. Expression of the reporter gene can be detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) identify an individual from a minute biological sample (tissue typing); and (*ii*) aid in forensic identification of a biological sample. Some of these applications are described in the subsections, below.

### Tissue Typing

The NOVX sequences of the invention can be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NO:2n-1, wherein n is an integer between 1-16are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (*e*.*g*., blood, serum, cells, tissue) to thereby determine whether an individual is afflicted with a disease or disorder, or is at risk of developing a disorder, associated with aberrant NOVX expression or activity. Disorders associated with aberrant NOVX expression of activity include, for example, cell proliferative, immunological, inflammatory, and tumor-related disorders and/or pathologies.

The invention also provides for prognostic (or predictive) assays for determining whether an individual is at risk of developing a disorder associated with NOVX protein, nucleic acid expression or activity. For example, mutations in a NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents (*e*.*g*., drugs) for therapeutic or prophylactic treatment of an individual based on the genotype of the individual (*e*.*g*., the genotype of the individual examined to determine the ability of the individual to respond to a particular agent.)

Yet another aspect of the invention pertains to monitoring the influence of agents (*e.g.*, drugs, compounds) on the expression or activity of NOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid (*e*.*g*., mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NO:2n-1, wherein n is an integer between 1-16, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

One agent for detecting NOVX protein is an antibody capable of binding to NOVX protein, preferably an antibody with a detectable label. Antibodies directed against a protein of the invention may be used in methods known within the art relating to the localization and/or quantitation of the protein (e.g., for use in measuring levels of the protein within appropriate physiological samples, for use in diagnostic methods, for use in imaging the protein, and the like). In a given embodiment, antibodies against the proteins, or derivatives, fragments, analogs or homologs thereof, that contain the antigen binding domain, are utilized as pharmacologically-active compounds.

An antibody specific for a protein of the invention can be used to isolate the protein by standard techniques, such as immunoaffinity chromatography or immunoprecipitation. Such an antibody can facilitate the purification of the natural protein antigen from cells and of recombinantly produced antigen expressed in host cells. Moreover, such an antibody can be used to detect the antigenic protein (e.g., in a cellular lysate or cell supernatant) in order to evaluate the abundance and pattern of expression of the antigenic protein. Antibodies directed against the protein can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, -galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

Antibodies can be polyclonal, or more preferably, monoclonal. An intact antibody, or a fragment thereof (*e*.*g*., Fab or F(ab')₂) can be used. The term "labeled", with regard to the probe or antibody, is intended to encompass direct labeling of the probe or antibody by coupling (*i*.*e*., physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection ofNOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection of NOVX protein include introducing into a subject a labeled anti-NOVX antibody. For example, the antibody can be labeled with a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In one embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Such disorders include for example cell proliferative, immunological, inflammatory, and tumor-related disorders and/or pathologies.

Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid (*e*.*g*., mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e*.*g*., serum), cell sample, or tissue.

Furthermore, the prognostic assays described herein can be used to determine whether a subject can be administered an agent (*e*.*g*., an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e*.*g*., wherein the presence ofNOVX protein or nucleic acid is diagnostic for a subject that can be administered the agent to treat a disorder associated with aberrant NOVX expression or activity).

The methods of the invention can also be used to detect genetic lesions in a NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding a NOVX-protein, or the misexpression of the NOVX gene. For example, such genetic lesions can be detected by ascertaining the existence of at least one of: (*i*) a deletion of one or more nucleotides from a NOVX gene; (*ii*) an addition of one or more nucleotides to a NOVX gene; (*iii*) a substitution of one or more nucleotides of a NOVX gene, (*iv*) a chromosomal rearrangement of a NOVX gene; (*v*) an alteration in the level of a messenger RNA transcript of a NOVX gene, (*vi*) aberrant modification of a NOVX gene, such as of the methylation pattern of the genomic DNA, (*vii*) the presence of a non-wild-type splicing pattern of a messenger RNA transcript of a NOVX gene, (*viii*) a non-wild-type level of a NOVX protein, (*ix*) allelic loss of a NOVX gene, and (*x*) inappropriate post-translational modification of a NOVX protein. As described herein, there are a large number of assay techniques known in the art which can be used for detecting lesions in a NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) *(see, e*.*g*., Landegran, *et al*., 1988. *Science* 241: 1077-1080; and Nakazawa, *et al*., 1994. *Proc. Natl. Acad. Sci. USA* 91: 360-364), the latter of which can be particularly useful for detecting point mutations in the NOVX-gene (*see*, Abravaya, *et al*., 1995. *Nucl. Acids Res*. 23: 675-682). This method can include the steps of collecting a sample of cells from a patient, isolating nucleic acid (*e*.*g*., genomic, mRNA or both) from the cells of the sample, contacting the nucleic acid sample with one or more primers that specifically hybridize to a NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (*see*, Guatelli, *et al*., 1990. *Proc. Natl. Acad Sci. USA* 87: 1874-1878), transcriptional amplification system *(see,* Kwoh, *et al*., 1989. *Proc. Natl. Acad. Sci. USA* 86: 1173-1177); Qβ Replicase *(see,* Lizardi, *et al*, 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in a NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes (*see*, *e*.*g*., U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, *e*.*g*., DNA or RNA, to high-density arrays containing hundreds or thousands of oligonucleotides probes. *See, e.g.,* Cronin, *et al*., 1996. *Human Mutation* 7: 244-255; Kozal, *et al*., 1996. *Nat. Med*. 2: 753-759. For example, genetic mutations in NOVX can be identified in two dimensional arrays containing light-generated DNA probes as described in Cronin, *et al*., *supra*. Briefly, a first hybridization array of probes can be used to scan through long stretches of DNA in a sample and control to identify base changes between the sequences by making linear arrays of sequential overlapping probes. This step allows the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. *Proc. Natl. Acad. Sci. USA* 74: 560 or Sanger, 1977. *Proc. Natl. Acad Sci. USA* 74: 5463. It is also contemplated that any of a variety of automated sequencing procedures can be utilized when performing the diagnostic assays *(see, e*.*g*., Naeve, *et al*., 1995. *Biotechniques* 19: 448), including sequencing by mass spectrometry (see, *e.g.,* PCT International Publication No. WO 94/16101; Cohen, *et al*., 1996. *Adv. Chromatography* 36: 127-162; and Griffin, *et al*., 1993. *Appl. Biochem. Biotechnol*. 38: 147-159).

Other methods for detecting mutations in the NOVX gene include methods in which protection from cleavage agents is used to detect mismatched bases in RNA/RNA or RNA/DNA heteroduplexes. *See, e*.*g*., Myers, *et al*., 1985. *Science* 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S, nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e*.*g*., Cotton, *et al*., 1988. *Proc. Natl. Acad. Sci. USA* 85: 4397; Saleeba, *et al*., 1992. *Methods Enzymol*. 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E. coli* cleaves A at G/A mismatches and the thymidine DNA glycosylase from HeLa cells cleaves T at G/T mismatches. *See, e*.*g*., Hsu, *et al*., 1994. *Carcinogenesis* 15: 1657-1662. According to an exemplary embodiment, a probe based on a NOVX sequence, *e*.*g*., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e*.*g*., U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e*.*g*., Orita, *et al*., 1989. *Proc. Natl. Acad. Sci. USA:* 86: 2766; Cotton, *1993. Mutat. Res*. 285: 125-144; Hayashi, 1992. *Genet. Anal. Tech. Appl*. 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e*.*g*., Keen, *et al*., 1991. *Trends Genet*. 7: 5.

In yet another embodiment, the movement of mutant or wild-type fragments in polyacrylamide gels containing a gradient of denaturant is assayed using denaturing gradient gel electrophoresis (DGGE). *See, e*.*g*., Myers, *et al*., 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e*.*g*., Rosenbaum and Reissner, 1987. *Biophys. Chem*. 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e*.*g*., Saiki, *et al*., 1986. *Nature* 324: 163; Saiki, *et al*., 1989. *Proc. Natl. Acad. Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, *et al*., 1989. *Nucl. Acids Res*. 17: 2437-2448) or at the extreme 3'-terminus of one primer where, under appropriate conditions, mismatch can prevent, or reduce polymerase extension (*see, e.g.,* Prossner, 1993. *Tibtech.* 11: 238). In addition it may be desirable to introduce a novel restriction site in the region of the mutation to create cleavage-based detection. *See, e*.*g*., Gasparini, *et al*., 1992. *Mol. Cell Probes* 6: 1. It is anticipated that in certain embodiments amplification may also be performed using *Taq* ligase for amplification. *See, e.g.,* Barany, 1991. *Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e*.*g*., in clinical settings to diagnose patients exhibiting symptoms or family history of a disease or illness involving a NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity (*e*.*g*., NOVX gene expression), as identified by a screening assay described herein can be administered to individuals to treat (prophylactically or therapeutically) disorders (*e*.*g* cell proliferative, immunological, inflammatory, and tumor-related disorders and/or pathologies.). In conjunction with such treatment, the pharmacogenomics (*i*.*e*., the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (*e*.*g*., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See *e*.*g*., Eichelbaum, 1996. *Clin. Exp. Pharmacol. Physiol.,* 23: 983-985; Linder, 1997. *Clin. Chem*., 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e*.*g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with a NOVX modulator, such as a modulator identified by one of the exemplary screening assays described herein.

### Monitoring of Effects During Clinical Trials

Monitoring the influence of agents (*e*.*g*., drugs, compounds) on the expression or activity of NOVX (*e*.*g*., the ability to modulate aberrant cell proliferation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (*e*.*g*., compound, drug or small molecule) that modulates NOVX activity (*e*.*g*., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression (*i*.*e*., a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (*e*.*g*., an agonist, antagonist, protein, peptide, peptidomimetic, nucleic acid, small molecule, or other drug candidate identified by the screening assays described herein) comprising the steps of (*i*) obtaining a pre-administration sample from a subject prior to administration of the agent; (*ii*) detecting the level of expression of a NOVX protein, mRNA, or genomic DNA in the preadministration sample; (*iii*) obtaining one or more post-administration samples from the subject; (*iv*) detecting the level of expression or activity of the NOVX protein, mRNA, or genomic DNA in the post-administration samples; (*v*) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity ofNOVX to higher levels than detected, *i*.*e*., to increase the effectiveness of the agent. Alternatively, decreased administration of the agent may be desirable to decrease expression or activity of NOVX to lower levels than detected, *i*.*e*., to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i*.*e*., reduce or inhibit) activity. Therapeutics that antagonize activity may be administered in a therapeutic or prophylactic manner. Therapeutics that may be utilized include, but are not limited to: (*i*) an aforementioned peptide, or analogs, derivatives, fragments or homologs thereof; (*ii*) antibodies to an aforementioned peptide; (*iii*) nucleic acids encoding an aforementioned peptide; (*iv*) administration of antisense nucleic acid and nucleic acids that are "dysfunctional" (*i*.*e*., due to a heterologous insertion within the coding sequences of coding sequences to an aforementioned peptide) that are utilized to "knockout" endogenous function of an aforementioned peptide by homologous recombination *(see, e*.*g*., Capecchi, 1989. *Science* 244: 1288-1292); or (*v*) modulators (*i*.*e*., inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase (*i*.*e*., are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (*e*.*g*., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e*.*g*., by Western blot analysis, immunoprecipitation followed by sodium dodecyl sulfate (SDS) polyacrylamide gel electrophoresis, immunocytochemistry, etc.) and/or hybridization assays to detect expression of mRNAs (*e*.*g*., Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, a NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of a NOVX protein, a peptide, a NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed *in vitro (e.g.,* by culturing the cell with the agent) or, alternatively, *in vivo* (*e*.*g*., by administering the agent to a subject). As such, the invention provides methods of treating an individual afflicted with a disease or disorder characterized by aberrant expression or activity of a NOVX protein or nucleic acid molecule. In one embodiment, the method involves administering an agent (*e*.*g*., an agent identified by a screening assay described herein), or combination of agents that modulates (*e*.*g*., up-regulates or down-regulates) NOVX expression or activity. In another embodiment, the method involves administering a NOVX protein or nucleic acid molecule as therapy to compensate for reduced or aberrant NOVX expression or activity.

Stimulation of NOVX activity is desirable in situations in which NOVX is abnormally downregulated and/or in which increased NOVX activity is likely to have a beneficial effect. One example of such a situation is where a subject has a disorder characterized by aberrant cell proliferation and/or differentiation (*e*.*g*., cancer or immune associated ). Another example of such a situation is where the subject has an immunodeficiency disease (*e*.*g*., AIDS).

Antibodies of the invention, including polyclonal, monoclonal, humanized and fully human antibodies, may used as therapeutic agents. Such agents will generally be employed to treat or prevent a disease or pathology in a subject. An antibody preparation, preferably one having high specificity and high affinity for its target antigen, is administered to the subject and will generally have an effect due to its binding with the target. Such an effect may be one of two kinds, depending on the specific nature of the interaction between the given antibody molecule and the target antigen in question. In the first instance, administration of the antibody may abrogate or inhibit the binding of the target with an endogenous ligand to which it naturally binds. In this case, the antibody binds to the target and masks a binding site of the naturally occurring ligand, wherein the ligand serves as an effector molecule. Thus the receptor mediates a signal transduction pathway for which ligand is responsible.

Alternatively, the effect may be one in which the antibody elicits a physiological result by virtue of binding to an effector binding site on the target molecule. In this case the target, a receptor having an endogenous ligand which may be absent or defective in the disease or pathology, binds the antibody as a surrogate effector ligand, initiating a receptor-based signal transduction event by the receptor.

A therapeutically effective amount of an antibody of the invention relates generally to the amount needed to achieve a therapeutic objective. As noted above, this may be a binding interaction between the antibody and its target antigen that, in certain cases, interferes with the functioning of the target, and in other cases, promotes a physiological response. The amount required to be administered will furthermore depend on the binding affinity of the antibody for its specific antigen, and will also depend on the rate at which an administered antibody is depleted from the free volume other subject to which it is administered. Common ranges for therapeutically effective dosing of an antibody or antibody fragment of the invention may be, by way of nonlimiting example, from about 0.1 mg/kg body weight to about 50 mg/kg body weight. Common dosing frequencies may range, for example, from twice daily to once a week.

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

SEQUENCE LISTING

<110> CuraGen Corporation
      Shimkets, Richard
      Lichenstein, Henri
      Vernet, Corine
      Fernandes, Elma

<120> NOVEL POLYPEPTIDES AND NUCLEIC ACIDS ENCODING SAME

<130> 15966-606-061

<140> US00/31543
<141> 2000-11-17

<150> 09/715,417
<151> 2000-11-16

<150> 60/166,336
<151> 1999-11-19

<160> 38

<170> PatentIn Ver. 2.0

<210> 1
<211> 791
<212> DNA
<213> Homo sapiens

<400> 1
attctgatca cagctgggtg gtattctgat cacagctggg tggtattctg atcacagctg 60
ggtggtattc tgatcacagc tgggtggtat tctgatcaca gctgggtggt attctgatca 120
cagctgggtg gtattctgat cacagctggg tggtattctg atcacagctg ggtggtattc 180
tgatcacagc tgggtggtat tctgatcaca gctgggtggt gatgcccaga gtctccattc 240
caccctatcc aattgctgga ggagttgatg acttagatga agacacaccc ccaatagtgt 300
cacaatttcc cgggaccatg gctaaacctc ctggatcatt agccagaagc agcagcctgt 360
gccgttcacg ccgcagcatt gtgccgtcct cgcctcagcc tcagcgagct cagcttgctc 420
cacacgcccc ccacccgtca caccctcggc accctcacca cccgcaacac acaccacact 480
ccttgccttc ccctgatcca gatatcctct cagtgtcaag ttgccctgcg ctttatcgaa 540
atgaagagga ggaagaggcc atttacttct ctgctgaaaa gcaatgtatg atcatagtca 600
ccagcaagat gcctttactg acagaactgg tcttgtgtgg tttctggaaa tcagaaggaa 660
aactcgagag ctgcactgtc taataaaact tcctgcattg atggaacgtt cagttctcat 720
ttcaatagca atgtcaaagt ttcatagcta gctctcataa ataagagaat gatttgaatt 780
tggaaaaaaa a                                                      791

<210> 2
<211> 153

<212> PRT
<213> Homo sapiens

<400> 2
Met Pro Arg Val Ser Ile Pro Pro Tyr Pro Ile Ala Gly Gly Val Asp
1               5                   10                  15

Asp Leu Asp Glu Asp Thr Pro Pro Ile Val Ser Gln Phe Pro Gly Thr
            20                  25                  30

Met Ala Lys Pro Pro Gly Ser Leu Ala Arg Ser Ser Ser Leu Cys Arg
        35                  40                  45

Ser Arg Arg Ser Ile Val Pro Ser Ser Pro Gln Pro Gln Arg Ala Gln
    50                  55                  60

Leu Ala Pro His Ala Pro His Pro Ser His Pro Arg His Pro His His
65                  70                  75                  80

Pro Gln His Thr Pro His Ser Leu Pro Ser Pro Asp Pro Asp Ile Leu
                85                  90                  95

Ser Val Ser Ser Cys Pro Ala Leu Tyr Arg Asn Glu Glu Glu Glu Glu
            100                 105                 110

Ala Ile Tyr Phe Ser Ala Glu Lys Gln Cys Met Ile Ile Val Thr Ser
        115                 120                 125

Lys Met Pro Leu Leu Thr Glu Leu Val Leu Cys Gly Phe Trp Lys Ser
    130                 135                 140

Glu Gly Lys Leu Glu Ser Cys Thr Val
145                 150


<210> 3
<211> 2011
<212> DNA
<213> Homo sapiens

<400> 3
gggtgtgatg ggcttctagt ttctctagct gcatcaccct tgaaccatcc agagtcccag 60
taagccacgg gcttgagcat ggaggagaat cctcagagac agaacccctg cccacatgtc 120
tgggccttgc tcaagccagc aaggggctga atccctgtgt ttcaggactc aggtttgctg 180
agtgtcatca ccgatcccat ccacacccca gtcactctct tctggcccac cgaccaagcc 240
ctccatgccc tacctgctga acaacaggac ttcctgttca accaagacaa caaggacaag 300
ctgaaggagt atttgaagtt tcatgtgata cgagatgcca aggttttagc tgtggatctt 360
cccacatcca ctgcctggaa gaccctgcaa ggttcagagc tgagtgtgaa atgtggagct 420

ggcagggaca tcggtgacct ctttctgaat ggccaaacct gcagaattgt gcagcgggag 480
ctcttgtttg acctgggtgt ggcctacggc attgactgtc tgctgattga tcccaccctg 540
gggggccgct gtgacacctt tactactttc gatgcctcgg gggagtgtgg gagctgtgtc 600
aatactccca gctgcccaag gtggagtaaa ccaaagggtg tgaagcagaa gtgtctctac 660
aacctgccct tcaagaggaa cctggaaggc tgccgggagc gatgcagcct ggtgatacag 720
atccccaggt gctgcaaggg ctacttcggg cgagactgtc aggcctgccc tggaggacca 780
gatgccccgt gtaataaccg gggtgtctgc cttgatcagt actcggccac cggagagtgt 840
aaatgcaaca ccggcttcaa tgggacggcg tgtgagatgt gctggccggg gagatttggg 900
cctgattgtc tgccctgtgg ctgctcagac cacggacagt gcgatgatgg catcacgggc 960
tccgggcagt gcctctgtga aacggggtgg acaggcccct cgtgtgacac tcaggcagtt 1020
ttgtctgcag tgtgtacgcc tccttgttct gctcatgcca cctgtaagga gaacaacacg 1080
tgtgagtgta acctggatta tgaaggtgac ggaatcacat gcacagttgt ggatttctgc 1140
aaacaggaca acgggggctg tgcaaaggtg gccagatgct cccagaaggg cacgaaggtc 1200
tcctgcagct gccagaaggg atacaaaggg gacgggcaca gctgcacaga gatagacccc 1260
tgtgcagacg gccttaacgg agggtgtcac gagcacgcca cctgtaagat gacaggcccg 1320
ggcaagcaca agtgtgagtg taaaagtcac tatgtcggag atgggctgaa ctgtgagccg 1380
gagcagctgc ccattgaccg ctgcttacag gacaatgggc agtgccatgc agacgccaaa 1440
tgtgccgacc tccacttcca ggataccact gttggggtgt tccatctacg ctccccactg 1500
ggccagtata agctgacctt tgacaaagcc agagaggcct gtgccaacga agctgcgacc 1560
atggcaacct acaaccagct ctcctatgcc cagaaggcca agtaccacct gtgctcagca 1620
ggctggctgg agaccgggcg ggttgcctac cccacagcct tcgcctccca gaactgtggc 1680
tctggtgtgg ttgggatagt ggactatgga cccagaccca acaagagtga aatgtgggat 1740
gtcttctgct atcggatgaa aggaagtgct ggcctattcc aacagctcag ctcgaggccg 1800
tgcatttcta gaacacctga ctgacctgtc catccgcggc accctctttg tgccacagaa 1860
cagtgggctg gggagaaatg agaccttgtc tgggcgggac atcgaacacc acctcgccaa 1920
tgtcagcatg tttttctaca atgaccttgt caatggcacc accctgcaaa cgaggctggg 1980
aagcaagctg ctcatcactg ccagccagga c 2011

<210> 4
<211> 315
<212> PRT
<213> Homo sapiens

<400> 4
Met Cys Trp Pro Gly Arg Phe Gly Pro Asp Cys Leu Pro Cys Gly Cys
1               5                   10                  15

Ser Asp His Gly Gln Cys Asp Asp Gly Ile Thr Gly Ser Gly Gln Cys
            20                  25                  30

Leu Cys Glu Thr Gly Trp Thr Gly Pro Ser Cys Asp Thr Gln Ala Val
        35                  40                  45

Leu Ser Ala Val Cys Thr Pro Pro Cys Ser Ala His Ala Thr Cys Lys
    50                  55                  60

Glu Asn Asn Thr Cys Glu Cys Asn Leu Asp Tyr Glu Gly Asp Gly Ile
65                  70                  75                  80

Thr Cys Thr Val Val Asp Phe Cys Lys Gln Asp Asn Gly Gly Cys Ala
                85                  90                  95

Lys Val Ala Arg Cys Ser Gln Lys Gly Thr Lys Val Ser Cys Ser Cys
            100                 105                 110

Gln Lys Gly Tyr Lys Gly Asp Gly His Ser Cys Thr Glu Ile Asp Pro
        115                 120                 125

Cys Ala Asp Gly Leu Asn Gly Gly Cys His Glu His Ala Thr Cys Lys
    130                 135                 140

Met Thr Gly Pro Gly Lys His Lys Cys Glu Cys Lys Ser His Tyr Val
145                 150                 155                 160

Gly Asp Gly Leu Asn Cys Glu Pro Glu Gln Leu Pro Ile Asp Arg Cys
                165                 170                 175

Leu Gln Asp Asn Gly Gln Cys His Ala Asp Ala Lys Cys Ala Asp Leu
            180                 185                 190

His Phe Gln Asp Thr Thr Val Gly Val Phe His Leu Arg Ser Pro Leu
        195                 200                 205

Gly Gln Tyr Lys Leu Thr Phe Asp Lys Ala Arg Glu Ala Cys Ala Asn
    210                 215                 220

Glu Ala Ala Thr Met Ala Thr Tyr Asn Gln Leu Ser Tyr Ala Gln Lys
225                 230                 235                 240

Ala Lys Tyr His Leu Cys Ser Ala Gly Trp Leu Glu Thr Gly Arg Val
                245                 250                 255

Ala Tyr Pro Thr Ala Phe Ala Ser Gln Asn Cys Gly Ser Gly Val Val
            260                 265                 270

Gly Ile Val Asp Tyr Gly Pro Arg Pro Asn Lys Ser Glu Met Trp Asp
        275                 280                 285

Val Phe Cys Tyr Arg Met Lys Gly Ser Ala Gly Leu Phe Gln Gln Leu
    290                 295                 300

Ser Ser Arg Pro Cys Ile Ser Arg Thr Pro Asp
305                 310                 315

<210> 5

<211> 1804
<212> DNA
<213> Homo sapiens

<400> 5
gggtgtgatg ggcttctagt ttctctagct gcatcaccct tgaaccatcc agagtcccag 60
taagccacgg gcttgagcat ggaggagaat cctcagagac agaacccctg cccacatgtc 120
tgggccttgc tcaagccagc aaggggctga atccctgtgt ttcaggactc aggtttgctg 180
agtgtcatca ccgatcccat ccacacccca gtcactctct tctggcccac cgaccaagcc 240
ctccatgccc tacctgctga acaacaggac ttcctgttca accaagacaa caaggacaag 300
ctgaaggagt atttgaagtt tcatgtgata cgagatgcca aggttttagc tgtggatctt 360
cccacatcca ctgcctggaa gaccctgcaa ggttcagagc tgagtgtgaa atgtggagct 420
ggcagggaca tcggtgacct ctttctgaat ggccaaacct gcagaattgt gcagcgggag 480
ctcttgtttg acctgggtgt ggcctacggc attgactgtc tgctgattga tcccaccctg 540
gggggccgct gtgacacctt tactactttc gatgcctcgg gggagtgtgg gagctgtgtc 600
aatactccca gctgcccaag gtggagtaaa ccaaagggtg tgaagcagaa gtgtctctac 660
aacctgccct tcaagaggaa cctggaaggc tgccgggagc gatgcagcct ggtgatacag 720
atccccaggt gctgcaaggg ctacttcggg cgagactgtc aggcctgccc tggaggacca 780
gatgccccgt gtaataaccg gggtgtctgc cttgatcagt actcggccac cggagagtgt 840
aaatgcaaca ccggcttcaa tgggacggcg tgtgagatgt gctggccggg gagatttggg 900
cctgattgtc tgccctgtgg ctgctcagac cacggacagt gcgatgatgg catcacgggc 960
tccgggcagt gcctctgtga aacggggtgg acaggcccct cgtgtgacac tcaggcagtt 1020
ttgtctgcag tgtgtacgcc tccttgttct gctcatgcca cctgtaagga gaacaacacg 1080
tgtgagtgta acctggatta tgaaggtgac ggaatcacat gcacagttgt ggatttctgc 1140
aaacaggaca acgggggctg tgcaaaggtg gccagatgct cccagaaggg cacgaaggtc 1200
tcctgcagct gccagaaggg atacaaaggg gacgggcaca gctgcacaga gatagacccc 1260
tgtgcagacg gccttaacgg agggtgtcac gagcacgcca cctgtaagat gacaggcccg 1320
ggcaagcaca agtgtgagtg taaaagtcac tatgtcggag atgggctgaa ctgtgagccg 1380
gagcagctgc ccattgaccg ctgcttacag gacaatgggc agtgccatgc agacgccaaa 1440
tgtgtcgacc tccacttcca ggataccact gttggggtgt tccatctacg ctccccactg 1500
ggccagtata agctgacctt tgacaaagcc agagaggcct gtgccaacga agctgcgacc 1560
atggcaacct acaaccagct ctcctatgcc cagaagagag aagagaaatg agtatgaaag 1620
acctgggcac ctacaagaaa gagaggacac ttttgttcac ccagtggctc aatcaaccag 1680
tcaacatcta atgaccacct actgtgtgcc aggcacagag gtgcaatagg caaagccaag 1740
taccacctgt gctcagcagg ctggctggag accgggcggg ttgcctaccc cacagccttc 1800
gcct 1804

<210> 6
<211> 244
<212> PRT
<213> Homo sapiens

<400> 6
Met Cys Trp Pro Gly Arg Phe Gly Pro Asp Cys Leu Pro Cys Gly Cys
1               5                   10                  15

Ser Asp His Gly Gln Cys Asp Asp Gly Ile Thr Gly Ser Gly Gln Cys
            20                  25                  30

Leu Cys Glu Thr Gly Trp Thr Gly Pro Ser Cys Asp Thr Gln Ala Val
        35                  40                  45

Leu Ser Ala Val Cys Thr Pro Pro Cys Ser Ala His Ala Thr Cys Lys
    50                  55                  60

Glu Asn Asn Thr Cys Glu Cys Asn Leu Asp Tyr Glu Gly Asp Gly Ile
65                  70                  75                  80

Thr Cys Thr Val Val Asp Phe Cys Lys Gln Asp Asn Gly Gly Cys Ala
                85                  90                  95

Lys Val Ala Arg Cys Ser Gln Lys Gly Thr Lys Val Ser Cys Ser Cys
            100                 105                 110

Gln Lys Gly Tyr Lys Gly Asp Gly His Ser Cys Thr Glu Ile Asp Pro
        115                 120                 125

Cys Ala Asp Gly Leu Asn Gly Gly Cys His Glu His Ala Thr Cys Lys
    130                 135                 140

Met Thr Gly Pro Gly Lys His Lys Cys Glu Cys Lys Ser His Tyr Val
145                 150                 155                 160

Gly Asp Gly Leu Asn Cys Glu Pro Glu Gln Leu Pro Ile Asp Arg Cys
                165                 170                 175

Leu Gln Asp Asn Gly Gln Cys His Ala Asp Ala Lys Cys Val Asp Leu
            180                 185                 190

His Phe Gln Asp Thr Thr Val Gly Val Phe His Leu Arg Ser Pro Leu
        195                 200                 205

Gly Gln Tyr Lys Leu Thr Phe Asp Lys Ala Arg Glu Ala Cys Ala Asn
    210                 215                 220

Glu Ala Ala Thr Met Ala Thr Tyr Asn Gln Leu Ser Tyr Ala Gln Lys
225                 230                 235                 240

Arg Glu Glu Lys

<210> 7
<211> 1450
<212> DNA
<213> Homo sapiens

<220>
<221> misc_feature
<222> (31)
<223> wherein n is g or a or t or c

<400> 7
cggcctgtta tttccttttg cgcgacacgg nctcagctgt tgcgcctttg gcgagtgacg 60
ctggccgcca acgaggtata cgtactggga ccctcgccct cagtctcgtc tccggcgcgg 120
ctacctgccc cgttttccct gtgagttgac ctgctccggg ccgcgggcgc caatggcagg 180
ggccgctccg accacggcct tcgggcaggc ggtgatcggc ccgccggcgt cagggaagac 240
cacgtactgc ctgggcatga gtgagttcct gcgcgcgctg ggccggcgct tggcggtgtg 300
tgaacctgga cccggccaac gaggggctgc cgtacgagtg tgccgtggac gtgggcgagc 360
tggtggggct gggcgacgtg atggacgcgc tgcccttggg ggcccaacgg cggcctgctc 420
tactgcatgg agtacctgga agccaacctg gactggctgc gtgccaagct cgaccccctc 480
cgcggccact acttcctctt cgactgccca ggccaggtgg agctctgcac gcatcacggc 540
gccttgcgag catcttctcc caaatggcgc agtgggacct caggctgact gccgtccacc 600
tcgtggattc tcactactgc acagaccctg ccaagttcat ttcagtactg tgtacctccc 660
tggccaccat gctgcacgtg gaactgagcc cacatcaacc tcctttccaa gatggacctc 720
attgagcatt atgggaagct ggccttcaac ctggactact acacagaggt tctggacctc 780
tcctacctgc ttgaccacct ggcttctgac cctttcttcc gccactaccg ccagctcaat 840
gagaagctag tgcagctcat cgaagactat agccttgtct cctttatccc tctcaacatc 900
caggacaagg agagcatcca gcgagtcctt caggctgtgg ataaagccaa tggatactgt 960
ttcggagccc aagagcagcg aagcttggaa gccatgatgt ctgccgcaat gggagccgac 1020
ttccatttct cttccacact gggcatccag gagaagtacc tggcaccctc gaaccagtca 1080
gtggagcagg aagccatgca gctgtagcaa caaggtggac cctggagagc aggatgcata 1140
atccagcact ggggaaagtg gaggctcctg atgcaggctg cagacccaag agcaagtcct 1200
cccagccaga gctggcgggc tggcaagggg atattcagct ctgcaaagga cttctggcca 1260
aaaagccaga catggtgcca agcagaacac cccccatact gtcagtggtg tccgtgagct 1320
ctgggccctg ccaccagaaa gtcgagcact ggtcctagtc aggctgtgat gaaatgtgct 1380
acaatacaag agtttatttt ctaaaaaaaa aaaaaaaaaa ccgcggcggc tcccacttca 1440
gattggtaac 1450

<210> 8
<211> 225
<212> PRT
<213> Homo sapiens

<400> 8
Met Ala Gly Ala Ala Pro Thr Thr Ala Phe Gly Gln Ala Val Ile Gly
1               5                   10                  15

Pro Pro Ala Ser Gly Lys Thr Thr Tyr Cys Leu Gly Met Ser Glu Phe
            20                  25                  30

Leu Arg Ala Leu Gly Arg Arg Leu Ala Val Cys Glu Pro Gly Pro Gly
        35                  40                  45

Gln Arg Gly Ala Ala Val Arg Val Cys Arg Gly Arg Gly Arg Ala Gly
     50                  55                  60

Gly Ala Gly Arg Arg Asp Gly Arg Ala Ala Leu Gly Gly Pro Thr Ala
 65                  70                  75                  80

Ala Cys Ser Thr Ala Trp Ser Thr Trp Lys Pro Thr Trp Thr Gly Cys
                 85                  90                  95

Val Pro Ser Ser Thr Pro Ser Ala Ala Thr Thr Ser Ser Ser Thr Ala
            100                 105                 110

Gln Ala Arg Trp Ser Ser Ala Arg Ile Thr Ala Pro Cys Glu His Leu
        115                 120                 125

Leu Pro Asn Gly Ala Val Gly Pro Gln Ala Asp Cys Arg Pro Pro Arg
    130                 135                 140

Gly Phe Ser Leu Leu His Arg Pro Cys Gln Val His Phe Ser Thr Val
145                 150                 155                 160

Tyr Leu Pro Gly His His Ala Ala Arg Gly Thr Glu Pro Thr Ser Thr
                165                 170                 175

Ser Phe Pro Arg Trp Thr Ser Leu Ser Ile Met Gly Ser Trp Pro Ser
            180                 185                 190

Thr Trp Thr Thr Thr Gln Arg Phe Trp Thr Ser Pro Thr Cys Leu Thr
        195                 200                 205

Thr Trp Leu Leu Thr Leu Ser Ser Ala Thr Thr Ala Ser Ser Met Arg
    210                 215                 220

Ser
225

<210> 9
<211> 1324
<212> DNA
<213> Homo sapiens

<400> 9
ggactgaaga gtagtagtgt gggctgggac cgctggcact catcctgcct gtccccccgc 60
aggtggcaat ggtggaggtg cagctggacg ctgaccacga ctacccaccg gggctgctca 120
tcgccttcag tgcctgcacc acagtgctgg tggctgtgca cctgtttgcg ctcatgatca 180
gcacctgcat cctgcccaac atcgaggcgg tgagcaacgt gcacaatctc aactcggtca 240
aggagtcccc ccatgagcgc atgcaccgcc acatcgagct ggcctgggcc ttctccaccg 300

tcatcggcac gctgctcttc ctagctgagg tggtgctgct ctgctgggtc aagttcttgc 360
ccctcaagaa gcagccaggc cagccaaggc ccaccagcaa gccccccgcc agtggcgcag 420
cagccaacgt cagcaccagc ggcatcaccc cgggccaggc agctgccatc gcctcgacca 480
ccatcatggt gcccttcggc ctgatcttta tcgtcttcgc cgtccacttc taccgctcac 540
tggttagcca taagactgac cgacagttcc aggagctcaa cgagctggcg gagtttgccc 600
gcttacagga ccagctggac cacagagggg accaccccct gacgcccggc agccactatg 660
cctaggccca tgtggtctgg gcccttccag tgctttggcc ttacgccctt ccccttgacc 720
ttgtcctgcc ccagcctcac ggacagcctg cgcagggggc tgggcttcag caaggggcag 780
agcatggagg gaagaggatt tttataagag aaatttctgc actttgaaac tgtcctctaa 840
gagaataagc atttcctgtt cttccagctc caggtccacc tcctgttggg aggcggtggg 900
gggccaaagt ggggccacac actcgctgtg tcccctctcc tcccctgtgc cagtgccacc 960
tgggtgcctc ctcctgtcct gtccgtctca acctccctcc cgtccagcat tgagtgtgta 1020
catgtgtgtg tgacacataa atatactcat aaggacacct ccttcccgtg tcttgtattt 1080
gttgggcctg ggctactgct caccctggtt aggtgagcct ctaggaaaac ttaaaacaaa 1140
ttttaagcca ggtatggtgg cacatacctg tggtctcagc tattcaggag gccaaggcag 1200
gaggatctct tgagcccagg agtttgagac cccatctcaa acaaaaaata caaaaattag 1260
ccagccacgg cgcctgcact tccagctcct ttgagagact gaggcaggaa gattgcctaa 1320
gccc 1324

<210> 10
<211> 198
<212> PRT
<213> Homo sapiens

<400> 10
Met Val Glu Val Gln Leu Asp Ala Asp His Asp Tyr Pro Pro Gly Leu
1               5                   10                  15

Leu Ile Ala Phe Ser Ala Cys Thr Thr Val Leu Val Ala Val His Leu
            20                  25                  30

Phe Ala Leu Met Ile Ser Thr Cys Ile Leu Pro Asn Ile Glu Ala Val
        35                  40                  45

Ser Asn Val His Asn Leu Asn Ser Val Lys Glu Ser Pro His Glu Arg
    50                  55                  60

Met His Arg His Ile Glu Leu Ala Trp Ala Phe Ser Thr Val Ile Gly
65                  70                  75                  80

Thr Leu Leu Phe Leu Ala Glu Val Val Leu Leu Cys Trp Val Lys Phe
                85                  90                  95

Leu Pro Leu Lys Lys Gln Pro Gly Gln Pro Arg Pro Thr Ser Lys Pro
            100                 105                 110

Pro Ala Ser Gly Ala Ala Ala Asn Val Ser Thr Ser Gly Ile Thr Pro
        115                 120                 125

Gly Gln Ala Ala Ala Ile Ala Ser Thr Thr Ile Met Val Pro Phe Gly
    130                 135                 140

Leu Ile Phe Ile Val Phe Ala Val His Phe Tyr Arg Ser Leu Val Ser
145                 150                 155                 160

His Lys Thr Asp Arg Gln Phe Gln Glu Leu Asn Glu Leu Ala Glu Phe
                165                 170                 175

Ala Arg Leu Gln Asp Gln Leu Asp His Arg Gly Asp His Pro Leu Thr
            180                 185                 190

Pro Gly Ser His Tyr Ala
        195

<210> 11
<211> 2512
<212> DNA
<213> Homo sapiens

<400> 11
atagggctcg agcggctgcc cgggcaggtc tcatgcctca gcctccggag tagtattttt 60
agtagagatg gtgtttacca tgtgggccag gctggtctcg aactcctggc ctcaagtgat 120
ccacccgcct cggcctccca gagtgctggg attacaggca tgagccactg cacccagcct 180
tgtttgtatt ttgaattcca aatggaaata ccttcatgat cttcccacta ctaaaggttt 240
aaatctggca ctgatacctc tccaagaggg ctatatacta tgcagtgttt cccagcatgt 300
ttcacaagaa aattcttttt tgaggatcat ctcacagaac ttgggatctt tgcaacatgt 360
attgtgaaat ccaggccaga ggaaccccat gttccttcca cactgatatt ccacaatgga 420
ggcaagaaag gagctagagt cacttcctcc cttttgtctg aacagcctcc actctataat 480
cctgaccaca aagcttactt cccagagtct gggtgggccg agaggtgtgg aagagagaat 540
ggaggacagg agagccaaat ggcacattgc agcaaaagac tcctgcctct ggctgaaacc 600
ctctgatctt ctgttacagg ttaaagactg ggacaaatac ggtttaatgc cccaggttct 660
tcggtaccat gtggtcgcct gccaccagct gcttctggaa aacctgaaat tgatctcaaa 720
tgctacttcc ctccaaggag agccaatagt catctccgtc tctcagagca cggtgtatat 780
aaacaataag gctaagatca tatccagtga tatcatcagt actaatggga ttgttcatat 840
catagacaaa ttgctatctc ccaaaaattt gcttatcact cccaaagaca actctggaag 900
aattctgcaa aatcttacga ctttggcaac aaacaatggc tacatcaaat ttagcaactt 960
aatacaggac tcaggtttgc tgagtgtcat caccgatccc atccacaccc cagtcactct 1020
cttctggccc accgaccaag ccctccatgc cctacctgct gaacaacagg acttcctgtt 1080
caaccaagac aacaaggaca agctgaagga gtatttgaag tttcatgtga tacgagatgc 1140
caaggtttta gctgtggatc ttcccacatc cactgcctgg aagaccctgc aaggttcaga 1200
gctgagtgtg aaatgtggag ctggcaggga catcggtgac ctctttctga atggccaaac 1260
ctgcagaatt gtgcagcgcg agctcttgtt tgacctgggt gtggcctacg gcattgactg 1320
tctgctgatt gatcccaccc tggggggccg ctgtgacacc tttactactt tcgatgcctc 1380
gggggagtgt gggagctgtg tcaatactcc cagctgccca aggtggagta aaccaaaggg 1440
tgtgaagcag aagtgtctct acaacctgcc cttcaagagg aacctggaag gctgccggga 1500

gcggtgcagc ctggtgatac agatccccag gtgctgcaag ggctacttcg ggcgagactg 1560
tcaggcctgc cctggaggac cagttgcccc gtgtaataac cggggtgtct gccttgatca 1620
gtactcggcc accggagagt gtaaatgcaa caccggcttc aatgggacgg cgtgtgagat 1680
gtgctggccg gggagatttg ggcctgattg tctgccctgt ggctgctcag accacggaca 1740
gtgcgatgat ggcatcacgg gctccgggca gtgcctctgt gaaacggggt ggacaggccc 1800
ctcgtgtgac actcaggcag ttttgtctgc agtgtgtacg cctccttgtt ctgctcatgc 1860
cacctgtaag gagaacaaca cgtgtgagtg taacctggat tatgaaggtg acggaatcac 1920
atgcacagtt gtggatttct gcaaacagga caacgggggc tgtgcaaagg tggccagatg 1980
ctcccagaag ggcacgaagg tctcctgcag ctgccagaag ggatacaaag gggacgggca 2040
cagctgcaca gagatagacc cctgtgcaga cggccttaac ggagggtgtc acgagcacgc 2100
cacctgtaag atgacaggcc cgggcaagca caagtgtgag tgtaaaagtc actatgtcgg 2160
agatgggctg aactgtgagc cggagcagct gcccattgac cgctgcttac aggacaatgg 2220
gcagtgccat gcagacgcca aatgtgtcga cctccacttc caggatacca ctgttggggt 2280
gttccatcta cgctccccac tgggccagta taagctgacc tttgacaaag ccagagaggc 2340
ctgtgccaac gaagctgcga ccatggcaac ctacaaccag ctctcctatg cccagaagac 2400
ctggtattcc tttaccaagg aataaagcct ttgatgccag gacccagact caaggagaat 2460
ctgaatctct gctctcctgc ttgctggtca tgtggccttg atatcaagcc ac 2512

<210> 12
<211> 669
<212> PRT
<213> Homo sapiens

<400> 12
Met Glu Ala Arg Lys Glu Leu Glu Ser Leu Pro Pro Phe Cys Leu Asn
1               5                   10                  15

Ser Leu His Ser Ile Ile Leu Thr Thr Lys Leu Thr Ser Gln Ser Leu
            20                  25                  30

Gly Gly Pro Arg Gly Val Glu Glu Arg Met Glu Asp Arg Arg Ala Lys
        35                  40                  45

Trp His Ile Ala Ala Lys Asp Ser Cys Leu Trp Leu Lys Pro Ser Asp
    50                  55                  60

Leu Leu Leu Gln Val Lys Asp Trp Asp Lys Tyr Gly Leu Met Pro Gln
65                  70                  75                  80

Val Leu Arg Tyr His Val Val Ala Cys His Gln Leu Leu Leu Glu Asn
                85                  90                  95

Leu Lys Leu Ile Ser Asn Ala Thr Ser Leu Gln Gly Glu Pro Ile Val
            100                 105                 110

Ile Ser Val Ser Gln Ser Thr Val Tyr Ile Asn Asn Lys Ala Lys Ile
        115                 120                 125

Ile Ser Ser Asp Ile Ile Ser Thr Asn Gly Ile Val His Ile Ile Asp
    130                 135                 140

Lys Leu Leu Ser Pro Lys Asn Leu Leu Ile Thr Pro Lys Asp Asn Ser
145                 150                 155                 160

Gly Arg Ile Leu Gln Asn Leu Thr Thr Leu Ala Thr Asn Asn Gly Tyr
                165                 170                 175

Ile Lys Phe Ser Asn Leu Ile Gln Asp Ser Gly Leu Leu Ser Val Ile
            180                 185                 190

Thr Asp Pro Ile His Thr Pro Val Thr Leu Phe Trp Pro Thr Asp Gln
        195                 200                 205

Ala Leu His Ala Leu Pro Ala Glu Gln Gln Asp Phe Leu Phe Asn Gln
    210                 215                 220

Asp Asn Lys Asp Lys Leu Lys Glu Tyr Leu Lys Phe His Val Ile Arg
225                 230                 235                 240

Asp Ala Lys Val Leu Ala Val Asp Leu Pro Thr Ser Thr Ala Trp Lys
                245                 250                 255

Thr Leu Gln Gly Ser Glu Leu Ser Val Lys Cys Gly Ala Gly Arg Asp
            260                 265                 270

Ile Gly Asp Leu Phe Leu Asn Gly Gln Thr Cys Arg Ile Val Gln Arg
        275                 280                 285

Glu Leu Leu Phe Asp Leu Gly Val Ala Tyr Gly Ile Asp Cys Leu Leu
    290                 295                 300

Ile Asp Pro Thr Leu Gly Gly Arg Cys Asp Thr Phe Thr Thr Phe Asp
305                 310                 315                 320

Ala Ser Gly Glu Cys Gly Ser Cys Val Asn Thr Pro Ser Cys Pro Arg
                325                 330                 335

Trp Ser Lys Pro Lys Gly Val Lys Gln Lys Cys Leu Tyr Asn Leu Pro
            340                 345                 350

Phe Lys Arg Asn Leu Glu Gly Cys Arg Glu Arg Cys Ser Leu Val Ile
        355                 360                 365

Gln Ile Pro Arg Cys Cys Lys Gly Tyr Phe Gly Arg Asp Cys Gln Ala
    370                 375                 380

Cys Pro Gly Gly Pro Val Ala Pro Cys Asn Asn Arg Gly Val Cys Leu
385                 390                 395                 400

Asp Gln Tyr Ser Ala Thr Gly Glu Cys Lys Cys Asn Thr Gly Phe Asn
                405                 410                 415

Gly Thr Ala Cys Glu Met Cys Trp Pro Gly Arg Phe Gly Pro Asp Cys
            420                 425                 430

Leu Pro Cys Gly Cys Ser Asp His Gly Gln Cys Asp Asp Gly Ile Thr
        435                 440                 445

Gly Ser Gly Gln Cys Leu Cys Glu Thr Gly Trp Thr Gly Pro Ser Cys
    450                 455                 460

Asp Thr Gln Ala Val Leu Ser Ala Val Cys Thr Pro Pro Cys Ser Ala
465                 470                 475                 480

His Ala Thr Cys Lys Glu Asn Asn Thr Cys Glu Cys Asn Leu Asp Tyr
                485                 490                 495

Glu Gly Asp Gly Ile Thr Cys Thr Val Val Asp Phe Cys Lys Gln Asp
            500                 505                 510

Asn Gly Gly Cys Ala Lys Val Ala Arg Cys Ser Gln Lys Gly Thr Lys
        515                 520                 525

Val Ser Cys Ser Cys Gln Lys Gly Tyr Lys Gly Asp Gly His Ser Cys
    530                 535                 540

Thr Glu Ile Asp Pro Cys Ala Asp Gly Leu Asn Gly Gly Cys His Glu
545                 550                 555                 560

His Ala Thr Cys Lys Met Thr Gly Pro Gly Lys His Lys Cys Glu Cys
                565                 570                 575

Lys Ser His Tyr Val Gly Asp Gly Leu Asn Cys Glu Pro Glu Gln Leu
            580                 585                 590

Pro Ile Asp Arg Cys Leu Gln Asp Asn Gly Gln Cys His Ala Asp Ala
        595                 600                 605

Lys Cys Val Asp Leu His Phe Gln Asp Thr Thr Val Gly Val Phe His
    610                 615                 620

Leu Arg Ser Pro Leu Gly Gln Tyr Lys Leu Thr Phe Asp Lys Ala Arg
625                 630                 635                 640

Glu Ala Cys Ala Asn Glu Ala Ala Thr Met Ala Thr Tyr Asn Gln Leu
                645                 650                 655

Ser Tyr Ala Gln Lys Thr Trp Tyr Ser Phe Thr Lys Glu
            660                 665

<210> 13
<211> 1624
<212> DNA
<213> Homo sapiens

<400> 13
ctcatgcctc agcctccgga gtagtatttt tagtagagat ggtgtttacc atgtgggcca 60
ggctggtctc gaactcctgg cctcaagtga tccacccgcc tcggcctccc agagtgctgg 120
gattacaggc atgagccact gcacccagcc ttgtttgtat tttgaattcc aaatggaaat 180
accttcatga tcttcccact actaaaggtt taaatctggc actgatacct ctccaagagg 240
gctatatact atgcagtgtt tcccagcatg tttcacaaga aaattctttt ttgaggatca 300
tctcacagaa cttgggatct ttgcaacatg tattgtgaaa tccaggccag aggaacccca 360
tgttccttcc acactgatat tccacaatgg aggcaagaaa ggagctagag tcacttcctc 420
ccttttgtct gaacagcctc cactctataa tcctgaccac aaagcttact tcccagagtc 480
tgggtgggcc gagaggtgtg gaagagagaa tggaggacag gagagccaaa tggcacattg 540
cagcaaaaga ctcctgcctc tggctgaaac cctctgatct tctgttacag gttaaagact 600
gggacaaata cggtttaatg ccccaggttc ttcggtacca tgtggtcgcc tgccaccagc 660
tgcttctgga aaacctgaaa ttgatctcaa atgctacttc cctccaagga gagccaatag 720
tcatctccgt ctctcagagc acggtgtata taaacaataa ggctaagatc atatccagtg 780
atatcatcag tactaatggg attgttcata tcatagacaa attgctatct cccaaaaatt 840
tgcttatcac tcccaaagac aactctggaa gaattctgca aaatcttacg actttggcaa 900
caaacaatgg ctacatcaaa tttagcaact taatacagga ctcaggtttg ctgagtgtca 960
tcaccgatcc catccacacc ccagtcactc tcttctggcc caccgaccaa gccctccatg 1020
ccctacctgc tgaacaacag gacttcctgt tcaaccaaga caacaaggac aagctgaagg 1080
agtatttgaa gtttcatgtg atacgagatg ccaaggtttt agctgtggat cttcccacat 1140
ccactgcctg gaagaccctg caaggttcag agctgagtgt gaaatgtgga gctggcaggg 1200
acatcggtga cctctttctg aatggccaaa cctgcagaat tgtgcagcgg gagctcttgt 1260
ttgacctggg tgtggcctac ggcattgact gtctgctgat tgatcccacc ctggggggcc 1320
gctgtgacac ctttactact ttcgatgcct cgggggagtg tgggagctgt gtcaatactc 1380
ccagctgccc aaggtggagt aaaccaaagg gtgtgaagca gaagtgtctc tacaacctgc 1440
ccttcaagag gaacctggaa ggctgccggg agcggtgcag cctggtgata cagatcccca 1500
gcctgccctg gaggaccaga tgccccgtgt aataaccggg gtgtctgcct tgatcagtac 1560
tcggccaccg gagagtgtaa atgcaacacc ggcttcaatg ggacggcgtg tgagatgtgc 1620
tggc                                                              1624

<210> 14
<211> 381
<212> PRT
<213> Homo sapiens

<400> 14

Met Glu Ala Arg Lys Glu Leu Glu Ser Leu Pro Pro Phe Cys Leu Asn
1               5                   10                  15

Ser Leu His Ser Ile Ile Leu Thr Thr Lys Leu Thr Ser Gln Ser Leu
            20                  25                  30

Gly Gly Pro Arg Gly Val Glu Glu Arg Met Glu Asp Arg Arg Ala Lys
        35                  40                  45

Trp His Ile Ala Ala Lys Asp Ser Cys Leu Trp Leu Lys Pro Ser Asp
    50                  55                  60

Leu Leu Leu Gln Val Lys Asp Trp Asp Lys Tyr Gly Leu Met Pro Gln
65                  70                  75                  80

Val Leu Arg Tyr His Val Val Ala Cys His Gln Leu Leu Leu Glu Asn
                85                  90                  95

Leu Lys Leu Ile Ser Asn Ala Thr Ser Leu Gln Gly Glu Pro Ile Val
            100                 105                 110

Ile Ser Val Ser Gln Ser Thr Val Tyr Ile Asn Asn Lys Ala Lys Ile
        115                 120                 125

Ile Ser Ser Asp Ile Ile Ser Thr Asn Gly Ile Val His Ile Ile Asp
    130                 135                 140

Lys Leu Leu Ser Pro Lys Asn Leu Leu Ile Thr Pro Lys Asp Asn Ser
145                 150                 155                 160

Gly Arg Ile Leu Gln Asn Leu Thr Thr Leu Ala Thr Asn Asn Gly Tyr
                165                 170                 175

Ile Lys Phe Ser Asn Leu Ile Gln Asp Ser Gly Leu Leu Ser Val Ile
            180                 185                 190

Thr Asp Pro Ile His Thr Pro Val Thr Leu Phe Trp Pro Thr Asp Gln
        195                 200                 205

Ala Leu His Ala Leu Pro Ala Glu Gln Gln Asp Phe Leu Phe Asn Gln
    210                 215                 220

Asp Asn Lys Asp Lys Leu Lys Glu Tyr Leu Lys Phe His Val Ile Arg
225                 230                 235                 240

Asp Ala Lys Val Leu Ala Val Asp Leu Pro Thr Ser Thr Ala Trp Lys
                245                 250                 255

Thr Leu Gln Gly Ser Glu Leu Ser Val Lys Cys Gly Ala Gly Arg Asp
            260                 265                 270

Ile Gly Asp Leu Phe Leu Asn Gly Gln Thr Cys Arg Ile Val Gln Arg
        275                 280                 285

Glu Leu Leu Phe Asp Leu Gly Val Ala Tyr Gly Ile Asp Cys Leu Leu
    290                 295                 300

Ile Asp Pro Thr Leu Gly Gly Arg Cys Asp Thr Phe Thr Thr Phe Asp
305                 310                 315                 320

Ala Ser Gly Glu Cys Gly Ser Cys Val Asn Thr Pro Ser Cys Pro Arg
                325                 330                 335

Trp Ser Lys Pro Lys Gly Val Lys Gln Lys Cys Leu Tyr Asn Leu Pro
            340                 345                 350

Phe Lys Arg Asn Leu Glu Gly Cys Arg Glu Arg Cys Ser Leu Val Ile
        355                 360                 365

Gln Ile Pro Ser Leu Pro Trp Arg Thr Arg Cys Pro Val
    370                 375                 380

<210> 15
<211> 2483
<212> DNA
<213> Homo sapiens

<400> 15
ctcatgcctc agcctccgga gtagtatttt tagtagagat ggtgtttacc atgtgggcca 60
ggctggtctc gaactcctgg cctcaagtga tccacccgcc tcggcctccc agagtgctgg 120
gattacaggc atgagccact gcacccagcc ttgtttgtat tttgaattcc aaatgggaat 180
tccttcatga tcttcccact actaaaggtt taaatctggc actgatacct ctccaagagg 240
gctatatact atgcagtgtt tcccagcatg tttcacaaga aaattctttt ttgaggatca 300
tctcacagaa cttgggatct ttgcaacatg tattgtgaaa tccaggccag aggaacccca 360
tgttccttcc acactgatat tccacaatgg aggcaagaaa ggagctagag tcacttcctc 420
ccttttgtct gaacagcctc cactctataa tcctgaccac aaagcttact tcccagagtc 480
tgggtgggcc gagaggtgtg gaagagagaa tggaggacag gagagccaaa tggcacattg 540
cagcaaaaga ctcctgcctc tggctgaaac cctctgatct tctgttacag gttaaagact 600
gggacaaata cggtttaatg ccccaggttc ttcggtacca tgtggtcgcc tgccaccagc 660
tgcttctgga aaacctgaaa ttgatctcaa atgctacttc cctccaagga gagccaatag 720
tcatctccgt ctctcagagc acggtgtata taaacaataa ggctaagatc atatccagtg 780
atatcatcag tactaatggg attgttcata tcatagacaa attgctatct cccaaaaatt 840
tgcttatcac tcccaaagac aactctggaa gaattctgca aaatcttacg actttggcaa 900
caaacaatgg ctacatcaaa tttagcaact taatacagga ctcaggtttg ctgagtgtca 960
tcaccgatcc catccacacc ccagtcactc tcttctggcc caccgaccaa gccctccatg 1020

ccctacctgc tgaacaacag gacttcctgt tcaaccaaga caacaaggac aagctgaagg 1080
agtatttgaa gtttcatgtg atacgagatg ccaaggtttt agctgtggat cttcccacat 1140
ccactgcctg gaagaccctg caaggttcag agctgagtgt gaaatgtgga gctggcaggg 1200
acatcggtga cctctttctg aatggccaaa cctgcagaat tgtgcagcgg gagctcttgt 1260
ttgacctggg tgtggcctac ggcattgact gtctgctgat tgatcccacc ctggggggcc 1320
gctgtgacac ctttactact ttcgatgcct cgggggagtg tgggagctgt gtcaatactc 1380
ccagctgccc aaggtggagt aaaccaaagg gtgtgaagca gaagtgtctc tacaacctgc 1440
ccttcaagag gaacctggaa ggctgccggg agcgatgcag cctggtgata cagatcccca 1500
ggtgctgcaa gggctacttc gggcgagact gtcaggcctg ccctggagga ccagatgccc 1560
cgtgtaataa ccggggtgtc tgccttgatc agtactcggc caccggagag tgtaaatgca 1620
acaccggctt caatgggacg gcgtgtgaga tgtgctggcc ggggagattt gggcctgatt 1680
gtctgccctg tggctgctca gaccacggac agtgcgatga tggcatcacg ggctccgggc 1740
agtgcctctg tgaaacgggg tggacaggcc cctcgtgtga cactcaggca gttttgtctg 1800
cagtgtgtac gcctccttgt tctgctcatg ccacctgtaa ggagaacaac acgtgtgagt 1860
gtaacctgga ttatgaaggt gacggaatca catgcacagt tgtggatttc tgcaaacagg 1920
acaacggggg ctgtgcaaag gtggccagat gctcccagaa gggcacgaag gtctcctgca 1980
gctgccagaa gggatacaaa ggggacgggc acagctgcac agagatagac ccctgtgcag 2040
acggccttaa cggagggtgt cacgagcacg ccacctgtaa gatgacaggc ccgggcaagc 2100
acaagtgtga gtgtaaaagt cactatgtcg gagatgggct gaactgtgag ccggagcagc 2160
tgcccattga ccgctgctta caggacaatg ggcagtgcca tgcagacgcc aaatgtgtcg 2220
acctccactt ccaggatacc actgttgggg tgttccatct acgctcccca ctgggccagt 2280
ataagctgac ctttgacaaa gccagagagg cctgtgccaa cgaagctgcg accatggcaa 2340
cctacaacca gctctcctat gcccagaaga cctggtattc ctttaccaag gaataaagcc 2400
tttgatgcca ggacccagac tcaaggagaa tctgaatctc tgctctcctg cttgctggtc 2460
atgtggcctt gatatcaagc cac 2483

<210> 16
<211> 669
<212> PRT
<213> Homo sapiens

<400> 16

<210> 17
<211> 3625
<212> DNA
<213> Homo sapiens

<400> 17
ctcatgcctc agcctccgga gtagtatttt tagtagagat ggtgtttacc atgtgggcca 60
ggctggtctc gaactcctgg cctcaagtga tccacccgcc tcggcctccc agagtgctgg 120
gattacaggc atgagccact gcacccagcc ttgtttgtat tttgaattcc aaatggaaat 180
accttcatga tcttcccact actaaaggtt taaatctggc actgatacct ctccaagagg 240
gctatatact atgcagtgtt tcccagcatg tttcacaaga aaattctttt ttgaggatca 300
tctcacagaa cttgggatct ttgcaacatg tattgtgaaa tccaggccag aggaacccca 360
tgttccttcc acactgatat tccacaatgg aggcaagaaa ggagctagag tcacttcctc 420
ccttttgtct gaacagcctc cactctataa tcctgaccac aaagcttact tcccagagtc 480
tgggtgggcc gagaggtgtg gaagagagaa tggaggacag gagagccaaa tggcacattg 540
cagcaaaaga ctcctgcctc tggctgaaac cctctgatct tctgttacag gttaaagact 600
gggacaaata cggtttaatg ccccaggttc ttcggtacca tgtggtcgcc tgccaccagc 660
tgcttctgga aaacctgaaa ttgatctcaa atgctacttc cctccaagga gagccaatag 720
tcatctccgt ctctcagagc acggtgtata taaataataa ggctaagatc atatccagtg 780
atatcatcag tactaatggg attgttcata tcatagacaa attgctatct cccaaaaatt 840
tgcttatcac tcccaaagac aactctggaa gaattctgca aaatcttacg actttggcaa 900
caaacaatgg ctacatcaaa tttagcaact taatacagga ctcaggtttg ctgagtgtca 960
tcaccgatcc catccacacc ccagtcactc tcttctggcc caccgaccaa gccctccatg 1020
ccctacctgc tgaacaacag gacttcctgt tcaaccaaga caacaaggac aagctgaagg 1080
agtatttgaa gtttcatgtg atacgagatg ccaaggtttt agctgtggat cttcccacat 1140
ccactgcctg gaagaccctg caaggttcag agctgagtgt gaaatgtgga gctggcaggg 1200
acatcggtga cctctttctg aatggccaaa cctgcagaat tgtgcagcgg gagctcttgt 1260
ttgacctggg tgtggcctac ggcattgact gtctgctgat tgatcccacc ctggggggcc 1320
gctgtgacac ctttactact ttcgatgcct cgggggagtg tgggagctgt gtcaatactc 1380
ccagctgccc aaggtggagt aaaccaaagg gtgtgaagca gaagtgtctc tacaacctgc 1440
ccttcaagag gaacctggaa ggctgccggg agcggtgcag cctggtgata cagatcccca 1500
ggtgctgcaa gggctacttc gggcgagact gtcagggtga gggtgcctct tcccccctcg 1560
caactctaaa agtgtctgcc ttgatcagta ctcggccacc ggagagtgta aatgcaacac 1620

cggcttcaat gggacggcgt gtgagatgtg ctggccgggg agatttgggc ctgattgtct 1680
gccctgtggc tgctcagacc acggacagtg cgatgatggc atcacgggct ccgggcagtg 1740
cctctgtgaa acggggtgga caggcccctc gtgtgacact caggcagttt tgcctgcagt 1800
gtgtacgcct ccttgttctg ctcatgccac ctgtaaggag aacaacacgt gtgagtgtaa 1860
cctggattat gaaggtgacg gaatcacatg cacagttgtg gatttctgca aacaggacaa 1920
cgggggctgt gcaaaggtgg ccagatgctc ccagaagggc acgaaggtct cctgcagctg 1980
ccagaaggga tacaaagggg acgggcacag ctgcacagag atagacccct gtgcagacgg 2040
ccttaacgga gggtgtcacg agcacgccac ctgtaagatg acaggcccgg gcaagcacaa 2100
gtgtgagtgt aaaagtcact atgtcggaga tgggctgaac tgtgagccgg agcagctgcc 2160
cattgaccgc tgcttacagg acaatgggca gtgccatgca gacgccaaat gtgtcgacct 2220
ccacttccag gataccactg ttggggtgtt ccatctacgc tccccactgg gccagtataa 2280
gctgaccttt gacaaagcca gagaggcctg tgccaacgaa gctgcgacca tggcaaccta 2340
caaccagctc tcctatgccc agaaggccaa gtaccacctg tgctcagcag gctggctgga 2400
gaccgggcgg gttgcctacc ccacagcctt cgcctcccag aactgtggct ctggtgtggt 2460
tgggatagtg gactatggac ctagacccaa caagagtgaa atgtgggatg tcttctgcta 2520
tcggatgaaa ggaagtgctg gcctattcca acagctcagc tcgaggccgt gcatttctag 2580
aacacctgac tgacctgtcc atccgcggca ccctctttgt gccacagaac agtgggctgg 2640
gggagaatga gaccttgtct gggcgggaca tcgagcacca cctcgccaat gtcagcatgt 2700
ttttctacaa tgaccttgtc aatggcacca ccctgcaaac gaggctggga agcaagctgc 2760
tcatcactgc cagccaggac ccactccaac cgacggagac caggtttgtt gatggaagag 2820
ccattctgca gtgggacatc tttgcctcca atgggatcat tcatgtcatt tccaggcctt 2880
taaaagcacc ccctgccccc gtgaccttga cccacactgg cttgggagca gggatcttct 2940
ttgccatcat cctggtgact ggggctgttg ccttggctgc ttactcctac tttcggataa 3000
accggagaac aatcggcttc cagcattttg agtcggaaga ggacattaat gttgcagctc 3060
ttggcaagca gcagcctgag aatatctcga accccttgta tgagagcaca acctcagctc 3120
ccccagaacc ttcctacgac cccttcacgg actctgaaga acggcagctt gagggcaatg 3180
accccttgag gacactgtga gggcctggac gggagatgcc agccatcact cactgccacc 3240
tgggccatca actgtgaatt ctcagcacca gttgcctttt aggaacgtaa agtcctttaa 3300
gcactcagaa gccatacctc atctctctgg ctgatctggg ggttgtttct gtgggtgaga 3360
gatgtgttgc tgtgcccacc cagtacagct tcctcctctg accctttggc tcttcttcct 3420
ttgtactctt cagctggcac ctgctccatt ctgccctaca tgatgggtaa ctgtgatctt 3480
tcttccctgt tagattgtaa gcctccgtct ttgtatccca gcccctagcc cagtgcctga 3540
cacaggaact gtgcacaata aaggtttatg gaacagaaac aaagtcaaaa aaaaaaaaaa 3600
aaaaaaaaaa aaaaaaaaaa aaaac 3625

<210> 18
<211> 545
<212> PRT
<213> Homo sapiens

<400> 18
Met Glu Ala Arg Lys Glu Leu Glu Ser Leu Pro Pro Phe Cys Leu Asn
1               5                   10                  15

Ser Leu His Ser Ile Ile Leu Thr Thr Lys Leu Thr Ser Gln Ser Leu
            20                  25                  30

Gly Gly Pro Arg Gly Val Glu Glu Arg Met Glu Asp Arg Arg Ala Lys

35                  40                  45

Trp His Ile Ala Ala Lys Asp Ser Cys Leu Trp Leu Lys Pro Ser Asp
    50                  55                  60

Leu Leu Leu Gln Val Lys Asp Trp Asp Lys Tyr Gly Leu Met Pro Gln
65                  70                  75                  80

Val Leu Arg Tyr His Val Val Ala Cys His Gln Leu Leu Leu Glu Asn
                85                  90                  95

Leu Lys Leu Ile Ser Asn Ala Thr Ser Leu Gln Gly Glu Pro Ile Val
            100                 105                 110

Ile Ser Val Ser Gln Ser Thr Val Tyr Ile Asn Asn Lys Ala Lys Ile
        115                 120                 125

Ile Ser Ser Asp Ile Ile Ser Thr Asn Gly Ile Val His Ile Ile Asp
    130                 135                 140

Lys Leu Leu Ser Pro Lys Asn Leu Leu Ile Thr Pro Lys Asp Asn Ser
145                 150                 155                 160

Gly Arg Ile Leu Gln Asn Leu Thr Thr Leu Ala Thr Asn Asn Gly Tyr
                165                 170                 175

Ile Lys Phe Ser Asn Leu Ile Gln Asp Ser Gly Leu Leu Ser Val Ile
            180                 185                 190

Thr Asp Pro Ile His Thr Pro Val Thr Leu Phe Trp Pro Thr Asp Gln
        195                 200                 205

Ala Leu His Ala Leu Pro Ala Glu Gln Gln Asp Phe Leu Phe Asn Gln
    210                 215                 220

Asp Asn Lys Asp Lys Leu Lys Glu Tyr Leu Lys Phe His Val Ile Arg
225                 230                 235                 240

Asp Ala Lys Val Leu Ala Val Asp Leu Pro Thr Ser Thr Ala Trp Lys
                245                 250                 255

Thr Leu Gln Gly Ser Glu Leu Ser Val Lys Cys Gly Ala Gly Arg Asp
            260                 265                 270

Ile Gly Asp Leu Phe Leu Asn Gly Gln Thr Cys Arg Ile Val Gln Arg
        275                 280                 285

Glu Leu Leu Phe Asp Leu Gly Val Ala Tyr Gly Ile Asp Cys Leu Leu

290                 295                 300

Ile Asp Pro Thr Leu Gly Gly Arg Cys Asp Thr Phe Thr Thr Phe Asp
305                 310                 315                 320

Ala Ser Gly Glu Cys Gly Ser Cys Val Asn Thr Pro Ser Cys Pro Arg
                325                 330                 335

Trp Ser Lys Pro Lys Gly Val Lys Gln Lys Cys Leu Tyr Asn Leu Pro
            340                 345                 350

Phe Lys Arg Asn Leu Glu Gly Cys Arg Glu Arg Cys Ser Leu Val Ile
        355                 360                 365

Gln Ile Pro Arg Cys Cys Lys Gly Tyr Phe Gly Arg Asp Cys Gln Gly
    370                 375                 380

Glu Gly Ala Ser Ser Pro Leu Ala Thr Leu Lys Val Ser Ala Leu Ile
385                 390                 395                 400

Ser Thr Arg Pro Pro Glu Ser Val Asn Ala Thr Pro Ala Ser Met Gly
                405                 410                 415

Arg Arg Val Arg Cys Ala Gly Arg Gly Asp Leu Gly Leu Ile Val Cys
            420                 425                 430

Pro Val Ala Ala Gln Thr Thr Asp Ser Ala Met Met Ala Ser Arg Ala
        435                 440                 445

Pro Gly Ser Ala Ser Val Lys Arg Gly Gly Gln Ala Pro Arg Val Thr
    450                 455                 460

Leu Arg Gln Phe Cys Leu Gln Cys Val Arg Leu Leu Val Leu Leu Met
465                 470                 475                 480

Pro Pro Val Arg Arg Thr Thr Arg Val Ser Val Thr Trp Ile Met Lys
                485                 490                 495

Val Thr Glu Ser His Ala Gln Leu Trp Ile Ser Ala Asn Arg Thr Thr
            500                 505                 510

Gly Ala Val Gln Arg Trp Pro Asp Ala Pro Arg Arg Ala Arg Arg Ser
        515                 520                 525

Pro Ala Ala Ala Arg Arg Asp Thr Lys Gly Thr Gly Thr Ala Ala Gln
    530                 535                 540

Arg

545

<210> 19
<211> 1577
<212> DNA
<213> Homo sapiens

<400> 19
ctcatgcctc agcctccgga gtagtatttt tagtagagat ggtgtttacc atgtgggcca 60
ggctggtctc gaactcctgg cctcaagtga tccacccgcc tcggcctccc agagtgctgg 120
gattacaggc atgagccact gcacccagcc ttgtttgtat tttgaattcc aaatggaaat 180
accttcatga tcttcccact actaaaggtt taaatctggc actgatacct ctccaagagg 240
gctatatact atgcagtgtt tcccagcatg tttcacaaga aaattctttt ttgaggatca 300
tctcacagaa cttgggatct ttgcaacatg tattgtgaaa tccaggccag aggaacccca 360
tgttccttcc acactgatat tccacaatgg aggcaagaaa ggagctagag tcacttcctc 420
ccttttgtct gaacagcctc cactctataa tcctgaccac aaagcttact tcccagagtc 480
tgggtgggcc gagaggtgtg gaagagagaa tggaggacag gagagccaaa tggcacattg 540
cagcaaaaga ctcctgcctc tggctgaaac cctctgatct tctgttacag gttaaagact 600
gggacaaata cggtttaatg ccccaggttc ttcggtacca tgtggtcgcc tgccaccagc 660
tgcttctgga aaacctgaaa ttgatctcaa atgctacttc cctccaagga gagccaatag 720
tcatctccgt ctctcagagc acggtgtata taaacaataa ggctaagatc atatccagtg 780
atatcatcag tactaatggg attgttcata tcatagacaa attgctatct cccaaaaatt 840
tgcttatcac tcccaaagac aactctggaa gaattctgca aaatcttacg actttggcaa 900
caaacaatgg ctacatcaaa tttagcaact taatacagga ctcaggtttg ctgagtgtca 960
tcaccgatcc catccacacc ccagtcactc tcttctggcc caccgaccaa gccctccatg 1020
ccctacctgc tgaacaacag gacttcctgt tcaaccaaga caacaaggac aagctgaagg 1080
agtatttgaa gtttcatgtg atacgagatg ccaaggtttt agctgtggat cttcccacat 1140
ccactgcctg gaagaccctg caaggttcag agctgagtgt gaaatgtgga gctggcaggg 1200
acatcggtga cctctttctg aatggccaaa cctgcagaat tgtgcagcgg gagctcttgt 1260
ttgacctggg tgtggcctac ggcattgact gtctgctgat tgatcccacc ctggggggcc 1320
gctgtgacac ctttactact ttcgatgcct cggtcagtcc taaaaacaac agtgtagtaa 1380
gagaacctta agccaaagaa tggccctcat gatccagtgt ggaccctgtt gtgaaaccat 1440
taagggcctg tcctcagcaa gactaggacc cagaagacct gagggccaaa tgatgtagtt 1500
ctttagactc agaagcaaca ggcatctact tagccccaca cagcctggaa tattcttgtt 1560
tatccaccca tctactc 1577

<210> 20
<211> 334
<212> PRT
<213> Homo sapiens

<400> 20
Met Glu Ala Arg Lys Glu Leu Glu Ser Leu Pro Pro Phe Cys Leu Asn
1               5                   10                  15

Ser Leu His Ser Ile Ile Leu Thr Thr Lys Leu Thr Ser Gln Ser Leu
            20                  25                  30

Gly Gly Pro Arg Gly Val Glu Glu Arg Met Glu Asp Arg Arg Ala Lys
        35                  40                  45

Trp His Ile Ala Ala Lys Asp Ser Cys Leu Trp Leu Lys Pro Ser Asp
    50                  55                  60

Leu Leu Leu Gln Val Lys Asp Trp Asp Lys Tyr Gly Leu Met Pro Gln
65                  70                  75                  80

Val Leu Arg Tyr His Val Val Ala Cys His Gln Leu Leu Leu Glu Asn
                85                  90                  95

Leu Lys Leu Ile Ser Asn Ala Thr Ser Leu Gln Gly Glu Pro Ile Val
            100                 105                 110

Ile Ser Val Ser Gln Ser Thr Val Tyr Ile Asn Asn Lys Ala Lys Ile
        115                 120                 125

Ile Ser Ser Asp Ile Ile Ser Thr Asn Gly Ile Val His Ile Ile Asp
    130                 135                 140

Lys Leu Leu Ser Pro Lys Asn Leu Leu Ile Thr Pro Lys Asp Asn Ser
145                 150                 155                 160

Gly Arg Ile Leu Gln Asn Leu Thr Thr Leu Ala Thr Asn Asn Gly Tyr
                165                 170                 175

Ile Lys Phe Ser Asn Leu Ile Gln Asp Ser Gly Leu Leu Ser Val Ile
            180                 185                 190

Thr Asp Pro Ile His Thr Pro Val Thr Leu Phe Trp Pro Thr Asp Gln
        195                 200                 205

Ala Leu His Ala Leu Pro Ala Glu Gln Gln Asp Phe Leu Phe Asn Gln
    210                 215                 220

Asp Asn Lys Asp Lys Leu Lys Glu Tyr Leu Lys Phe His Val Ile Arg
225                 230                 235                 240

Asp Ala Lys Val Leu Ala Val Asp Leu Pro Thr Ser Thr Ala Trp Lys
                245                 250                 255

Thr Leu Gln Gly Ser Glu Leu Ser Val Lys Cys Gly Ala Gly Arg Asp
            260                 265                 270

Ile Gly Asp Leu Phe Leu Asn Gly Gln Thr Cys Arg Ile Val Gln Arg
        275                 280                 285

Glu Leu Leu Phe Asp Leu Gly Val Ala Tyr Gly Ile Asp Cys Leu Leu
    290                 295                 300

Ile Asp Pro Thr Leu Gly Gly Arg Cys Asp Thr Phe Thr Thr Phe Asp
305                 310                 315                 320

Ala Ser Val Ser Pro Lys Asn Asn Ser Val Val Arg Glu Pro
                325                 330

<210> 21
<211> 2070
<212> DNA
<213> Homo sapiens

<400> 21
cttggctagt aattctctac tggttttatg attgctaata tattcaaaac caaaacaaac 60
tttattatgt tatatataca aaaatgtctg tatttttttc aaacctagcc ttatttagtc 120
cttttcggtt tctaggcaga aaaaattaaa aagaagagaa gcacagtctt tgggactctg 180
cacgttgcac acagctcctc cctagatgag gtagaccaca aaattctgga agcaaagtaa 240
gaatgttgtt ttcactttta tttgatttat gtttattgtg ttaaaatgag taatttgtga 300
acaatttata tttatcattt atataattac ataatttaca ttagttttaa gagtgggtta 360
tttcttcttg aaattagtta attgccatgg tctgttcatg tattgccttt tttcagtgcc 420
atattaaaga ccttttgatg cagtaagtaa tttctttatt ggcttttcca ggaaagctct 480
ctctgagttg acaacttgtt tacgagaacg actttttcgc tggcaacaaa ttgagaagat 540
ctgtggcttt cagatagccc ataactcagg actccccagc ctgacctctt ccctttattc 600
tgatcacagc tgggtggtga tgcccagagt ctccattcca ccctatccaa ttgctggagg 660
agttgatgac ttagatgaag acacaccccc aatagtgtca caatttcccg ggaccatggc 720
taaacctcct ggatcattag ccagaagcag cagcctgtgc cgttcacgcc gcagcattgt 780
gccgtcctcg cctcagcctc agcgagctca gcttgctcca cacgcccccc acccgtcaca 840
ccctcggcac cctcaccacc cgcaacacac accacactcc ttgccttccc ctgatccaga 900
tatcctctca gtgtcaagtt gccctgcgct ttatcgaaat gaagaggagg aagaggccat 960
ttacttctct gctgaaaagc aatgggaagt gccagacaca gcttcagaat gtgactcctt 1020
aaattcttcc attggaagga aacagtctcc tcctttaagc ctcgagatat accaaacatt 1080
atctccgcga aagatatcaa gagatgaggt gtccctagag gattcctccc gaggggattc 1140
gcctgtaact gtggatgtgt cttggggttc tcccgactgt gtaggtctga cagaaactaa 1200
gagtatgatc ttcagtcctg caagcaaagt gtacaatggc attttggaga aatcctgtag 1260
catgaaccag ctttccagtg gcatcccggt gcctaaacct cgccacacat catgttcctc 1320
agctggcaac gacagtaaac cagttcagga agccccaagt gttgccagaa taagcagcat 1380
cccacatgac ctttgtcata atggagagaa aagcaaaaag ccatcaaaaa tcaaaagcct 1440
ttttaagaag aaatctaagt gaactggctg acttgatgga atcatgttca agtggcatct 1500
gtaaactatt atcccccacc ctccactccc cacctttttt tggtttaatt ttaggaatgt 1560
aactccattg gggctttcca ggccggatgc catagtggaa catccagaag ggcaactgcc 1620
tactgtctgc ttatttaagt gactatatat aatcaattca tcaagccagt tattactgaa 1680
aaatcattga aatgagacag tttacagtca tttctgccta tttatttctg ctttgttctc 1740
agtgatgtat atgcaacatt ttgttgaaag ccacgatgga cttacaagct ttaatggact 1800
cgtaagccag catgggcttg caaaaatttc ttgtttacca gagcatcttc ttatctttcc 1860

acagagctat ttacatcctg gactatataa cttaaaagaa gtaaaacgta attgcactac 1920
tgttttccag actggaaaaa aaaaaaatct ctgcaagtga aactgtatag agtttataaa 1980
atgactatgg ataggggact gttttcactt ttagatcaaa atgggttttt aagtaaaacc 2040
tagggtttct aattgacttg attctggaaa 2070

<210> 22
<211> 280
<212> PRT
<213> Homo sapiens

<400> 22

<210> 23
<211> 1347
<212> DNA
<213> Homo sapiens

<400> 23
aagtcactgg gagggagcat gcagggaaga agtcaaggca gccctggaat tctactccgt 60
gctcaataaa aacaaaacgt gaagaagcaa tacatcatgc aaacgaaata atgaccggaa 120
gtgggcgcat ctagttagaa tgaagtgact ttcgtaagga gtcaatgttc gcgaactgaa 180
acatgagttc aacctccttg tgccgtctct gggtgttttg cgcgtgtgta aataccggcc 240
cgttttcccc agcatggccc gcacagcctg cagagcacct cagcgtcatc atcacatgag 300
accatggggt gaccaccgtg aagaagagac ccaatgtcaa caagatccct tgtccaacta 360
catcaagttc agggactgtg tcaagtttga tattgtgggc tacggtggct ttgggatgcc 420
cctaaccaaa ttggggcaag aggaagccct ttaccaggca ctgaagaatg tgcaccctga 480
cctccacgtc tacaagaagg agtttccaga agacttccat ctcgctaaac atgaccaagt 540
tctgccaatc atgatgtatg ccaactgtgg ttacagtatc aatgggagaa ttataatgtg 600
tttcaacaaa ggcagccatg gctttgataa tgtcctcatg gatataaaga ccatcttcag 660
agatttcggg ccagatttca agaggaatcg cctggccgag ccttttaaca gcatccacat 720
ctacccattc gtgtgtaagc tcctgggagt cacccccaaa cccacaacgg ctccctggca 780
gtcacccagg aaatgctcat gagctcttat gaccagcagc caggtgagac acaaaagcag 840
ctgccagaaa actgtcagca gagtctgctc tgtcctgaga tagaaaagaa tcaaaaagtg 900
gtctcatggt ggggaggagg gaattcaagc agaacaatcc tgtttcccag cagctttgga 960
gccccaggaa caagattcca atagctccaa acagatagca cgggaggtag ggaatccctc 1020
gacctgctgg taacatttga catagtgcct tttaggcaaa gggaagttgc tctatagaga 1080
aagtcgggct gtaatccttc cggtcctaag gaaatcactg tgtacagact gcccccaaga 1140
tgccccttcc agatacggaa atctgccctc cttcaatagc acagaaagct tttcatagtg 1200
gaggagcaaa accctgctgt tcactcgata ctgaaaaaag gagaggggag agtttgaaac 1260
gagactgcaa attttcaaga cttcaaaccc cttcaatttg ggtaatacaa aggaagaata 1320
aaatcatctc agaatttgct gttgcct                                     1347

<210> 24
<211> 182
<212> PRT
<213> Homo sapiens

<400> 24
Met Ala Arg Thr Ala Cys Arg Ala Pro Gln Arg His His His Met Arg
1               5                   10                  15

Pro Trp Gly Asp His Arg Glu Glu Glu Thr Gln Cys Gln Gln Asp Pro
            20                  25                  30

Leu Ser Asn Tyr Ile Lys Phe Arg Asp Cys Val Lys Phe Asp Ile Val
        35                  40                  45

Gly Tyr Gly Gly Phe Gly Met Pro Leu Thr Lys Leu Gly Gln Glu Glu
    50                  55                  60

Ala Leu Tyr Gln Ala Leu Lys Asn Val His Pro Asp Leu His Val Tyr
65                  70                  75                  80

Lys Lys Glu Phe Pro Glu Asp Phe His Leu Ala Lys His Asp Gln Val
                85                  90                  95

Leu Pro Ile Met Met Tyr Ala Asn Cys Gly Tyr Ser Ile Asn Gly Arg
            100                 105                 110

Ile Ile Met Cys Phe Asn Lys Gly Ser His Gly Phe Asp Asn Val Leu
        115                 120                 125

Met Asp Ile Lys Thr Ile Phe Arg Asp Phe Gly Pro Asp Phe Lys Arg
    130                 135                 140

Asn Arg Leu Ala Glu Pro Phe Asn Ser Ile His Ile Tyr Pro Phe Val
145                 150                 155                 160

Cys Lys Leu Leu Gly Val Thr Pro Lys Pro Thr Thr Ala Pro Trp Gln
                165                 170                 175

Ser Pro Arg Lys Cys Ser
            180


<210> 25
<211> 1683
<212> DNA
<213> Homo sapiens

<400> 25
tcattttgcc atctctgaag ttggaataca ccttacaatc actggaatgt cacggtctcg 60
ttggcagcat ttttctgctt agtagcccat aaaataatag cacatcttgt aactaacagt 120
gttgtagatg ctatgagatc ctggggaagc ccagaatcta actccacctt gtctgactcc 180
aaagaccaca tattttctac gtctttggac tggggtacaa atgtagacaa ctcgagcttt 240
gctgattgtg agaaaggtat gagaaatggc cctgatggaa ttttcttctt gtacttgcag 300
gggaacaaag cagcatcatc ccactattcc agggaggtgc taaatatgag ggtgaggctt 360
gtcaagcggt ccctggtgga gtcctacact cacccgaaca gcaaggagac agagcggagg 420
gagaacatcg ataccgtatt gaactggttc accaaggaag aatttgactt tgtgactctg 480
tactacagag agccagataa catgggacat cgattcaggc cagaggcaga gaacaggaag 540
ttgatgattc agcaaatcaa caggaccatc gggtatctgg tgggagccac tgagaagcac 600
agcctgcaga gcacctcagc gtcatcatca catgagacca tggggtgacc accgtgaaga 660
agagacccaa tgtcaacaag atcccttgtc caactacatc aagttcaggg actgtgtcaa 720
gtttgatatt gtgggctacg gtggctttgg gatgccccta accaaattgg ggcaagagga 780
agccctttac caggcactga agaatgtgca ccctgacctc cacgtctaca agaaggagtt 840
tccagaagac ttccatctcg ctaaacatga ccaagttctg ccaatcatga tgtatgccaa 900
ctgtggttac agtatcaatg ggagaattat aatgtgtttc aacaaaggca gccatggctt 960
tgataatgtc ctcatggata taaagaccat cttcagagat ttcgggccag atttcaagag 1020
gaatcgcctg gccgagcctt ttaacagcat ccacatctac ccattcgtgt gtaagctcct 1080
gggagtcacc cccaaaccca caacggctcc ctggcagtca cccaggaaat gctcatgagc 1140
tcttatgacc agcagccagg tgagacacaa aagcagctgc cagaaaactg tcagcagagt 1200
ctgctctgtc ctgagataga aaagaatcaa aaagtggtct catggtgggg aggagggaat 1260
tcaagcagaa caatcctgtt tcccagcagc tttggagccc caggaacaag attccaatag 1320
ctccaaacag atagcacggg aggtagggaa tccctcgacc tgctggtaac atttgacata 1380
gtgcctttta ggcaaaggga agttgctcta tagagaaagt cgggctgtaa tccttccggt 1440
cctaaggaaa tcactgtgta cagactgccc ccaagatgcc ccttccagat acggaaatct 1500
gccctccttc aatagcacag aaagcttttc atagtggagg agcaaaaccc tgctgttcac 1560
tcgatactga aaaaaggaga ggggagagtt tgaaacgaga ctgcaaattt tcaagacttc 1620
aaaccccttc aatttgggta atacaaagga agaataaaat catctcagaa tttgctgttg 1680
cct 1683

<210> 26
<211> 171
<212> PRT
<213> Homo sapiens

<400> 26
Met Arg Ser Trp Gly Ser Pro Glu Ser Asn Ser Thr Leu Ser Asp Ser
1               5                   10                  15

Lys Asp His Ile Phe Ser Thr Ser Leu Asp Trp Gly Thr Asn Val Asp
            20                  25                  30

Asn Ser Ser Phe Ala Asp Cys Glu Lys Gly Met Arg Asn Gly Pro Asp
        35                  40                  45

Gly Ile Phe Phe Leu Tyr Leu Gln Gly Asn Lys Ala Ala Ser Ser His
    50                  55                  60

Tyr Ser Arg Glu Val Leu Asn Met Arg Val Arg Leu Val Lys Arg Ser
65                  70                  75                  80

Leu Val Glu Ser Tyr Thr His Pro Asn Ser Lys Glu Thr Glu Arg Arg
                85                  90                  95

Glu Asn Ile Asp Thr Val Leu Asn Trp Phe Thr Lys Glu Glu Phe Asp
            100                 105                 110

Phe Val Thr Leu Tyr Tyr Arg Glu Pro Asp Asn Met Gly His Arg Phe
        115                 120                 125

Arg Pro Glu Ala Glu Asn Arg Lys Leu Met Ile Gln Gln Ile Asn Arg
    130                 135                 140

Thr Ile Gly Tyr Leu Val Gly Ala Thr Glu Lys His Ser Leu Gln Ser
145                 150                 155                 160

Thr Ser Ala Ser Ser Ser His Glu Thr Met Gly
                165                 170

<210> 27
<211> 2912
<212> DNA
<213> Homo sapiens

<400> 27
cagctttaac agccccggcg tctttgtcgt agaaaacaca acagtggaat tttagagggg 60
ctccgagagg caaactttta agattccagg ccctttgatg gctgatttca tcttcaagac 120
caggtacact gcagccaaag acagcgtggt tcagttcttc ttttaccagc ccatcagtca 180
tcagtggaga caaactgact tctttccctg cactgtgacg tgtggaggag gttatcagct 240
caattctgct gaatgtgtgg atatccgctt gaagagggta gttcctgacc attattgtca 300
ctactaccct gaaaatgtaa aaccaaaacc aaaactgaag gaatgcagca tggatccctg 360
cccatcaagt gatggattta aagagataat gccctatgac cacttccaac ctcttcctcg 420
ctgggaacat aatccttgga ctgcatgttc cgtgtcctgt ggaggaggga ttcagagacg 480
gagctttgtg tgtgtagagg aatccatgca tggagagata ttgcaggtgg aagaatggaa 540
gtgcatgtac gcacccaaac ccaaggttat gcaaacttgt aatctgtttg attgccccaa 600
gtggattgcc atggagtggt ctcagtgcac agtgacttgt ggccgagggt tacggtaccg 660
ggttgttctg tgtattaacc accgcggaga gcatgttggg ggctgcaatc cacaactgaa 720
gttacacatc aaagaagaat gtgtcattcc catcccgtgt tataaaccaa aagaaaaaag 780
tccagtggaa gcaaaattgc cttggctgaa acaagcacaa gaactagaag agaccagaat 840
agcaacagaa gaaccaacgt tcattccaga accctggtca gcctgcagta ccacgtgtgg 900
gccgggtgtg caggtccgtg aggtgaagtg ccgtgtgctc ctcacattca cgcagactga 960
gactgagctg cccgaggaag agtgtgaagg ccccaagctg cccaccgaac ggccctgcct 1020
cctggaagca tgtgatgaga gcccggcctc ccgagagcta gacatccctc tccctgagga 1080
cagtgagacg acttacgact gggagtacgc tgggttcacc ccttgcacag caacatgcgt 1140

gggaggccat caagaagcca tagcagtgtg cttacatatc cagacccagc agacagtcaa 1200
tgacagcttg tgtgatatgg tccaccgtcc tccagccatg agccaggcct gtaacacaga 1260
gccctgtccc cccaggtggc atgtgggctc ttgggggccc tgctcagcta cctgtggagt 1320
tggaattcag acccgagatg tgtactgcct gcacccaggg gagacccctg cccctcctga 1380
ggagtgccga gatgaaaagc cccatgcttt acaagcatgc aatcagtttg actgccctcc 1440
tggctggcac attgaagaat ggcagcagtg ttccaggact tgtggcgggg gaactcagaa 1500
cagaagagtc acctgtcggc agctgctaac ggatggcagc tttttgaatc tctcagatga 1560
attgtgccaa ggacccaagg catcgtctca caagtcctgt gccaggacag actgtcctcc 1620
acatttagct gtgggagact ggtcgaagtg ttctgtcagt tgtggtgttg gaatccagag 1680
aagaaagcag gtgtgtcaaa ggctggcagc caaaggtcgg cgcatccccc tcagtgagat 1740
gatgtgcagg gatctaccag ggttccctct tgtaagatct tgccagatgc ctgagtgcag 1800
taaaatcaaa tcagagatga agacaaaact tggtgagcag ggtccgcaga tcctcagtgt 1860
ccagagagtc tacattcaga caagggaaga gaagcgtatt aacctgacca ttggtagcag 1920
agcctatttg ctgcccaaca catccgtgat tattaagtgc ccagtgcgac gattccagaa 1980
atctctgatc cagtgggaga aggatggccg ttgcctgcag aactccaaac ggcttggcat 2040
caccaagtca ggctcactaa aaatccacgg tcttgctgcc cccgacatcg gcgtgtaccg 2100
gtgcattgca ggctctgcac aggaaacagt tgtgctcaag ctcattggta ctgacaaccg 2160
gctcatcgca cgcccagccc tcagggagcc tatgagggaa tatcctggga tggaccacag 2220
cgaagccaat agtttgggag tcacatggca caaaatgagg caaatgtgga ataacaaaaa 2280
tgacctttat ctggatgatg accacattag taaccagcct ttcttgagag ctctgttagg 2340
ccactgcagc aattctgcag gaagcaccaa ctcctgggag ttgaagaata agcagtttga 2400
agcagcagtt aaacaaggag catatagcat ggatacagcc cagtttgatg agctgataag 2460
aaacatgagt cagctcatgg aaaccggaga ggtcagcgat gatcttgcgt cccagctgat 2520
atatcagctg gtggccgaat tagccaaggc acagccaaca cacatgcagt ggcggggcat 2580
ccaggaagag acacctcctg ctgctcagct cagaggggaa acagggagtg tgtcccaaag 2640
ctcgcatgca aaaaactcag gcaagctgac attcaagccg aaaggacctg ttctcatgag 2700
gcaaagccaa cctccctcaa tttcatttaa taaaacaata aattccagga ttggaaatac 2760
agtatacatt acaaaaagga cagaggtcat caatatactg tgtgacctta ttacccccag 2820
tgaggccaca tatacatgga ccaaggatgg aaccttgtta cagccctcag taaagtaagt 2880
aaaataaaaa tgcagtattc atttttgcaa aa 2912

<210> 28
<211> 926
<212> PRT
<213> Homo sapiens

<400> 28
Met Ala Asp Phe Ile Phe Lys Thr Arg Tyr Thr Ala Ala Lys Asp Ser
1               5                   10                  15

Val Val Gln Phe Phe Phe Tyr Gln Pro Ile Ser His Gln Trp Arg Gln
            20                  25                  30

Thr Asp Phe Phe Pro Cys Thr Val Thr Cys Gly Gly Gly Tyr Gln Leu
        35                  40                  45

Asn Ser Ala Glu Cys Val Asp Ile Arg Leu Lys Arg Val Val Pro Asp
    50                  55                  60

His Tyr Cys His Tyr Tyr Pro Glu Asn Val Lys Pro Lys Pro Lys Leu
65                  70                  75                  80

Lys Glu Cys Ser Met Asp Pro Cys Pro Ser Ser Asp Gly Phe Lys Glu
                85                  90                  95

Ile Met Pro Tyr Asp His Phe Gln Pro Leu Pro Arg Trp Glu His Asn
            100                 105                 110

Pro Trp Thr Ala Cys Ser Val Ser Cys Gly Gly Gly Ile Gln Arg Arg
        115                 120                 125

Ser Phe Val Cys Val Glu Glu Ser Met His Gly Glu Ile Leu Gln Val
    130                 135                 140

Glu Glu Trp Lys Cys Met Tyr Ala Pro Lys Pro Lys Val Met Gln Thr
145                 150                 155                 160

Cys Asn Leu Phe Asp Cys Pro Lys Trp Ile Ala Met Glu Trp Ser Gln
                165                 170                 175

Cys Thr Val Thr Cys Gly Arg Gly Leu Arg Tyr Arg Val Val Leu Cys
            180                 185                 190

Ile Asn His Arg Gly Glu His Val Gly Gly Cys Asn Pro Gln Leu Lys
        195                 200                 205

Leu His Ile Lys Glu Glu Cys Val Ile Pro Ile Pro Cys Tyr Lys Pro
    210                 215                 220

Lys Glu Lys Ser Pro Val Glu Ala Lys Leu Pro Trp Leu Lys Gln Ala
225                 230                 235                 240

Gln Glu Leu Glu Glu Thr Arg Ile Ala Thr Glu Glu Pro Thr Phe Ile
                245                 250                 255

Pro Glu Pro Trp Ser Ala Cys Ser Thr Thr Cys Gly Pro Gly Val Gln
            260                 265                 270

Val Arg Glu Val Lys Cys Arg Val Leu Leu Thr Phe Thr Gln Thr Glu
        275                 280                 285

Thr Glu Leu Pro Glu Glu Glu Cys Glu Gly Pro Lys Leu Pro Thr Glu
    290                 295                 300

Arg Pro Cys Leu Leu Glu Ala Cys Asp Glu Ser Pro Ala Ser Arg Glu
305                 310                 315                 320

Leu Asp Ile Pro Leu Pro Glu Asp Ser Glu Thr Thr Tyr Asp Trp Glu
                325                 330                 335

Tyr Ala Gly Phe Thr Pro Cys Thr Ala Thr Cys Val Gly Gly His Gln
            340                 345                 350

Glu Ala Ile Ala Val Cys Leu His Ile Gln Thr Gln Gln Thr Val Asn
        355                 360                 365

Asp Ser Leu Cys Asp Met Val His Arg Pro Pro Ala Met Ser Gln Ala
    370                 375                 380

Cys Asn Thr Glu Pro Cys Pro Pro Arg Trp His Val Gly Ser Trp Gly
385                 390                 395                 400

Pro Cys Ser Ala Thr Cys Gly Val Gly Ile Gln Thr Arg Asp Val Tyr
                405                 410                 415

Cys Leu His Pro Gly Glu Thr Pro Ala Pro Pro Glu Glu Cys Arg Asp
            420                 425                 430

Glu Lys Pro His Ala Leu Gln Ala Cys Asn Gln Phe Asp Cys Pro Pro
        435                 440                 445

Gly Trp His Ile Glu Glu Trp Gln Gln Cys Ser Arg Thr Cys Gly Gly
    450                 455                 460

Gly Thr Gln Asn Arg Arg Val Thr Cys Arg Gln Leu Leu Thr Asp Gly
465                 470                 475                 480

Ser Phe Leu Asn Leu Ser Asp Glu Leu Cys Gln Gly Pro Lys Ala Ser
                485                 490                 495

Ser His Lys Ser Cys Ala Arg Thr Asp Cys Pro Pro His Leu Ala Val
            500                 505                 510

Gly Asp Trp Ser Lys Cys Ser Val Ser Cys Gly Val Gly Ile Gln Arg
        515                 520                 525

Arg Lys Gln Val Cys Gln Arg Leu Ala Ala Lys Gly Arg Arg Ile Pro
    530                 535                 540

Leu Ser Glu Met Met Cys Arg Asp Leu Pro Gly Phe Pro Leu Val Arg
545                 550                 555                 560

Ser Cys Gln Met Pro Glu Cys Ser Lys Ile Lys Ser Glu Met Lys Thr
                565                 570                 575

Lys Leu Gly Glu Gln Gly Pro Gln Ile Leu Ser Val Gln Arg Val Tyr
            580                 585                 590

Ile Gln Thr Arg Glu Glu Lys Arg Ile Asn Leu Thr Ile Gly Ser Arg
        595                 600                 605

Ala Tyr Leu Leu Pro Asn Thr Ser Val Ile Ile Lys Cys Pro Val Arg
    610                 615                 620

Arg Phe Gln Lys Ser Leu Ile Gln Trp Glu Lys Asp Gly Arg Cys Leu
625                 630                 635                 640

Gln Asn Ser Lys Arg Leu Gly Ile Thr Lys Ser Gly Ser Leu Lys Ile
                645                 650                 655

His Gly Leu Ala Ala Pro Asp Ile Gly Val Tyr Arg Cys Ile Ala Gly
            660                 665                 670

Ser Ala Gln Glu Thr Val Val Leu Lys Leu Ile Gly Thr Asp Asn Arg
        675                 680                 685

Leu Ile Ala Arg Pro Ala Leu Arg Glu Pro Met Arg Glu Tyr Pro Gly
    690                 695                 700

Met Asp His Ser Glu Ala Asn Ser Leu Gly Val Thr Trp His Lys Met
705                 710                 715                 720

Arg Gln Met Trp Asn Asn Lys Asn Asp Leu Tyr Leu Asp Asp Asp His
                725                 730                 735

Ile Ser Asn Gln Pro Phe Leu Arg Ala Leu Leu Gly His Cys Ser Asn
            740                 745                 750

Ser Ala Gly Ser Thr Asn Ser Trp Glu Leu Lys Asn Lys Gln Phe Glu
        755                 760                 765

Ala Ala Val Lys Gln Gly Ala Tyr Ser Met Asp Thr Ala Gln Phe Asp
    770                 775                 780

Glu Leu Ile Arg Asn Met Ser Gln Leu Met Glu Thr Gly Glu Val Ser
785                 790                 795                 800

Asp Asp Leu Ala Ser Gln Leu Ile Tyr Gln Leu Val Ala Glu Leu Ala
                805                 810                 815

Lys Ala Gln Pro Thr His Met Gln Trp Arg Gly Ile Gln Glu Glu Thr
            820                 825                 830

Pro Pro Ala Ala Gln Leu Arg Gly Glu Thr Gly Ser Val Ser Gln Ser
        835                 840                 845

Ser His Ala Lys Asn Ser Gly Lys Leu Thr Phe Lys Pro Lys Gly Pro
    850                 855                 860

Val Leu Met Arg Gln Ser Gln Pro Pro Ser Ile Ser Phe Asn Lys Thr
865                 870                 875                 880

Ile Asn Ser Arg Ile Gly Asn Thr Val Tyr Ile Thr Lys Arg Thr Glu
                885                 890                 895

Val Ile Asn Ile Leu Cys Asp Leu Ile Thr Pro Ser Glu Ala Thr Tyr
            900                 905                 910

Thr Trp Thr Lys Asp Gly Thr Leu Leu Gln Pro Ser Val Lys
        915                 920                 925

<210> 29
<211> 3905
<212> DNA
<213> Homo sapiens

<400> 29
ggatttgaga gcctgaactt agccatacac cagatctacc tttggaccgc aaaagggacc 60
cagtgcttca tgaagctggt tttttttgtt ttgttttgtt ttttttccgt tgttttgttt 120
cggctttacc aacctgactg ggtgtttttc aatatccacc attcagactt tcctcaacag 180
cagaggatgt ggcagtggca aagacaaggg gatgggggga gacgaaaggg aaaggggcct 240
gcatgaaaga ccatgtctgt cttcctgctg gtgccagttc cctgaacctc atcttgttgt 300
tcagcccctt actgcagcct gcccagggct ccactccatg gcttcatcct aggccagacc 360
agcacccagc ccgggggctc catccacttt ggctgcaacg ccggctaccg cctggtggga 420
cacagcatgg ccatctgtac ccggcacccc cagggctacc acctgtggag cgaagccatc 480
cctctctgtc aagctctttc ctgtgggctt cctgaggccc ccaagaatgg aatggtgttt 540
ggcaaggagt acacagtggg aaccaaggcc gtgtacagct gcagtgaagg ctaccacctc 600
caggcaggcg ctgaggccac tgcagagtgt ctggacacag gcctatggag caaccgcaat 660
gtcccaccac agtgtgtccc tgtgacttgt cctgatgtca gtagcatcag cgtggagcat 720
ggccgatgga ggcttatctt tgagacacag tatcagttcc aggcccagct gatgctcatc 780
tgtgaccctg gctactacta tactggccaa agggtcatcc gctgtcaggc caatggcaaa 840
tggagcctcg gggactctac gcccacctgc cgaatcatct cctgtggaga gctcccgatt 900
ccccccaatg gccaccgcat cggaacactg tctgtctacg gggcaacagc catcttctcc 960
tgcaattccg gatacacact ggtgggctcc agggtgcgtg agtgcatggc caatgggctc 1020
tggagtggct ctgaagtccg ctgccttgct ggacactgtg ggactcctga gcccattgtc 1080
aacggacaca tcaatgggga gaactacagc taccggggca gtgtggtgta ccaatgcaat 1140
gctggcttcc gcctgatcgg catgtctgtg cgcatctgcc agcaggatca tcactggtcg 1200
ggcaagaccc ctttctgtgt gccaattacc tgtggacacc caggcaaccc tgtcaacggc 1260
ctcactcagg gtaaccagtt taacctcaac gatgtggtca agtttgtttg caaccctggg 1320

tatatggctg agggggctgc taggtcccaa tgcctggcca gcgggcaatg gagtgacatg 1380
ctgcccacct gcagaatcat caactgtaca gatcctggac accaagaaaa tagtgttcgt 1440
caggtccacg ccagcggccc gcacaggttc agcttcggca ccactgtgtc ttaccggtgc 1500
nnaaccacgg cttctacctc ctgggcaacc ccagtgctca gctgccaggg agatggcaca 1560
tgggaccgtc cccgccccca gtgtctcttg gtgtcctgtg gccatccggg ctccccgcct 1620
cactcccaga tgtctggaga cagttatact gtgggagcag tggtgcggta cagctgcatc 1680
ggcaagcgta ctctggtggg aaacagcacc cgcatgtgtg ggctggatgg acactggact 1740
ggctccctcc ctcactgctc aggaaccagc gtgggagttt gcggtgaccc tgggatcccg 1800
gctcatggca tccgtttggg ggacagcttt gatccaggca ctgtgatgcg cttcagctgt 1860
gaagctggcc acgtgctccg gggatcgtca gagcgcacct gtcaagccaa tggctcgtgg 1920
agcggctcgc agcctgagtg tggagtgatc tcttgtggga accctgggac tccaagtaat 1980
gcccgagttg tgttcagtga tggcctggtt ttctccagct ctatcgtcta tgagtgccgg 2040
gaaggatact acgccacagg cctgctcagc cgtcactgct cggtcaatgg tacctggaca 2100
ggcagtgacc ctgagtgcct cgtcataaac tgtggtgacc ctgggattcc agccaatggc 2160
cttcggctgg gcaatgactt caggtacaac aaaactgtga catatcagtg tgtccctggc 2220
tatatgatgg agtcacatag agtatctgtg ctgagctgca ccaaggaccg gacatggaat 2280
ggaaccaagc ccgtctgcaa agctctcatg tgcaagccac ctccgctcat ccccaatggg 2340
aaggtggtgg ggtctgactt catgtggggc tcaagtgtga cttatgcctg cctggagggg 2400
taccagctct ccctgcccgc ggtgttcacc tgtgagggaa atgggtcctg gaccggagag 2460
ctgcctcagt gtttccctgt gttctgcggg gatcctggtg tcccgtcccg tgggaggaga 2520
gaggaccgag gcttctccta caggtcatct gtctccttct cctgccatcc ccctctggtg 2580
ctggtgggct ctccacgcag gttttgccag tcagatggga catggagtgg cacccagccc 2640
agctgcatag atccgaccct gaccacgtgt gcggaccctg gtgtgccaca gtttgggata 2700
cagaacaatt ctcagggcta ccaggttgga agcacagtcc tcttccgttg tcaaaaaggc 2760
tacctgcttc agggctccac caccaggacc tgcctcccaa acctgacctg gagtggaacc 2820
ccacctgact gtgtccccca ccactgcagg cagccagaga cgccaacgca tgccaacgtc 2880
ggggccctgg atttgccctc catgggctac acgctcatta ctcctgccag gagggcttct 2940
ccctcaaggg tggctccgag caccgcacct gcaaggcgga tggcagctgg acaggcaagc 3000
cgcccatctg cctggcagag gtccggccca gtgggagacc catcaacact gcccgggagc 3060
caccgctcac ccaagccttg attcctgggg atgtttttgc caagaattcc ctgtggaaag 3120
gggcctatga ataccagggg aagaagcagc cagccatgct cagagtgact ggcttccaag 3180
ttgccaacag caaggtcaat gccaccatga tcgaccacag tggcgtggag ctgcacttgg 3240
ctggaactta caagaaagaa gattttcatc tcctactcca ggtgtaccag attacagggc 3300
ctgtggagat ctttatgaat aagttcaaag atgatcactg ggctttagat ggccatgtct 3360
cgtcagagtc ctccggagcc accttcatct accaaggctc tgtcaagggc caaggctttg 3420
ggcagttcgg ctttcaaaga ctggacctca ggctgctgga gtcagacccc gagtccattg 3480
gccgccactt tgcttccaac agcagctcag tggcagccgc gatcctggtg cctttcatcg 3540
ccctcattat tgcgggcttc gtgctctatc tctacaagca caggagaaga cccaaagttc 3600
ctttcaatgg ctatgctggc cacgagaaca ccaatgttcg ggccacattt gagaacccaa 3660
tgtacgaccg caacatccag cccacagaca tcatggccag cgaggcggag ttcacagtca 3720
gcacagtgtg cacagcagta tagccacccg gcctggccgc tttttttgct aggttgaact 3780
ggtactccag cagccgccga agctggactg tactgctgcc atctcagctc actgcaacct 3840
ccctgcctga ttcccctgcc tcagcctgcc gagtgcctgc gattgcaggc gcgcaccgcc 3900
acnnt 3905

<210> 30
<211> 883
<212> PRT

<213> Homo sapiens

<400> 30
Met Ala Ile Cys Thr Arg His Pro Gln Gly Tyr His Leu Trp Ser Glu
1               5                   10                  15

Ala Ile Pro Leu Cys Gln Ala Leu Ser Cys Gly Leu Pro Glu Ala Pro
            20                  25                  30

Lys Asn Gly Met Val Phe Gly Lys Glu Tyr Thr Val Gly Thr Lys Ala
        35                  40                  45

Val Tyr Ser Cys Ser Glu Gly Tyr His Leu Gln Ala Gly Ala Glu Ala
    50                  55                  60

Thr Ala Glu Cys Leu Asp Thr Gly Leu Trp Ser Asn Arg Asn Val Pro
65                  70                  75                  80

Pro Gln Cys Val Pro Val Thr Cys Pro Asp Val Ser Ser Ile Ser Val
                85                  90                  95

Glu His Gly Arg Trp Arg Leu Ile Phe Glu Thr Gln Tyr Gln Phe Gln
            100                 105                 110

Ala Gln Leu Met Leu Ile Cys Asp Pro Gly Tyr Tyr Tyr Thr Gly Gln
        115                 120                 125

Arg Val Ile Arg Cys Gln Ala Asn Gly Lys Trp Ser Leu Gly Asp Ser
    130                 135                 140

Thr Pro Thr Cys Arg Ile Ile Ser Cys Gly Glu Leu Pro Ile Pro Pro
145                 150                 155                 160

Asn Gly His Arg Ile Gly Thr Leu Ser Val Tyr Gly Ala Thr Ala Ile
                165                 170                 175

Phe Ser Cys Asn Ser Gly Tyr Thr Leu Val Gly Ser Arg Val Arg Glu
            180                 185                 190

Cys Met Ala Asn Gly Leu Trp Ser Gly Ser Glu Val Arg Cys Leu Ala
        195                 200                 205

Gly His Cys Gly Thr Pro Glu Pro Ile Val Asn Gly His Ile Asn Gly
    210                 215                 220

Glu Asn Tyr Ser Tyr Arg Gly Ser Val Val Tyr Gln Cys Asn Ala Gly
225                 230                 235                 240

Phe Arg Leu Ile Gly Met Ser Val Arg Ile Cys Gln Gln Asp His His
                245                 250                 255

Trp Ser Gly Lys Thr Pro Phe Cys Val Pro Ile Thr Cys Gly His Pro
            260                 265                 270

Gly Asn Pro Val Asn Gly Leu Thr Gln Gly Asn Gln Phe Asn Leu Asn
        275                 280                 285

Asp Val Val Lys Phe Val Cys Asn Pro Gly Tyr Met Ala Glu Gly Ala
    290                 295                 300

Ala Arg Ser Gln Cys Leu Ala Ser Gly Gln Trp Ser Asp Met Leu Pro
305                 310                 315                 320

Thr Cys Arg Ile Ile Asn Cys Thr Asp Pro Gly His Gln Glu Asn Ser
                325                 330                 335

Val Arg Gln Val His Ala Ser Gly Pro His Arg Phe Ser Phe Gly Thr
            340                 345                 350

Thr Val Ser Tyr Arg Cys Thr Thr Ala Ser Thr Ser Trp Ala Thr Pro
        355                 360                 365

Val Leu Ser Cys Gln Gly Asp Gly Thr Trp Asp Arg Pro Arg Pro Gln
    370                 375                 380

Cys Leu Leu Val Ser Cys Gly His Pro Gly Ser Pro Pro His Ser Gln
385                 390                 395                 400

Met Ser Gly Asp Ser Tyr Thr Val Gly Ala Val Val Arg Tyr Ser Cys
                405                 410                 415

Ile Gly Lys Arg Thr Leu Val Gly Asn Ser Thr Arg Met Cys Gly Leu
            420                 425                 430

Asp Gly His Trp Thr Gly Ser Leu Pro His Cys Ser Gly Thr Ser Val
        435                 440                 445

Gly Val Cys Gly Asp Pro Gly Ile Pro Ala His Gly Ile Arg Leu Gly
    450                 455                 460

Asp Ser Phe Asp Pro Gly Thr Val Met Arg Phe Ser Cys Glu Ala Gly
465                 470                 475                 480

His Val Leu Arg Gly Ser Ser Glu Arg Thr Cys Gln Ala Asn Gly Ser
                485                 490                 495

Trp Ser Gly Ser Gln Pro Glu Cys Gly Val Ile Ser Cys Gly Asn Pro
            500                 505                 510

Gly Thr Pro Ser Asn Ala Arg Val Val Phe Ser Asp Gly Leu Val Phe
        515                 520                 525

Ser Ser Ser Ile Val Tyr Glu Cys Arg Glu Gly Tyr Tyr Ala Thr Gly
    530                 535                 540

Leu Leu Ser Arg His Cys Ser Val Asn Gly Thr Trp Thr Gly Ser Asp
545                 550                 555                 560

Pro Glu Cys Leu Val Ile Asn Cys Gly Asp Pro Gly Ile Pro Ala Asn
                565                 570                 575

Gly Leu Arg Leu Gly Asn Asp Phe Arg Tyr Asn Lys Thr Val Thr Tyr
            580                 585                 590

Gln Cys Val Pro Gly Tyr Met Met Glu Ser His Arg Val Ser Val Leu
        595                 600                 605

Ser Cys Thr Lys Asp Arg Thr Trp Asn Gly Thr Lys Pro Val Cys Lys
    610                 615                 620

Ala Leu Met Cys Lys Pro Pro Pro Leu Ile Pro Asn Gly Lys Val Val
625                 630                 635                 640

Gly Ser Asp Phe Met Trp Gly Ser Ser Val Thr Tyr Ala Cys Leu Glu
                645                 650                 655

Gly Tyr Gln Leu Ser Leu Pro Ala Val Phe Thr Cys Glu Gly Asn Gly
            660                 665                 670

Ser Trp Thr Gly Glu Leu Pro Gln Cys Phe Pro Val Phe Cys Gly Asp
        675                 680                 685

Pro Gly Val Pro Ser Arg Gly Arg Arg Glu Asp Arg Gly Phe Ser Tyr
    690                 695                 700

Arg Ser Ser Val Ser Phe Ser Cys His Pro Pro Leu Val Leu Val Gly
705                 710                 715                 720

Ser Pro Arg Arg Phe Cys Gln Ser Asp Gly Thr Trp Ser Gly Thr Gln
                725                 730                 735

Pro Ser Cys Ile Asp Pro Thr Leu Thr Thr Cys Ala Asp Pro Gly Val
            740                 745                 750

Pro Gln Phe Gly Ile Gln Asn Asn Ser Gln Gly Tyr Gln Val Gly Ser
        755                 760                 765

Thr Val Leu Phe Arg Cys Gln Lys Gly Tyr Leu Leu Gln Gly Ser Thr
    770                 775                 780

Thr Arg Thr Cys Leu Pro Asn Leu Thr Trp Ser Gly Thr Pro Pro Asp
785                 790                 795                 800

Cys Val Pro His His Cys Arg Gln Pro Glu Thr Pro Thr His Ala Asn
                805                 810                 815

Val Gly Ala Leu Asp Leu Pro Ser Met Gly Tyr Thr Leu Ile Thr Pro
            820                 825                 830

Ala Arg Arg Ala Ser Pro Ser Arg Val Ala Pro Ser Thr Ala Pro Ala
        835                 840                 845

Arg Arg Met Ala Ala Gly Gln Ala Ser Arg Pro Ser Ala Trp Gln Arg
    850                 855                 860

Ser Gly Pro Val Gly Asp Pro Ser Thr Leu Pro Gly Ser His Arg Ser
865                 870                 875                 880

Pro Lys Pro

<210> 31
<211> 3896
<212> DNA
<213> Homo sapiens

<400> 31
ggatttgaga gcctgaactt agccatacac cagatctacc tttggaccgc aaaagggacc 60
cagtgcttca tgaagctggt tttttttgtt ttgttttgtt ttttttccgt tgttttgttt 120
cggctttacc aacctgactg ggtgtttttc aatatccacc attcagactt tcctcaacag 180
cagaggatgt ggcagtggca aagacaaggg gatgggggga gacgaaaggg aaaggggcct 240
gcatgaaaga ccatgtctgt cttcctgctg gtgccagttc cctgaacctc atcttgttgt 300
tcagcccctt actgcagcct gcccagggct ccactccatg gcttcatcct aggccagacc 360
agcacccagc ccgggggctc catccacttt ggctgcaacg ccggctaccg cctggtggga 420
cacagcatgg ccatctgtac ccggcacccc cagggctacc acctgtggag cgaagccatc 480
cctctctgtc aagctctttc ctgtgggctt cctgaggccc ccaagaatgg aatggtgttt 540
ggcaaggagt acacagtggg aaccaaggcc gtgtacagct gcagtgaagg ctaccacctc 600
caggcaggcg ctgaggccac tgcagagtgt ctggacacag gcctatggag caaccgcaat 660
gtcccaccac agtgtgtccc tgtgacttgt cctgatgtca gtagcatcag cgtggagcat 720
ggccgatgga ggcttatctt tgagacacag tatcagttcc aggcccagct gatgctcatc 780
tgtgaccctg gctactacta tactggccaa agggtcatcc gctgtcaggc caatggcaaa 840

tggagcctcg gggactctac gcccacctgc cgaatcatct cctgtggaga gctcccgatt 900
ccccccaatg gccaccgcat cggaacactg tctgtctacg gggcaacagc catcttctcc 960
tgcaattccg gatacacact ggtgggctcc agggtgcgtg agtgcatggc caatgggctc 1020
tggagtggct ctgaagtccg ctgccttgct ggacactgtg ggactcctga gcccattgtc 1080
aacggacaca tcaatgggga gaactacagc taccggggca gtgtggtgta ccaatgcaat 1140
gctggcttcc gcctgatcgg catgtctgtg cgcatctgcc agcaggatca tcactggtcg 1200
ggcaagaccc ctttctgtgt gccaattacc tgtggacacc caggcaaccc tgtcaacggc 1260
ctcactcagg gtaaccagtt taacctcaac gatgtggtca agtttgtttg caaccctggg 1320
tatatggctg agggggctgc taggtcccaa tgcctggcca gcgggcaatg gagtgacatg 1380
ctgcccacct gcagaatcat caactgtaca gatcctggac accaagaaaa tagtgttcgt 1440
caggtccacg ccagcggccc gcacaggttc agcttcggca ccactgtgtc ttaccggtgc 1500
aaccacggct tctacctcct gggcacccca gtgctcagct gccagggaga tggcacatgg 1560
gaccgtcccc gcccccagtg tctcttggtg tcctgtggcc atccgggctc cccgcctcac 1620
tcccagatgt ctggagacag ttatactgtg ggagcagtgg tgcggtacag ctgcatcggc 1680
aagcgtactc tggtgggaaa cagcacccgc atgtgtgggc tggatggaca ctggactggc 1740
tccctccctc actgctcagg aaccagcgtg ggagtttgcg gtgaccctgg gatcccggct 1800
catggcatcc gtttggggga cagctttgat ccaggcactg tgatgcgctt cagctgtgaa 1860
gctggccacg tgctccgggg atcgtcagag cgcacctgtc aagccaatgg ctcgtggagc 1920
ggctcgcagc ctgagtgtgg agtgatctct tgtgggaacc ctgggactcc aagtaatgcc 1980
cgagttgtgt tcagtgatgg cctggttttc tccagctcta tcgtctatga gtgccgggaa 2040
ggatactacg ccacaggcct gctcagccgt cactgctcgg tcaatggtac ctggacaggc 2100
agtgaccctg agtgcctcgt cataaactgt ggtgaccctg ggattccagc caatggcctt 2160
cggctgggca atgacttcag gtacaacaaa actgtgacat atcagtgtgt ccctggctat 2220
atgatggagt cacatagagt atctgtgctg agctgcacca aggaccggac atggaatgga 2280
accaagcccg tctgcaaagc tctcatgtgc aagccacctc cgctcatccc caatgggaag 2340
gtggtggggt ctgacttcat gtggggctca agtgtgactt atgcctgcct ggaggggtac 2400
cagctctccc tgcccgcggt gttcacctgt gagggaaatg ggtcctggac cggagagctg 2460
cctcagtgtt tccctgtgtt ctgcggggat cctggtgtcc cgtcccgtgg gaggagagag 2520
gaccgaggct tctcctacag gtcatctgtc tccttctcct gccatccccc tctggtgctg 2580
gtgggctctc cacgcaggtt ttgccagtca gatgggacat ggagtggcac ccagcccagc 2640
tgcatagatc cgaccctgac cacgtgtgcg gaccctggtg tgccacagtt tgggatacag 2700
aacaattctc agggctacca ggttggaagc acagtcctct tccgttgtca aaaaggctac 2760
ctgcttcagg gctccaccac caggacctgc ctcccaaacc tgacctggag tggaacccca 2820
cctgactgtg tcccccacca ctgcaggcag ccagagacgc caacgcatgc caacgtcggg 2880
gccctggatt tgccctccat gggctacacg ctcattactc ctgccaggag ggcttctccc 2940
tcaagggtgg ctccgagcac cgcacctgca aggcggatgg cagctggaca ggcaagccgc 3000
ccatctgcct ggaggtccgg cccagtggga gacccatcaa cactgcccgg gagccaccgc 3060
tcacccaagc cttgattcct ggggatgttt ttgccaagaa ttccctgtgg aaaggggcct 3120
atgaatacca ggggaagaag cagccagcca tgctcagagt gactggcttc caagttgcca 3180
acagcaaggt caatgccacc atgatcgacc acagtggcgt ggagctgcac ttggctggaa 3240
cttacaagaa agaagatttt catctcctac tccaggtgta ccagattaca gggcctgtgg 3300
agatctttat gaataagttc aaagatgatc actgggcttt agatggccat gtctcgtcag 3360
agtcctccgg agccaccttc atctaccaag gctctgtcaa gggccaaggc tttgggcagt 3420
tcggctttca aagactggac ctcaggctgc tggagtcaga ccccgagtcc attggccgcc 3480
actttgcttc caacagcagc tcagtggcag ccgcgatcct ggtgcctttc atcgccctca 3540
ttattgcggg cttcgtgctc tatctctaca agcacaggag aagacccaaa gttcctttca 3600
atggctatgc tggccacgag aacaccaatg ttcgggccac atttgagaac ccaatgtacg 3660
accgcaacat ccagcccaca gacatcatgg ccagcgaggc ggagttcaca gtcagcacag 3720

tgtgcacagc agtatagcca cccggcctgg ccgctttttt tgctaggttg aactggtact 3780
ccagcagccg ccgaagctgg actgtactgc tgccatctca gctcactgca acctccctgc 3840
ctgattcccc tgcctcagcc tgccgagtgc ctgcgattgc aggcgcgcac cgccac 3896

<210> 32
<211> 882
<212> PRT
<213> Homo sapiens

<400> 32
Met Ala Ile Cys Thr Arg His Pro Gln Gly Tyr His Leu Trp Ser Glu
1               5                   10                  15

Ala Ile Pro Leu Cys Gln Ala Leu Ser Cys Gly Leu Pro Glu Ala Pro
            20                  25                  30

Lys Asn Gly Met Val Phe Gly Lys Glu Tyr Thr Val Gly Thr Lys Ala
        35                  40                  45

Val Tyr Ser Cys Ser Glu Gly Tyr His Leu Gln Ala Gly Ala Glu Ala
    50                  55                  60

Thr Ala Glu Cys Leu Asp Thr Gly Leu Trp Ser Asn Arg Asn Val Pro
65                  70                  75                  80

Pro Gln Cys Val Pro Val Thr Cys Pro Asp Val Ser Ser Ile Ser Val
                85                  90                  95

Glu His Gly Arg Trp Arg Leu Ile Phe Glu Thr Gln Tyr Gln Phe Gln
            100                 105                 110

Ala Gln Leu Met Leu Ile Cys Asp Pro Gly Tyr Tyr Tyr Thr Gly Gln
        115                 120                 125

Arg Val Ile Arg Cys Gln Ala Asn Gly Lys Trp Ser Leu Gly Asp Ser
    130                 135                 140

Thr Pro Thr Cys Arg Ile Ile Ser Cys Gly Glu Leu Pro Ile Pro Pro
145                 150                 155                 160

Asn Gly His Arg Ile Gly Thr Leu Ser Val Tyr Gly Ala Thr Ala Ile
                165                 170                 175

Phe Ser Cys Asn Ser Gly Tyr Thr Leu Val Gly Ser Arg Val Arg Glu
            180                 185                 190

Cys Met Ala Asn Gly Leu Trp Ser Gly Ser Glu Val Arg Cys Leu Ala
        195                 200                 205

Gly His Cys Gly Thr Pro Glu Pro Ile Val Asn Gly His Ile Asn Gly
    210                 215                 220

Glu Asn Tyr Ser Tyr Arg Gly Ser Val Val Tyr Gln Cys Asn Ala Gly
225                 230                 235                 240

Phe Arg Leu Ile Gly Met Ser Val Arg Ile Cys Gln Gln Asp His His
                245                 250                 255

Trp Ser Gly Lys Thr Pro Phe Cys Val Pro Ile Thr Cys Gly His Pro
            260                 265                 270

Gly Asn Pro Val Asn Gly Leu Thr Gln Gly Asn Gln Phe Asn Leu Asn
        275                 280                 285

Asp Val Val Lys Phe Val Cys Asn Pro Gly Tyr Met Ala Glu Gly Ala
    290                 295                 300

Ala Arg Ser Gln Cys Leu Ala Ser Gly Gln Trp Ser Asp Met Leu Pro
305                 310                 315                 320

Thr Cys Arg Ile Ile Asn Cys Thr Asp Pro Gly His Gln Glu Asn Ser
                325                 330                 335

Val Arg Gln Val His Ala Ser Gly Pro His Arg Phe Ser Phe Gly Thr
            340                 345                 350

Thr Val Ser Tyr Arg Cys Asn His Gly Phe Tyr Leu Leu Gly Thr Pro
        355                 360                 365

Val Leu Ser Cys Gln Gly Asp Gly Thr Trp Asp Arg Pro Arg Pro Gln
    370                 375                 380

Cys Leu Leu Val Ser Cys Gly His Pro Gly Ser Pro Pro His Ser Gln
385                 390                 395                 400

Met Ser Gly Asp Ser Tyr Thr Val Gly Ala Val Val Arg Tyr Ser Cys
                405                 410                 415

Ile Gly Lys Arg Thr Leu Val Gly Asn Ser Thr Arg Met Cys Gly Leu
            420                 425                 430

Asp Gly His Trp Thr Gly Ser Leu Pro His Cys Ser Gly Thr Ser Val
        435                 440                 445

Gly Val Cys Gly Asp Pro Gly Ile Pro Ala His Gly Ile Arg Leu Gly
    450                 455                 460

Asp Ser Phe Asp Pro Gly Thr Val Met Arg Phe Ser Cys Glu Ala Gly
465                 470                 475                 480

His Val Leu Arg Gly Ser Ser Glu Arg Thr Cys Gln Ala Asn Gly Ser
                485                 490                 495

Trp Ser Gly Ser Gln Pro Glu Cys Gly Val Ile Ser Cys Gly Asn Pro
            500                 505                 510

Gly Thr Pro Ser Asn Ala Arg Val Val Phe Ser Asp Gly Leu Val Phe
        515                 520                 525

Ser Ser Ser Ile Val Tyr Glu Cys Arg Glu Gly Tyr Tyr Ala Thr Gly
    530                 535                 540

Leu Leu Ser Arg His Cys Ser Val Asn Gly Thr Trp Thr Gly Ser Asp
545                 550                 555                 560

Pro Glu Cys Leu Val Ile Asn Cys Gly Asp Pro Gly Ile Pro Ala Asn
                565                 570                 575

Gly Leu Arg Leu Gly Asn Asp Phe Arg Tyr Asn Lys Thr Val Thr Tyr
            580                 585                 590

Gln Cys Val Pro Gly Tyr Met Met Glu Ser His Arg Val Ser Val Leu
        595                 600                 605

Ser Cys Thr Lys Asp Arg Thr Trp Asn Gly Thr Lys Pro Val Cys Lys
    610                 615                 620

Ala Leu Met Cys Lys Pro Pro Pro Leu Ile Pro Asn Gly Lys Val Val
625                 630                 635                 640

Gly Ser Asp Phe Met Trp Gly Ser Ser Val Thr Tyr Ala Cys Leu Glu
                645                 650                 655

Gly Tyr Gln Leu Ser Leu Pro Ala Val Phe Thr Cys Glu Gly Asn Gly
            660                 665                 670

Ser Trp Thr Gly Glu Leu Pro Gln Cys Phe Pro Val Phe Cys Gly Asp
        675                 680                 685

Pro Gly Val Pro Ser Arg Gly Arg Arg Glu Asp Arg Gly Phe Ser Tyr
    690                 695                 700

Arg Ser Ser Val Ser Phe Ser Cys His Pro Pro Leu Val Leu Val Gly
705                 710                 715                 720

Ser Pro Arg Arg Phe Cys Gln Ser Asp Gly Thr Trp Ser Gly Thr Gln
                725                 730                 735

Pro Ser Cys Ile Asp Pro Thr Leu Thr Thr Cys Ala Asp Pro Gly Val
            740                 745                 750

Pro Gln Phe Gly Ile Gln Asn Asn Ser Gln Gly Tyr Gln Val Gly Ser
        755                 760                 765

Thr Val Leu Phe Arg Cys Gln Lys Gly Tyr Leu Leu Gln Gly Ser Thr
    770                 775                 780

Thr Arg Thr Cys Leu Pro Asn Leu Thr Trp Ser Gly Thr Pro Pro Asp
785                 790                 795                 800

Cys Val Pro His His Cys Arg Gln Pro Glu Thr Pro Thr His Ala Asn
                805                 810                 815

Val Gly Ala Leu Asp Leu Pro Ser Met Gly Tyr Thr Leu Ile Thr Pro
            820                 825                 830

Ala Arg Arg Ala Ser Pro Ser Arg Val Ala Pro Ser Thr Ala Pro Ala
        835                 840                 845

Arg Arg Met Ala Ala Gly Gln Ala Ser Arg Pro Ser Ala Trp Arg Ser
    850                 855                 860

Gly Pro Val Gly Asp Pro Ser Thr Leu Pro Gly Ser His Arg Ser Pro
865                 870                 875                 880

Lys Pro

<210> 33
<211> 3
<212> PRT
<213> Artificial Sequence

<220>
<221> VARIANT
<222> (1)
<223> Wherein Xaa  is Ser  or Thr

<220>
<221> VARIANT
<222> (2)

<223> Wherein Xaa  is any two amino acids as set forth
      in the specification

<220>
<221> VARIANT
<222> (3)
<223> Wherein Xaa  is Asp  or Glu

<220>
<223> Description of Artificial Sequence: consensus
      sequence

<400> 33
Xaa Xaa Xaa
  1



<210> 34
<211> 7
<212> PRT
<213> Artificial Sequence

<220>
<221> VARIANT
<222> (2)
<223> Wherein Xaa is any amino acid as set forth in the
      specification

<220>
<221> VARIANT
<222> (4)
<223> Wherein Xaa is any 5 amino acids as set forth in
      the specification

<220>
<221> VARIANT
<222> (6)
<223> Wherein Xaa is any 2 amino acids as set forth in
      the specification

<220>
<223> Description of Artificial Sequence: consensus
      sequence

<400> 34
Cys Xaa Cys Xaa Gly Xaa Cys
  1               5

<210> 35
<211> 8
<212> PRT
<213> Artificial Sequence

<220>
<221> VARIANT
<222> (2)
<223> Wherein Xaa is any amino acid as set forth in the
      specification

<220>
<221> VARIANT
<222> (4)
<223> Wherein Xaa is any two amino acids as set forth in
      the specification

<220>
<221> VARIANT
<222> (5)
<223> Wherein Xaa is Gly or Pro

<220>
<221> VARIANT
<222> (6)
<223> Wherein Xaa is Phe or Tyr or Trp

<220>
<221> VARIANT
<222> (7)
<223> Wherein Xaa is any 4 or 8 amino acids as set forth
      in the specification

<220>
<223> Description of Artificial Sequence: consensus
      sequence

<400> 35
Cys Xaa Cys Xaa Xaa Xaa Xaa Cys
  1               5


<210> 36
<211> 13
<212> PRT
<213> Artificial Sequence

<220>
<221> VARIANT
<222> (2)
<223> Wherein Xaa is any 1 or 2 amino acids as set forth
      in the specification

<220>
<221> VARIANT
<222> (4)
<223> Wherein Xaa is any 5 amino acids as set forth in
      the specification

<220>
<221> VARIANT
<222> (6)
<223> Wherein Xaa is any 2 amino acids as set forth in
      the specification

<220>
<221> VARIANT
<222> (8)
<223> Wherein Xaa is any 2 amino acids as set forth in
      the specification

<220>
<221> VARIANT
<222> (10)
<223> Wherein Xaa is any 3 or 4 amino acids as set forth
      in the specification

<220>
<221> VARIANT
<222> (11)
<223> Wherein Xaa is Phe or Tyr or Trp

<220>
<221> VARIANT
<222> (12)
<223> Wherein Xaa is any 3 or 15 amino acids as set
      forth in the specification

<220>
<223> Description of Artificial Sequence: consensus
      sequence

<400> 36
Cys Xaa Cys Xaa Gly Xaa Cys Xaa Cys Xaa Xaa Xaa Cys
  1               5                  10

<210> 37
<211> 5
<212> PRT
<213> Artificial Sequence

<220>
<221> VARIANT
<222> (1)
<223> Wherein Xaa is Arg or Lys

<220>
<221> VARIANT
<222> (2)
<223> Wherein Xaa is any 3 amino acids as set forth in
      the specification

<220>
<221> VARIANT
<222> (3)
<223> Wherein Xaa is D or E

<220>
<221> VARIANT
<222> (4)
<223> Wherein Xaa is any 2  amino acids as set forth in
      the specification

<220>
<223> Description of Artificial Sequence: consensus
      sequence

<400> 37
Xaa Xaa Xaa Xaa Tyr
  1               5


<210> 38
<211> 5
<212> PRT
<213> Artificial Sequence

<220>
<221> VARIANT
<222> (1)
<223> Wherein Xaa is R or K

<220>
<221> VARIANT
<222> (2)
<223> Wherein Xaa is any 2 amino acids as set forth in
      the specification

<220>
<221> VARIANT
<222> (3)
<223> Wherein Xaa is D or E

<220>
<221> VARIANT
<222> (4)
<223> Wherein Xaa is any 3 amino acids as set forth in
      the specification

<220>
<223> Description of Artificial Sequence: Consensus
      sequence

<400> 38
Xaa Xaa Xaa Xaa Tyr
  1               5

NOV 15 and 16:

CG50377-10 is 16401346asm.0.174 , NOV 15, SEQ ID NO:29 & 30 in the application
CG50377-11 is 1640134asm0.174_1, NOV 16, SEQ ID NO:31 & 32 in the application

CG50377-10 1 [?]GATTTGAGAGCCTGAACTTAGCCATACACCAGATCTACCTTTGGACCGCAAAAGGGACC 60
CG50377-11 1 [?]GATTTGAGAGCCTGAACTTAGCCATACACCAGATCTACCTTTGGACCGCAAAAGGGACC 60

CG50377-10 61 [?]AGTGCTTCATGAAGCTGGTTTTTTTTGTTTTGTTTTGTTTTTTTTCCGTTGTTTTGTTT 120
CG50377-11 61 [?]AGTGCTTCATGAAGCTGGTTTTTTTTGTTTTGTTTTGTTTTTTTTCCGTTGTTTTGTTT 120

CG50377-10 121 [?]GGCTTTACCAACCTGACTGGGTGTTTTTCAATATCCACCATTCAGACTTTCCTCAACAG 180
CG50377-11 121 [?]GGCTTTACCAACCTGACTGGGTGTTTTTCAATATCCACCATTCAGACTTTCCTCAACAG 180

CG50377-10 181 [?]AGAGGATGTGGCAGTGGCAAAGACAAGGGGATGGGGGGAGACGAAAGGGAAAGGGGCCT 240
CG50377-11 181 [?]AGAGGATGTGGCAGTGGCAAAGACAAGGGGATGGGGGGAGACGAAAGGGAAAGGGGCCT 240

CG50377-10 241 [?]CATGAAAGACCATGTCTGTCTTCCTGCTGGTGCCAGTTCCCTGAACCTCATCTTGTTGT 300
CG50377-11 241 [?]CATGAAAGACCATGTCTGTCTTCCTGCTGGTGCCAGTTCCCTGAACCTCATCTTGTTGT 300

CG50377-10 301 TCAGCCCCTTACTGCAGCCTGCCCAGGGCTCCACTCCATGGCTTCATCCTAGGCCAGACC 360
CG50377-11 301 TCAGCCCCTTACTGCAGCCTGCCCAGGGCTCCACTCCATGGCTTCATCCTAGGCCAGACC 360

CG50377-10 361 AGCACCCAGCCCGGGGGCTCCATCCACTTTGGCTGCAACGCCGGCTACCGCCTGGTGGGA 420
CG50377-11 361 AGCACCCAGCCCGGGGGCTCCATCCACTTTGGCTGCAACGCCGGCTACCGCCTGGTGGGA 420

CG50377-10 421 [?]ACAGCATGGCCATCTGTACCCGGCACCCCCAGGGCTACCACCTGTGGAGCGAAGCCATC 480
CG50377-11 421 [?]ACAGCATGGCCATCTGTACCCGGCACCCCCAGGGCTACCACCTGTGGAGCGAAGCCATC 480

CG50377-10 481 [?]CTCTCTGTCAAGCTCTTTCCTGTGGGCTTCCTGAGGCCCCCAAGAATGGAATGGTGTTT 540
CG50377-11 481 [?]CTCTCTGTCAAGCTCTTTCCTGTGGGCTTCCTGAGGCCCCCAAGAATGGAATGGTGTTT 540

CG50377-10 541 [?]GCAAGGAGTACACAGTGGGAACCAAGGCCGTGTACAGCTGCAGTGAAGGCTACCACCTC 600
CG50377-11 541 [?]GCAAGGAGTACACAGTGGGAACCAAGGCCGTGTACAGCTGCAGTGAAGGCTACCACCTC 600

CG50377-10 601 [?]AGGCAGGCGCTGAGGCCACTGCAGAGTGTCTGGACACAGGCCTATGGAGCAACCGCAAT 660
CG50377-11 601 [?]AGGCAGGCGCTGAGGCCACTGCAGAGTGTCTGGACACAGGCCTATGGAGCAACCGCAAT 660

CG50377-10 661 [?]TCCCACCACAGTGTGTCCCTGTGACTTGTCCTGATGTCAGTAGCATCAGCGTGGAGCAT 720
CG50377-11 661 [?]TCCCACCACAGTGTGTCCCTGTGACTTGTCCTGATGTCAGTAGCATCAGCGTGGAGCAT 720

CG50377-10 721 GGCCGATGGAGGCTTATCTTTGAGACACAGTATCAGTTCCAGGCCCAGCTGATGCTCATC 780
CG50377-11 721 GGCCGATGGAGGCTTATCTTTGAGACACAGTATCAGTTCCAGGCCCAGCTGATGCTCATC 780

CG50377-10 781 TGTGACCCTGGCTACTACTATACTGGCCAAAGGGTCATCCGCTGTCAGGCCAATGGCAAA 840
CG50377-11 781 TGTGACCCTGGCTACTACTATACTGGCCAAAGGGTCATCCGCTGTCAGGCCAATGGCAAA 840

CG50377-10 841 TGGAGCCTCGGGGACTCTACGCCCACCTGCCGAATCATCTCCTGTGGAGAGCTCCCGATT 900
CG50377-11 841 TGGAGCCTCGGGGACTCTACGCCCACCTGCCGAATCATCTCCTGTGGAGAGCTCCCGATT 900

CG50377-10 901 CCCCCCAATGGCCACCGCATCGGAACACTGTCTGTCTACGGGGCAACAGCCATCTTCTCC 960
CG50377-11 901 CCCCCCAATGGCCACCGCATCGGAACACTGTCTGTCTACGGGGCAACAGCCATCTTCTCC 960

CG50377-10 961 TGCAATTCCGGATACACACTGGTGGGCTCCAGGGTGCGTGAGTGCATGGCCAATGGGCTC 1020
CG50377-11 961 TGCAATTCCGGATACACACTGGTGGGCTCCAGGGTGCGTGAGTGCATGGCCAATGGGCTC 1020

CG50377-10 1021 TGGAGTGGCTCTGAAGTCCGCTGCCTTGCTGGACACTGTGGGACTCCTGAGCCCATTGTC 1080
CG50377-11 1021 TGGAGTGGCTCTGAAGTCCGCTGCCTTGCTGGACACTGTGGGACTCCTGAGCCCATTGTC 1080

CG50377-10 1081 AACGGACACATCAATGGGGAGAACTACAGCTACCGGGGCAGTGTGGTGTACCAATGCAAT 1140
CG50377-11 1081 AACGGACACATCAATGGGGAGAACTACAGCTACCGGGGCAGTGTGGTGTACCAATGCAAT 1140

CG50377-10 1141 GCTGGCTTCCGCCTGATCGGCATGTCTGTGCGCATCTGCCAGCAGGATCATCACTGGTCG 1200
CG50377-11 1141 GCTGGCTTCCGCCTGATCGGCATGTCTGTGCGCATCTGCCAGCAGGATCATCACTGGTCG 1200

CG50377-10 1201 GGCAAGACCCCTTTCTGTGTGCCAATTACCTGTGGACACCCAGGCAACCCTGTCAACGGC 1260
CG50377-11 1201 GGCAAGACCCCTTTCTGTGTGCCAATTACCTGTGGACACCCAGGCAACCCTGTCAACGGC 1260

CG50377-10 1261 CTCACTCAGGGTAACCAGTTTAACCTCAACGATGTGGTCAAGTTTGTTTGCAACCCTGGG 1320
CG50377-11 1261 CTCACTCAGGGTAACCAGTTTAACCTCAACGATGTGGTCAAGTTTGTTTGCAACCCTGGG 1320

CG50377-10 1321 TATATGGCTGAGGGGGCTGCTAGGTCCCAATGCCTGGCCAGCGGGCAATGGAGTGACATG 1380
CG50377-11 1321 TATATGGCTGAGGGGGCTGCTAGGTCCCAATGCCTGGCCAGCGGGCAATGGAGTGACATG 1380

CG50377-10 1381 CTGCCCACCTGCAGAATCATCAACTGTACAGATCCTGGACACCAAGAAAATAGTGTTCGT 1440
CG50377-11 1381 CTGCCCACCTGCAGAATCATCAACTGTACAGATCCTGGACACCAAGAAAATAGTGTTCGT 1440

CG50377-10 1441 CAGGTCCACGCCAGCGGCCCGCACAGGTTCAGCTTCGGCACCACTGTGTCTTACCGGTGC 1500
CG50377-11 1441 CAGGTCCACGCCAGCGGCCCGCACAGGTTCAGCTTCGGCACCACTGTGTCTTACCGGTGC 1500

CG50377-10 1501 NNAACCACGGCTTCTACCTCCTGGGCAACCCCAGTGCTCAGCTGCCAGGGAGATGGCACA 1560
CG50377-11 1501 A--ACCACGGCTTCTACCTCCTGGGCA-CCCCAGTGCTCAGCTGCCAGGGAGATGGCACA 1557

CG50377-10 1561 TGGGACCGTCCCCGCCCCCAGTGTCTCTTGGTGTCCTGTGGCCATCCGGGCTCCCCGCCT 1620
CG50377-11 1558 TGGGACCGTCCCCGCCCCCAGTGTCTCTTGGTGTCCTGTGGCCATCCGGGCTCCCCGCCT 1617

CG50377-10 1621 CACTCCCAGATGTCTGGAGACAGTTATACTGTGGGAGCAGTGGTGCGGTACAGCTGCATC 1680
CG50377-11 1618 CACTCCCAGATGTCTGGAGACAGTTATACTGTGGGAGCAGTGGTGCGGTACAGCTGCATC 1677

CG50377-10 1681 GGCAAGCGTACTCTGGTGGGAAACAGCACCCGCATGTGTGGGCTGGATGGACACTGGACT 1740
CG50377-11 1678 GGCAAGCGTACTCTGGTGGGAAACAGCACCCGCATGTGTGGGCTGGATGGACACTGGACT 1737

CG50377-10 1741 GGCTCCCTCCCTCACTGCTCAGGAACCAGCGTGGGAGTTTGCGGTGACCCTGGGATCCCG 1800
CG50377-11 1738 GGCTCCCTCCCTCACTGCTCAGGAACCAGCGTGGGAGTTTGCGGTGACCCTGGGATCCCG 1797

CG50377-10 1801 GCTCATGGCATCCGTTTGGGGGACAGCTTTGATCCAGGCACTGTGATGCGCTTCAGCTGT 1860
CG50377-11 1798 GCTCATGGCATCCGTTTGGGGGACAGCTTTGATCCAGGCACTGTGATGCGCTTCAGCTGT 1857

CG50377-10 1861 GAAGCTGGCCACGTGCTCCGGGGATCGTCAGAGCGCACCTGTCAAGCCAATGGCTCGTGG 1920
CG50377-11 1858 GAAGCTGGCCACGTGCTCCGGGGATCGTCAGAGCGCACCTGTCAAGCCAATGGCTCGTGG 1917

CG50377-10 1921 AGCGGCTCGCAGCCTGAGTGTGGAGTGATCTCTTGTGGGAACCCTGGGACTCCAAGTAAT 1980
CG50377-11 1918 AGCGGCTCGCAGCCTGAGTGTGGAGTGATCTCTTGTGGGAACCCTGGGACTCCAAGTAAT 1977

CG50377-10 1981 GCCCGAGTTGTGTTCAGTGATGGCCTGGTTTTCTCCAGCTCTATCGTCTATGAGTGCCGG 2040
CG50377-11 1978 GCCCGAGTTGTGTTCAGTGATGGCCTGGTTTTCTCCAGCTCTATCGTCTATGAGTGCCGG 2037

CG50377-10 2041 GAAGGATACTACGCCACAGGCCTGCTCAGCCGTCACTGCTCGGTCAATGGTACCTGGACA 2100
CG50377-11 2038 GAAGGATACTACGCCACAGGCCTGCTCAGCCGTCACTGCTCGGTCAATGGTACCTGGACA 2097

CG50377-10 2101 GGCAGTGACCCTGAGTGCCTCGTCATAAACTGTGGTGACCCTGGGATTCCAGCCAATGGC 2160
CG50377-11 2098 GGCAGTGACCCTGAGTGCCTCGTCATAAACTGTGGTGACCCTGGGATTCCAGCCAATGGC 2157

CG50377-10 2161 CTTCGGCTGGGCAATGACTTCAGGTACAACAAAACTGTGACATATCAGTGTGTCCCTGGC 2220
CG50377-11 2158 CTTCGGCTGGGCAATGACTTCAGGTACAACAAAACTGTGACATATCAGTGTGTCCCTGGC 2217

CG50377-10 2221 TATATGATGGAGTCACATAGAGTATCTGTGCTGAGCTGCACCAAGGACCGGACATGGAAT 2280
CG50377-11 2218 TATATGATGGAGTCACATAGAGTATCTGTGCTGAGCTGCACCAAGGACCGGACATGGAAT 2277

CG50377-10 2281 GGAACCAAGCCCGTCTGCAAAGCTCTCATGTGCAAGCCACCTCCGCTCATCCCCAATGGG 2340
CG50377-11 2278 GGAACCAAGCCCGTCTGCAAAGCTCTCATGTGCAAGCCACCTCCGCTCATCCCCAATGGG 2337

CG50377-10 2341 AAGGTGGTGGGGTCTGACTTCATGTGGGGCTCAAGTGTGACTTATGCCTGCCTGGAGGGG 2400
CG50377-11 2338 AAGGTGGTGGGGTCTGACTTCATGTGGGGCTCAAGTGTGACTTATGCCTGCCTGGAGGGG 2397

CG50377-10 2401 TACCAGCTCTCCCTGCCCGCGGTGTTCACCTGTGAGGGAAATGGGTCCTGGACCGGAGAG 2460
CG50377-11 2398 TACCAGCTCTCCCTGCCCGCGGTGTTCACCTGTGAGGGAAATGGGTCCTGGACCGGAGAG 2457

CG50377-10 2461 CTGCCTCAGTGTTTCCCTGTGTTCTGCGGGGATCCTGGTGTCCCGTCCCGTGGGAGGAGA 2520
CG50377-11 2458 CTGCCTCAGTGTTTCCCTGTGTTCTGCGGGGATCCTGGTGTCCCGTCCCGTGGGAGGAGA 2517

CG50377-10 2521 GAGGACCGAGGCTTCTCCTACAGGTCATCTGTCTCCTTCTCCTGCCATCCCCCTCTGGTG 2580
CG50377-11 2518 GAGGACCGAGGCTTCTCCTACAGGTCATCTGTCTCCTTCTCCTGCCATCCCCCTCTGGTG 2577

CG50377-10 2581 CTGGTGGGCTCTCCACGCAGGTTTTGCCAGTCAGATGGGACATGGAGTGGCACCCAGCCC 2640
CG50377-11 2578 CTGGTGGGCTCTCCACGCAGGTTTTGCCAGTCAGATGGGACATGGAGTGGCACCCAGCCC 2637

CG50377-10 2641 AGCTGCATAGATCCGACCCTGACCACGTGTGCGGACCCTGGTGTGCCACAGTTTGGGATA 2700
CG50377-11 2638 AGCTGCATAGATCCGACCCTGACCACGTGTGCGGACCCTGGTGTGCCACAGTTTGGGATA 2697

CG50377-10 2701 CAGAACAATTCTCAGGGCTACCAGGTTGGAAGCACAGTCCTCTTCCGTTGTCAAAAAGGC 2760
CG50377-11 2698 CAGAACAATTCTCAGGGCTACCAGGTTGGAAGCACAGTCCTCTTCCGTTGTCAAAAAGGC 2757

CG50377-10 2761 TACCTGCTTCAGGGCTCCACCACCAGGACCTGCCTCCCAAACCTGACCTGGAGTGGAACC 2820
CG50377-11 2758 TACCTGCTTCAGGGCTCCACCACCAGGACCTGCCTCCCAAACCTGACCTGGAGTGGAACC 2817

CG50377-10 2821 CCACCTGACTGTGTCCCCCACCACTGCAGGCAGCCAGAGACGCCAACGCATGCCAACGTC 2880
CG50377-11 2818 CCACCTGACTGTGTCCCCCACCACTGCAGGCAGCCAGAGACGCCAACGCATGCCAACGTC 2877

CG50377-10 2881 GGGGCCCTGGATTTGCCCTCCATGGGCTACACGCTCATTACTCCTGCCAGGAGGGCTTCT 2940
CG50377-11 2878 GGGGCCCTGGATTTGCCCTCCATGGGCTACACGCTCATTACTCCTGCCAGGAGGGCTTCT 2937

CG50377-10 2941 CCCTCAAGGGTGGCTCCGAGCACCGCACCTGCAAGGCGGATGGCAGCTGGACAGGCAAGC 3000
CG50377-11 2938 CCCTCAAGGGTGGCTCCGAGCACCGCACCTGCAAGGCGGATGGCAGCTGGACAGGCAAGC 2997

CG50377-10 3001 CGCCCATCTGCCTGGCAGAGGTCCGGCCCAGTGGGAGACCCATCAACACTGCCCGGGAGC 3060
CG50377-11 2998 CGCCCATCTGCCTGG---AGGTCCGGCCCAGTGGGAGACCCATCAACACTGCCCGGGAGC 3054

CG50377-10 3061 CACCGCTCACCCAAGCCTTGATTCCTGGGGATGTTTTTGCCAAGAATTCCCTGTGGAAAG 3120
CG50377-11 3055 CACCGCTCACCCAAGCCTTGATTCCTGGGGATGTTTTTGCCAAGAATTCCCTGTGGAAAG 3114

CG50377-10 3121 GGGCCTATGAATACCAGGGGAAGAAGCAGCCAGCCATGCTCAGAGTGACTGGCTTCCAAG 3180
CG50377-11 3115 GGGCCTATGAATACCAGGGGAAGAAGCAGCCAGCCATGCTCAGAGTGACTGGCTTCCAAG 3174

CG50377-10 3181 TTGCCAACAGCAAGGTCAATGCCACCATGATCGACCACAGTGGCGTGGAGCTGCACTTGG 3240
CG50377-11 3175 TTGCCAACAGCAAGGTCAATGCCACCATGATCGACCACAGTGGCGTGGAGCTGCACTTGG 3234

CG50377-10 3241 CTGGAACTTACAAGAAAGAAGATTTTCATCTCCTACTCCAGGTGTACCAGATTACAGGGC 3300
CG50377-11 3235 CTGGAACTTACAAGAAAGAAGATTTTCATCTCCTACTCCAGGTGTACCAGATTACAGGGC 3294

CG50377-10 3301 CTGTGGAGATCTTTATGAATAAGTTCAAAGATGATCACTGGGCTTTAGATGGCCATGTCT 3360
CG50377-11 3295 CTGTGGAGATCTTTATGAATAAGTTCAAAGATGATCACTGGGCTTTAGATGGCCATGTCT 3354

CG50377-10 3361 CGTCAGAGTCCTCCGGAGCCACCTTCATCTACCAAGGCTCTGTCAAGGGCCAAGGCTTTG 3420
CG50377-11 3355 CGTCAGAGTCCTCCGGAGCCACCTTCATCTACCAAGGCTCTGTCAAGGGCCAAGGCTTTG 3414

CG50377-10 3421 GGCAGTTCGGCTTTCAAAGACTGGACCTCAGGCTGCTGGAGTCAGACCCCGAGTCCATTG 3480
CG50377-11 3415 GGCAGTTCGGCTTTCAAAGACTGGACCTCAGGCTGCTGGAGTCAGACCCCGAGTCCATTG 3474

CG50377-10 3481 GCCGCCACTTTGCTTCCAACAGCAGCTCAGTGGCAGCCGCGATCCTGGTGCCTTTCATCG 3540
CG50377-11 3475 GCCGCCACTTTGCTTCCAACAGCAGCTCAGTGGCAGCCGCGATCCTGGTGCCTTTCATCG 3534

CG50377-10 3541 CCCTCATTATTGCGGGCTTCGTGCTCTATCTCTACAAGCACAGGAGAAGACCCAAAGTTC 3600
CG50377-11 3535 CCCTCATTATTGCGGGCTTCGTGCTCTATCTCTACAAGCACAGGAGAAGACCCAAAGTTC 3594

CG50377-10 3601 CTTTCAATGGCTATGCTGGCCACGAGAACACCAATGTTCGGGCCACATTTGAGAACCCAA 3660
CG50377-11 3595 CTTTCAATGGCTATGCTGGCCACGAGAACACCAATGTTCGGGCCACATTTGAGAACCCAA 3654

CG50377-10 3661 TGTACGACCGCAACATCCAGCCCACAGACATCATGGCCAGCGAGGCGGAGTTCACAGTCA 3720
CG50377-11 3655 TGTACGACCGCAACATCCAGCCCACAGACATCATGGCCAGCGAGGCGGAGTTCACAGTCA 3714

CG50377-10 3721 GCACAGTGTGCACAGCAGTATAGCCACCCGGCCTGGCCGCTTTTTTTGCTAGGTTGAACT 3780
CG50377-11 3715 GCACAGTGTGCACAGCAGTATAGCCACCCGGCCTGGCCGCTTTTTTTGCTAGGTTGAACT 3774

CG50377-10 3781 GGTACTCCAGCAGCCGCCGAAGCTGGACTGTACTGCTGCCATCTCAGCTCACTGCAACCT 3840
CG50377-11 3775 GGTACTCCAGCAGCCGCCGAAGCTGGACTGTACTGCTGCCATCTCAGCTCACTGCAACCT 3834

CG50377-10 3841 CCCTGCCTGATTCCCCTGCCTCAGCCTGCCGAGTGCCTGCGATTGCAGGCGCGCACCGCC 3900
CG50377-11 3835 CCCTGCCTGATTCCCCTGCCTCAGCCTGCCGAGTGCCTGCGATTGCAGGCGCGCACCGCC 3894

CG50377-10 3901 ACNNT 3905
CG50377-11 3895 AC--- 3896

CG50377-10 1 MAICTRHPQGYHLWSEAIPLCQALSCGLPEAPKNGMVFGKEYTVGTKAVYSCSEGYHLQA 60
CG50377-11 1 MAICTRHPQGYHLWSEAIPLCQALSCGLPEAPKNGMVFGKEYTVGTKAVYSCSEGYHLQA 60

CG50377-10 61 GAEATAECLDTGLWSNRNVPPQCVPVTCPDVSSISVEHGRWRLIFETQYQFQAQLMLICD 120
CG50377-11 61 GAEATAECLDTGLWSNRNVPPQCVPVTCPDVSSISVEHGRWRLIFETQYQFQAQLMLICD 120

CG50377-10 121 PGYYYTGQRVIRCQANGKWSLGDSTPTCRIISCGELPIPPNGHRIGTLSVYGATAIFSCN 180
CG50377-11 121 PGYYYTGQRVIRCQANGKWSLGDSTPTCRIISCGELPIPPNGHRIGTLSVYGATAIFSCN 180

CG50377-10 181 SGYTLVGSRVRECMANGLWSGSEVRCLAGHCGTPEPIVNGHINGENYSYRGSVVYQCNAG 240
CG50377-11 181 SGYTLVGSRVRECMANGLWSGSEVRCLAGHCGTPEPIVNGHINGENYSYRGSVVYQCNAG 240

CG50377-10 241 FRLIGMSVRICQQDHHWSGKTPFCVPITCGHPGNPVNGLTQGNQFNLNDVVKFVCNPGYM 300
CG50377-11 241 FRLIGMSVRICQQDHHWSGKTPFCVPITCGHPGNPVNGLTQGNQFNLNDVVKFVCNPGYM 300

CG50377-10 301 AEGAARSQCLASGQWSDMLPTCRIINCTDPGHQENSVRQVHASGPHRFSFGTTVSYRCXT 360
CG50377-11 301 AEGAARSQCLASGQWSDMLPTCRIINCTDPGHQENSVRQVHASGPHRFSFGTTVSYRC-N 359

CG50377-10 361 TASTSWATPVLSCQGDGTWDRPRPQCLLVSCGHPGSPPHSQMSGDSYTVGAVVRYSCIGK 420
CG50377-11 360 HGFYLLGTPVLSCQGDGTWDRPRPQCLLVSCGHPGSPPHSQMSGDSYTVGAVVRYSCIGK 419

CG50377-10 421 RTLVGNSTRMCGLDGHWTGSLPHCSGTSVGVCGDPGIPAHGIRLGDSFDPGTVMRFSCEA 480
CG50377-11 420 RTLVGNSTRMCGLDGHWTGSLPHCSGTSVGVCGDPGIPAHGIRLGDSFDPGTVMRFSCEA 479

CG50377-10 481 GHVLRGSSERTCQANGSWSGSQPECGVISCGNPGTPSNARVVFSDGLVFSSSIVYECREG 540
CG50377-11 480 GHVLRGSSERTCQANGSWSGSQPECGVISCGNPGTPSNARVVFSDGLVFSSSIVYECREG 539

CG50377-10 541 YYATGLLSRHCSVNGTWTGSDPECLVINCGDPGIPANGLRLGNDFRYNKTVTYQCVPGYM 600
CG50377-11 540 YYATGLLSRHCSVNGTWTGSDPECLVINCGDPGIPANGLRLGNDFRYNKTVTYQCVPGYM 599

CG50377-10 601 MESHRVSVLSCTKDRTWNGTKPVCKALMCKPPPLIPNGKVVGSDFMWGSSVTYACLEGYQ 660
CG50377-11 600 MESHRVSVLSCTKDRTWNGTKPVCKALMCKPPPLIPNGKVVGSDFMWGSSVTYACLEGYQ 659

CG50377-10 661 LSLPAVFTCEGNGSWTGELPQCFPVFCGDPGVPSRGRREDRGFSYRSSVSFSCHPPLVLV 720
CG50377-11 660 LSLPAVFTCEGNGSWTGELPQCFPVFCGDPGVPSRGRREDRGFSYRSSVSFSCHPPLVLV 719

CG50377-10 721 GSPRRFCQSDGTWSGTQPSCIDPTLTTCADPGVPQFGIQNNSQGYQVGSTVLFRCQKGYL 780
CG50377-11 720 GSPRRFCQSDGTWSGTQPSCIDPTLTTCADPGVPQFGIQNNSQGYQVGSTVLFRCQKGYL 779

CG50377-10 781 LQGSTTRTCLPNLTWSGTPPDCVPHHCRQPETPTHANVGALDLPSMGYTLITPARRASPS 840
CG50377-11 780 LQGSTTRTCLPNLTWSGTPPDCVPHHCRQPETPTHANVGALDLPSMGYTLITPARRASPS 839

CG50377-10 841 RVAPSTAPARRMAAGQASRPSAWQRSGPVGDPSTLPGSHRSPKP 884
CG50377-11 840 RVAPSTAPARRMAAGQASRPSAWR-SGPVGDPSTLPGSHRSPKP 882

## Claims

1. An isolated polypeptide comprising an amino acid sequence selected from the group consisting of:
a) a mature form of the amino acid sequence given by SEQ ID NO: 30 or 32;
b) a variant of a mature form of the amino acid sequence given by SEQ ID NO: 30 or 32, wherein any amino acid in the mature form is changed to a different amino acid, provided that no more than 15% of the amino acid residues in the sequence of the mature form are so changed;
c) the amino acid sequence given by SEQ ID NO: 30 or 32;
d) a variant of the amino acid sequence given by SEQ ID NO: 30 or 32, wherein any amino acid specified in the chosen sequence is changed to a different amino acid, provided that no more than 15% of the amino acid residues in the sequence are so changed; and
e) a fragment of any of a) through d).

2. The polypeptide of Claim 1 that is a naturally occurring allelic variant of the sequence given by SEQ ID NO: 30 or 32.

3. The polypeptide of Claim 2, wherein the variant is the translation of a single nucleotide polymorphism.

4. The polypeptide of Claim 1 that is a variant polypeptide described therein, wherein any amino acid specified in the chosen sequence is changed to provide a conservative substitution.

5. An isolated nucleic acid molecule comprising a nucleic acid sequence encoding a polypeptide comprising an amino acid sequence selected from the group consisting of:
a) a mature form of the amino acid sequence given SEQ ID NO: 30 or 32;
b) a variant of a mature form of the amino acid sequence given by SEQ ID NO: 30 or 32, wherein any amino acid in the mature form of the chosen sequence is changed to a different amino acid, provided that no more than 15% of the amino acid residues in the sequence of the mature form are so changed;
c) the amino acid sequence given by SEQ ID NO: 30 or 32;
d) a variant of the amino acid sequence given by SEQ ID NO: 30 or 32, in which any amino acid specified in the chosen sequence is changed to a different amino acid, provided that no more than 15% of the amino acid residues in the sequence are so changed;
e) a nucleic acid fragment encoding at least a portion of a polypeptide comprising the amino acid sequence given by SEQ ID NO: 30 or 32 or any variant of said polypeptide wherein any amino acid of the chosen sequence is changed to a different amino acid, provided that no more than 10% of the amino acid residues in the sequence are so changed; and
f) the complement of any of said nucleic acid molecules.

6. The nucleic acid molecules of Claim 5, wherein the nucleic acid molecule comprises the nucleotide sequence of a naturally occurring allelic nucleic acid variant.

7. The nucleic acid molecules of Claim 5 that encodes a variant polypeptide, wherein the variant polypeptide has the polypeptide sequence of a naturally occurring polypeptide variant.

8. The nucleic acid molecules of Claim 5, wherein the nucleic acid molecule comprises a single nucleotide polymorphism encoding said variant polypeptide.

9. The nucleic acid molecules of Claim 5, wherein said nucleic acid molecule comprises a nucleotide sequence selected from the group consisting of
a) the nucleotide sequence given by SEQ ID NO: 29 or 31;
b) a nucleotide sequence wherein one or more nucleotides in the nucleotide sequence given by SEQ ID NO: 29 or 31 is changed from that given by the chosen sequence to a different nucleotide provided that no more than 15% of the nucleotides are so changed;
c) a nucleic acid fragment of the sequence given by SEQ ID NO: 29 or 31; and
d) a nucleic acid fragment wherein one or more nucleotides in the nucleotide sequence given by SEQ ID NO: 29 or 31 is changed from that given by the chosen sequence to a different nucleotide provided that no more than 15% of the nucleotides are so changed.

10. The nucleic acid molecules of Claim 5, wherein said nucleic acid molecule hybridises under stringent conditions to the nucleotides sequence given by SEQ ID NO: 29 or 31, or a complement of said nucleotide sequence.

11. The nucleic acid molecule of Claim 5, wherein the nucleic acid molecule comprises a nucleotide sequence in which any nucleotide specified in the coding sequence of the chosen nucleotide sequence is changed from that given by the chosen sequence to a different nucleotide provided that no more than 15% of the nucleotides in the chosen coding sequence are so changed, an isolated second polynucleotide that is a complement of the first polynucleotide, or a fragment of any of them.

12. A vector comprising the nucleic acid molecule of Claim 11.

13. The vector of Claim 12, further comprising a promoter operably linked to said nucleic acid molecule.

14. A cell comprising the vector of Claim 12.

15. An antibody that binds immunospecifically to the polypeptide according to any of Claims 1-4.

16. The antibody of Claim 15, wherein said antibody is a monoclonal antibody.

17. The antibody of Claim 15, wherein the antibody is a humanized antibody.

18. An *in vitro* method for determining the presence or amount of the polypeptide according to any of Claims 1-4 in a sample, the method comprising:
a) contacting said sample with an antibody that binds immunospecifically to the polypeptide; and
b) determining the presence or amount of antibody bound to said polypeptide, thereby determining the presence or amount of polypeptide in said sample.

19. An *in vitro* method for determining the presence or amount of the nucleic acid molecule according to any of Claims 5-11 in a sample, the method comprising:
a) contacting said sample with a probe that binds to said nucleic acid molecule; and
b) determining the presence or amount of said probe bound to said nucleic acid molecule,
thereby determining the presence or amount of the nucleic acid molecule in said sample.

20. An *in vitro* method of identifying an agent that binds to the polypeptide according to any of Claims 1-4, the method comprising:
a) contacting said polypeptide with said agent; and
b) determining whether said agent binds to said polypeptide.

21. An *in vitro* method for identifying a potential therapeutic agent suitable for use in treatment of a pathology, wherein the pathology is related to aberrant expression or aberrant physiological interactions of the polypeptide according to any of Claims 1-4, the method comprising:
a) contacting a cell expressing the polypeptide according to any of Claims 1-4 and having a property or function ascribable to the polypeptide with a composition comprising a candidate substance; and
b) determining whether the substance alters the property or function ascribable to the polypeptide;
whereby, if any alteration observed in the presence of the substance is not observed when the cell is contacted with a composition devoid of the substance, the substance is identified as a potential therapeutic agent.

22. An *in vitro* method for modulating the activity of the polypeptide according to any of Claims 1-4, the method comprising contacting a cell sample expressing the polypeptide of said claim with a compound that binds to said polypeptide in an amount sufficient to modulate the activity of the polypeptide.

23. A pharmaceutical composition comprising the polypeptide according to any of Claims 1-4, or the nucleic acid molecule according to any of Claims 5-11, or the antibody according to any of Claims 15-17; and a pharmaceutically acceptable carrier.

24. A kit comprising in one or more containers, and the pharmaceutical composition of Claim 23.

25. The use of a therapeutic for the manufacture of a medicament for treating a syndrome associated with a human disease selected from a pathology associated with the polypeptide according to any of Claims 1-4, wherein said therapeutic is the polypeptide, a nucleic acid according to any of Claims 5-11 or an antibody according to any of Claims 15-17.

26. The use according to Claim 25, for treating or preventing a pathology associated with the polypeptide according to any of Claims 1-4.

27. The use according to Claim 25 or Claim 26, wherein said subject is a human.

28. A method for screening for a modulator of activity or of latency or predisposition to a pathology associated with the polypeptide according to any of Claims 1-4, said method comprising:
a) administering a test compound to a test animal at increased risk for a pathology associated with the polypeptide according to any of Claims 1-4, wherein said test animal recombinantly expresses the polypeptide according to any of Claims 1-4;
b) measuring the activity of said polypeptide in said test animal after administering the compound of step (a) and
c) comparing the activity of said polypeptide in said sample with the activity of said polypeptide in a control animal not administered said polypeptide, wherein a change in the activity of said polypeptide in said test animal relative to said control animal indicates the test compound is a modulator of latency of, or predisposition to, a pathology associated with the polypeptide according to any of Claims 1-4;
wherein said method is not a method for treatment of the human or animal body by surgery or therapy, or a diagnostic method practised on the human or animal body.

29. The method of Claim 28, wherein said test animal is a recombinant test animal that expresses a test protein transgene or expresses said transgene under the control of a promoter at an increased level relative to a wild-type test animal, and wherein said promoter is not the native gene promoter of said transgene.

30. An *in vitro* method for determining the presence of or predisposition to a disease associated with altered levels of the polypeptide according to any of Claims 1-4 in a first mammalian subject, the method comprising:
a) measuring *in vitro* the level of expression of the polypeptide in a sample from the first mammalian subject; and
b) comparing *in vitro* the amount of said polypeptide in the sample of step (a) to the amount of the polypeptide present in a control sample from a second mammalian subject known not to have, or not to be predisposed to, said disease,
wherein an alteration in the expression level of the polypeptide in the first subject as compared to the control sample indicates the presence of or predisposition to said disease.

31. An *in vitro* method for determining the presence of or predisposition to a disease associated with altered levels of the nucleic acid molecule according to any of Claims 5-11 in a first mammalian subject, the method comprising:
a) measuring *in vitro* the amount of the nucleic acid in a sample from the first mammalian subject; and
b) comparing *in vitro* the amount of said nucleic acid in the sample of step (a) to the amount of the nucleic acid present in a control sample from a second mammalian subject known not to have or not be predisposed to, the disease;
wherein an alteration in the level of the nucleic acid in the first subject as compared to the control sample indicates the presence of or predisposition to the disease.

32. Use of a polypeptide having an amino acid sequence at least 95% identical to a polypeptide comprising the amino acid sequence given by the SEQ ID NO: 30 or 32, or a biologically active fragment thereof, for the manufacture of a medicament for treating a pathological state in a mammal.

33. Use of the antibody of Claim 15 for the manufacture of a medicament for treating a pathological state in a mammal.
